# EUROPEAN PATENT SPECIFICATION

(11) **EP 2 985 348 B1**
(45) Date of publication and mention of the grant of the patent: **30.12.2020**
(21) Application number: 15180289.9
(22) Date of filing: 23.07.2010
(51) Int. Cl.: C12N 15/63, C12N 15/11, C07H 19/00, C12Q 1/68

(54) **TREATMENT OF SIRTUIN 6 (SIRT6) RELATED DISEASES BY INHIBITION OF NATURAL ANTISENSE TRANSCRIPT TO SIRT6**
BEHANDLUNG VON SIRTUIN 6 (SIRT6)-BEDINGTEN ERKRANKUNGEN MITTELS HEMMUNG DES NATÜRLICHEN ANTISENSE-TRANSKRIPTS GEGEN SIRT6
TRAITEMENT DE MALADIES LIÉES À LA SIRTUINE 6 (SIRT6) PAR INHIBITION DU TRANSCRIT ANTISENS NATUREL DE SIRT6

(30) Priority: 24.07.2009 US 228392 P; 06.11.2009 US 259072 P; 02.12.2009 WO PCT/US2009/066445; 03.03.2010 WO PCT/US2010/026119; 03.05.2010 US 330427 P
(43) Date of publication of application: 17.02.2016
(62) Document number(s) of the earlier application(s) in accordance with Art. 76 EPC: 10802960.4 / 2 467 481
(73) Proprietor: CuRNA, Inc., Miami, FL 33137 (US)
(72) Inventor: COLLARD, Joseph, Delray Beach, FL Florida 33483 (US); KHORKOVA SHERMAN, Olga, Tequesta, FL Florida 33469 (US)
(74) Representative: Mathys & Squire

(56) References cited:
- WO-A1-2005/090996
- WO-A2-2005/110464
- WO-A2-2010/065662
- DATABASE BIOSIS [Online] BIOSCIENCES INFORMATION SERVICE, PHILADELPHIA, PA, US; October 2008 (2008-10), KUEHBACHER ANGELIKA ET AL: "MicroRNA 92a Controls Vessel Growth And Functional Recovery After Ischemia", XP002684082, Database accession no. PREV200900197869
- BONAUER ANGELIKA ET AL: "MicroRNA-92a controls angiogenesis and functional recovery of ischemic tissues in mice", SCIENCE, AMERICAN ASSOCIATION FOR THE ADVANCEMENT OF SCIENCE, WASHINGTON, DC; US, vol. 324, no. 5935, 21 May 2009 (2009-05-21), pages 1710-1713, XP002575234, ISSN: 0036-8075 -& A. Bonauer ET AL: "Supporting Online Material for MicroRNA-92a Controls Angiogenesis and Functional Recovery of Ischemic Tissues in Mice", Science, 21 May 2009 (2009-05-21), pages 1-35, XP055042749, Retrieved from the Internet: URL:http://www.sciencemag.org/content/supp l/2009/05/21/1174381.DC1/Bonauer_SOM.pdf [retrieved on 2012-10-31]
- DATABASE GENBANK [Online] NCBI; 26 June 2007 (2007-06-26), anonymous: "Homo sapiens ankyrin repeat domain 24 (ANKRD24), mRNA", XP002746819, retrieved from http://www.ncbi.nlm.nih.gov/genbank/ Database accession no. NM_133475
- DATABASE ENA [Online] EMBL; 26 February 2004 (2004-02-26), anonymous: "CM4-IT0042-131200-611-h08 IT0042 Homo sapiens cDNA, mRNA sequence.", XP002746820, retrieved from EBI accession no. EMBL:BF772662 Database accession no. BF772662
- DATABASE ENA [Online] EMBL; 16 December 2008 (2008-12-16), anonymous: "Homo sapiens piRNA piR-44760, complete sequence.", XP002746821, retrieved from EBI accession no. EMBL:DQ576648 Database accession no. DQ576648
- DATABASE Geneseq [Online] 28 December 2007 (2007-12-28), "Human BCR target sequence SEQ ID NO:5051.", XP002746822, retrieved from EBI accession no. GSN:AJU95541 Database accession no. AJU95541
- MOSTOSLAVSKY RAUL ET AL: "Genomic Instability and Aging-like Phenotype in the Absence of Mammalian SIRT6", CELL, vol. 124, no. 2, 27 January 2006 (2006-01-27), pages 315-329, XP029052003, ISSN: 0092-8674, DOI: 10.1016/J.CELL.2005.11.044
- DATABASE Geneseq [Online] 29 October 2009 (2009-10-29), "Human genome sequence PCR primer SEQ ID NO:74973.", XP002746823, retrieved from EBI accession no. GSN:AXA68544 Database accession no. AXA68544
- L R Saunders ET AL: "Sirtuins: critical regulators at the crossroads between cancer and aging", ONCOGENE, vol. 26, no. 37, 1 August 2007 (2007-08-01), pages 5489-5504, XP055439842, ISSN: 0950-9232, DOI: 10.1038/sj.onc.1210616

### SEQUENCE LISTING

<110> CuRNA, Inc.
<120> TREATMENT OF SIRTUIN (SIRT) RELATED DISEASES BY INHIBITION OF NATURAL ANTISENSE TRANSCRIPT TO SIRTUIN
<130> P38346EP-D1-PCT
<140> Not yet assigned
   <141> 2010-07-23
<150> EP10802960.4
   <151> 2010-07-23
<150> US61/228392
   <151> 2009-07-24
<150> US61/259,072
   <151> 2009-11-06
<150> PCT/US09/66445
   <151> 2009-12-02
<150> PCT/US10/26119
   <151> 2010-03-03
<150> US61/330427
   <151> 2010-05-03
<160> 99
<170> PatentIn version 3.5
<210> 1
   <211> 4107
   <212> DNA
   <213> Homo sapiens
<300>
   <308> NM_012238.3
   <309> 2008-11-30
   <313> (1)..(4107)
<400> 1 
gtcgagcggg agcagaggag gcgagggagg agggccagag aggcagttgg aagatggcgg 60

acgaggcggc cctcgccctt cagcccggcg gctccccctc ggcggcgggg gccgacaggg 120

aggccgcgtc gtcccccgcc ggggagccgc tccgcaagag gccgcggaga gatggtcccg 180

gcctcgagcg gagcccgggc gagcccggtg gggcggcccc agagcgtgag gtgccggcgg 240

cggccagggg ctgcccgggt gcggcggcgg cggcgctgtg gcgggaggcg gaggcagagg 300

cggcggcggc aggcggggag caagaggccc aggcgactgc ggcggctggg gaaggagaca 360

atgggccggg cctgcagggc ccatctcggg agccaccgct ggccgacaac ttgtacgacg 420

aagacgacga cgacgagggc gaggaggagg aagaggcggc ggcggcggcg attgggtacc 480

gagataacct tctgttcggt gatgaaatta tcactaatgg ttttcattcc tgtgaaagtg 540
 
atgaggagga tagagcctca catgcaagct ctagtgactg gactccaagg ccacggatag 600

gtccatatac ttttgttcag caacatctta tgattggcac agatcctcga acaattctta 660

aagatttatt gccggaaaca atacctccac ctgagttgga tgatatgaca ctgtggcaga 720

ttgttattaa tatcctttca gaaccaccaa aaaggaaaaa aagaaaagat attaatacaa 780

ttgaagatgc tgtgaaatta ctgcaagagt gcaaaaaaat tatagttcta actggagctg 840

gggtgtctgt ttcatgtgga atacctgact tcaggtcaag ggatggtatt tatgctcgcc 900

ttgctgtaga cttcccagat cttccagatc ctcaagcgat gtttgatatt gaatatttca 960

gaaaagatcc aagaccattc ttcaagtttg caaaggaaat atatcctgga caattccagc 1020

catctctctg tcacaaattc atagccttgt cagataagga aggaaaacta cttcgcaact 1080

atacccagaa catagacacg ctggaacagg ttgcgggaat ccaaaggata attcagtgtc 1140

atggttcctt tgcaacagca tcttgcctga tttgtaaata caaagttgac tgtgaagctg 1200

tacgaggaga tatttttaat caggtagttc ctcgatgtcc taggtgccca gctgatgaac 1260

cgcttgctat catgaaacca gagattgtgt tttttggtga aaatttacca gaacagtttc 1320

atagagccat gaagtatgac aaagatgaag ttgacctcct cattgttatt gggtcttccc 1380

tcaaagtaag accagtagca ctaattccaa gttccatacc ccatgaagtg cctcagatat 1440

taattaatag agaacctttg cctcatctgc attttgatgt agagcttctt ggagactgtg 1500

atgtcataat taatgaattg tgtcataggt taggtggtga atatgccaaa ctttgctgta 1560

accctgtaaa gctttcagaa attactgaaa aacctccacg aacacaaaaa gaattggctt 1620

atttgtcaga gttgccaccc acacctcttc atgtttcaga agactcaagt tcaccagaaa 1680

gaacttcacc accagattct tcagtgattg tcacactttt agaccaagca gctaagagta 1740

atgatgattt agatgtgtct gaatcaaaag gttgtatgga agaaaaacca caggaagtac 1800

aaacttctag gaatgttgaa agtattgctg aacagatgga aaatccggat ttgaagaatg 1860

ttggttctag tactggggag aaaaatgaaa gaacttcagt ggctggaaca gtgagaaaat 1920

gctggcctaa tagagtggca aaggagcaga ttagtaggcg gcttgatggt aatcagtatc 1980

tgtttttgcc accaaatcgt tacattttcc atggcgctga ggtatattca gactctgaag 2040

atgacgtctt atcctctagt tcttgtggca gtaacagtga tagtgggaca tgccagagtc 2100

caagtttaga agaacccatg gaggatgaaa gtgaaattga agaattctac aatggcttag 2160

aagatgagcc tgatgttcca gagagagctg gaggagctgg atttgggact gatggagatg 2220

atcaagaggc aattaatgaa gctatatctg tgaaacagga agtaacagac atgaactatc 2280

catcaaacaa atcatagtgt aataattgtg caggtacagg aattgttcca ccagcattag 2340

gaactttagc atgtcaaaat gaatgtttac ttgtgaactc gatagagcaa ggaaaccaga 2400

aaggtgtaat atttataggt tggtaaaata gattgttttt catggataat ttttaacttc 2460
 
attatttctg tacttgtaca aactcaacac taactttttt ttttttaaaa aaaaaaaggt 2520

actaagtatc ttcaatcagc tgttggtcaa gactaacttt cttttaaagg ttcatttgta 2580

tgataaattc atatgtgtat atataatttt ttttgttttg tctagtgagt ttcaacattt 2640

ttaaagtttt caaaaagcca tcggaatgtt aaattaatgt aaagggacag ctaatctaga 2700

ccaaagaatg gtattttcac ttttctttgt aacattgaat ggtttgaagt actcaaaatc 2760

tgttacgcta aacttttgat tctttaacac aattattttt aaacactggc attttccaaa 2820

actgtggcag ctaacttttt aaaatctcaa atgacatgca gtgtgagtag aaggaagtca 2880

acaatatgtg gggagagcac tcggttgtct ttacttttaa aagtaatact tggtgctaag 2940

aatttcagga ttattgtatt tacgttcaaa tgaagatggc ttttgtactt cctgtggaca 3000

tgtagtaatg tctatattgg ctcataaaac taacctgaaa aacaaataaa tgctttggaa 3060

atgtttcagt tgctttagaa acattagtgc ctgcctggat ccccttagtt ttgaaatatt 3120

tgccattgtt gtttaaatac ctatcactgt ggtagagctt gcattgatct tttccacaag 3180

tattaaactg ccaaaatgtg aatatgcaaa gcctttctga atctataata atggtacttc 3240

tactggggag agtgtaatat tttggactgc tgttttccat taatgaggag agcaacaggc 3300

ccctgattat acagttccaa agtaataaga tgttaattgt aattcagcca gaaagtacat 3360

gtctcccatt gggaggattt ggtgttaaat accaaactgc tagccctagt attatggaga 3420

tgaacatgat gatgtaactt gtaatagcag aatagttaat gaatgaaact agttcttata 3480

atttatcttt atttaaaagc ttagcctgcc ttaaaactag agatcaactt tctcagctgc 3540

aaaagcttct agtctttcaa gaagttcata ctttatgaaa ttgcacagta agcatttatt 3600

tttcagacca tttttgaaca tcactcctaa attaataaag tattcctctg ttgctttagt 3660

atttattaca ataaaaaggg tttgaaatat agctgttctt tatgcataaa acacccagct 3720

aggaccatta ctgccagaga aaaaaatcgt attgaatggc catttcccta cttataagat 3780

gtctcaatct gaatttattt ggctacacta aagaatgcag tatatttagt tttccatttg 3840

catgatgttt gtgtgctata gatgatattt taaattgaaa agtttgtttt aaattatttt 3900

tacagtgaag actgttttca gctcttttta tattgtacat agtcttttat gtaatttact 3960

ggcatatgtt ttgtagactg tttaatgact ggatatcttc cttcaacttt tgaaatacaa 4020

aaccagtgtt ttttacttgt acactgtttt aaagtctatt aaaattgtca tttgactttt 4080

ttctgttaaa aaaaaaaaaa aaaaaaa 4107
<210> 2
   <211> 3806
   <212> DNA
   <213> Mus musculus
<300>
   <308> NM_001159589.1
   <309> 2010-07-18
   <313> (1)..(3806)
<400> 2 
gccagtgccg cgcgtcgagc ggagcagagg aggcgagggc ggagggccag agaggcagtt 60

ggaagatggc ggacgaggtg gcgctcgccc ttcaggccgc cggctcccct tccgcggcgg 120

ccgccatgga ggccgcgtcg cagccggcgg acgagccgct ccgcaagagg ccccgccgag 180

acgggcctgg cctcgggcgc agcccgggcg agccgagcgc agcagtggcg ccggcggccg 240

cggggtgtga ggcggcgagc gccgcggccc cggcggcgct gtggcgggag gcggcagggg 300

cggcggcgag cgcggagcgg gaggccccgg cgacggccgt ggccggggac ggagacaatg 360

ggtccggcct gcggcgggag ccgagggcgg ctgacgactt cgacgacgac gagggcgagg 420

aggaggacga ggcggcggcg gcagcggcgg cggcagcgat cggctaccga ggtccatata 480

cttttgttca gcaacatctc atgattggca ccgatcctcg aacaattctt aaagatttat 540

taccagaaac aattcctcca cctgagctgg atgatatgac gctgtggcag attgttatta 600

atatcctttc agaaccacca aagcggaaaa aaagaaaaga tatcaataca attgaagatg 660

ctgtgaagtt actgcaggag tgtaaaaaga taatagttct gactggagct ggggtttctg 720

tctcctgtgg gattcctgac ttcagatcaa gagacggtat ctatgctcgc cttgcggtgg 780

acttcccaga cctcccagac cctcaagcca tgtttgatat tgagtatttt agaaaagacc 840

caagaccatt cttcaagttt gcaaaggaaa tatatcccgg acagttccag ccgtctctgt 900

gtcacaaatt catagctttg tcagataagg aaggaaaact acttcgaaat tatactcaaa 960

atatagatac cttggagcag gttgcaggaa tccaaaggat ccttcagtgt catggttcct 1020

ttgcaacagc atcttgcctg atttgtaaat acaaagttga ttgtgaagct gttcgtggag 1080

acatttttaa tcaggtagtt cctcggtgcc ctaggtgccc agctgatgag ccacttgcca 1140

tcatgaagcc agagattgtc ttctttggtg aaaacttacc agaacagttt catagagcca 1200

tgaagtatga caaagatgaa gttgacctcc tcattgttat tggatcttct ctgaaagtga 1260

gaccagtagc actaattcca agttctatac cccatgaagt gcctcaaata ttaataaata 1320

gggaaccttt gcctcatcta cattttgatg tagagctcct tggagactgc gatgttataa 1380

ttaatgagtt gtgtcatagg ctaggtggtg aatatgccaa actttgttgt aaccctgtaa 1440

agctttcaga aattactgaa aaacctccac gcccacaaaa ggaattggtt catttatcag 1500

agttgccacc aacacctctt catatttcgg aagactcaag ttcacctgaa agaactgtac 1560

cacaagactc ttctgtgatt gctacacttg tagaccaagc aacaaacaac aatgttaatg 1620

atttagaagt atctgaatca agttgtgtgg aagaaaaacc acaagaagta cagactagta 1680

ggaatgttga gaacattaat gtggaaaatc cagattttaa ggctgttggt tccagtactg 1740
 
cagacaaaaa tgaaagaact tcagttgcag aaacagtgag aaaatgctgg cctaatagac 1800

ttgcaaagga gcagattagt aagcggcttg agggtaatca atacctgttt gtaccaccaa 1860

atcgttacat attccacggt gctgaggtat actcagactc tgaagatgac gtcttgtcct 1920

ctagttcctg tggcagtaac agtgacagtg gcacatgcca gagtccaagt ttagaagaac 1980

ccttggaaga tgaaagtgaa attgaagaat tctacaatgg cttggaagat gatacggaga 2040

ggcccgaatg tgctggagga tctggatttg gagctgatgg aggggatcaa gaggttgtta 2100

atgaagctat agctacaaga caggaattga cagatgtaaa ctatccatca gacaaatcat 2160

aacactattg aagctgtccg gattcaggaa ttgctccacc agcattggga actttagcat 2220

gtcaaaaaat gaatgtttac ttgtgaactt gaacaaggaa atctgaaaga tgtattattt 2280

atagactgga aaatagattg tcttcttgga taatttctaa agttccatca tttctgtttg 2340

tacttgtaca ttcaacactg ttggttgact tcatcttcct ttcaaggttc atttgtatga 2400

tacattcgta tgtatgtata attttgtttt ttgcctaatg agtttcaacc ttttaaagtt 2460

ttcaaaagcc attggaatgt taatgtaaag ggaacagctt atctagacca aagaatggta 2520

tttcacactt ttttgtttgt aacattgaat agtttaaagc cctcaatttc tgttctgctg 2580

aacttttatt tttaggacag ttaacttttt aaacactggc attttccaaa acttgtggca 2640

gctaactttt taaaatcaca gatgacttgt aatgtgagga gtcagcaccg tgtctggagc 2700

actcaaaact tggtgctcag tgtgtgaagc gtacttactg catcgttttt gtacttgctg 2760

cagacgtggt aatgtccaaa caggcccctg agactaatct gataaatgat ttggaaatgt 2820

gtttcagttg ttctagaaac aatagtgcct gtctatatag gtccccttag tttgaatatt 2880

tgccattgtt taattaaata cctatcactg tggtagagcc tgcatagatc ttcaccacaa 2940

atactgccaa gatgtgaata tgcaaagcct ttctgaatct aataatggta cttctactgg 3000

ggagagtgta atattttgga ctgctgtttt tccattaatg aggaaagcaa taggcctctt 3060

aattaaagtc ccaaagtcat aagataaatt gtagctcaac cagaaagtac actgttgcct 3120

gttgaggatt tggtgtaatg tatcccaagg tgttagcctt gtattatgga gatgaataca 3180

gatccaatag tcaaatgaaa ctagttctta gttatttaaa agcttagctt gccttaaaac 3240

tagggatcaa ttttctcaac tgcagaaact tttagccttt caaacagttc acacctcaga 3300

aagtcagtat ttattttaca gacttctttg gaacattgcc cccaaattta aatattcatg 3360

tgggtttagt atttattaca aaaaaatgat ttgaaatata gctgttcttt atgcataaaa 3420

tacccagtta ggaccattac tgccagagga gaaaagtatt aagtagctca tttccctacc 3480

taaaagataa ctgaatttat ttggctacac taaagaatgc agtatattta gttttccatt 3540

tgcatgatgt gtttgtgcta tagacaatat tttaaattga aaatttgttt ttaaattatt 3600
 
tttacagtga agactgtttt cagctctttt tatattgtac atagactttt atgtaatctg 3660

gcatatgttt tgtagaccgt ttaatgactg gattatcttc ctccaacttt tgaaatacaa 3720

aaacagtgtt ttatacttgt atcttgtttt aaagtcttat attaaaattg tcatttgact 3780

tttttcccgt taaaaaaaaa aaaaaa 3806
<210> 3
   <211> 2919
   <212> DNA
   <213> Homo sapiens
<300>
   <308> NM_012239
   <309> 2010-07-04
   <313> (1)..(2919)
<400> 3 
gcgagtccgg aggactcctt ggactgcgcg gaacatggcg ttctggggtt ggcgcgccgc 60

ggcagccctc cggctgtggg gccgggtagt tgaacgggtc gaggccgggg gaggcgtggg 120

gccgtttcag gcctgcggct gtcggctggt gcttggcggc agggacgatg tgagtgcggg 180

gctgagaggc agccatgggg cccgcggtga gcccttggac ccggcgcgcc ccttgcagag 240

gcctcccaga cccgaggtgc ccagggcatt ccggaggcag ccgagggcag cagctcccag 300

tttcttcttt tcgagtatta aaggtggaag aaggtccata tctttttctg tgggtgcttc 360

aagtgttgtt ggaagtggag gcagcagtga caaggggaag ctttccctgc aggatgtagc 420

tgagctgatt cgggccagag cctgccagag ggtggtggtc atggtggggg ccggcatcag 480

cacacccagt ggcattccag acttcagatc gccggggagt ggcctgtaca gcaacctcca 540

gcagtacgat ctcccgtacc ccgaggccat ttttgaactc ccattcttct ttcacaaccc 600

caagcccttt ttcactttgg ccaaggagct gtaccctgga aactacaagc ccaacgtcac 660

tcactacttt ctccggctgc ttcatgacaa ggggctgctt ctgcggctct acacgcagaa 720

catcgatggg cttgagagag tgtcgggcat ccctgcctca aagctggttg aagctcatgg 780

aacctttgcc tctgccacct gcacagtctg ccaaagaccc ttcccagggg aggacattcg 840

ggctgacgtg atggcagaca gggttccccg ctgcccggtc tgcaccggcg ttgtgaagcc 900

cgacattgtg ttctttgggg agccgctgcc ccagaggttc ttgctgcatg tggttgattt 960

ccccatggca gatctgctgc tcatccttgg gacctccctg gaggtggagc cttttgccag 1020

cttgaccgag gccgtgcgga gctcagttcc ccgactgctc atcaaccggg acttggtggg 1080

gcccttggct tggcatcctc gcagcaggga cgtggcccag ctgggggacg tggttcacgg 1140

cgtggaaagc ctagtggagc ttctgggctg gacagaagag atgcgggacc ttgtgcagcg 1200

ggaaactggg aagcttgatg gaccagacaa ataggatgat ggctgccccc acacaataaa 1260

tggtaacata ggagacatcc acatcccaat tctgacaaga cctcatgcct gaagacagct 1320
 
tgggcaggtg aaaccagaat atgtgaactg agtggacacc cgaggctgcc actggaatgt 1380

cttctcaggc catgagctgc agtgactggt agggctgtgt ttacagtcag ggccaccccg 1440

tcacatatac aaaggagctg cctgcctgtt tgctgtgttg aactcttcac tctgctgaag 1500

ctcctaatgg aaaaagcttt cttctgactg tgaccctctt gaactgaatc agaccaactg 1560

gaatcccaga ccgagtctgc tttctgtgcc tagttgaacg gcaagctcgg catctgttgg 1620

ttacaagatc cagacttggg ccgagcggtc cccagccctc ttcatgttcc gaagtgtagt 1680

cttgaggccc tggtgccgca cttctagcat gttggtctcc tttagtgggg ctatttttaa 1740

tgagagaaaa tctgttcttt ccagcatgaa atacatttag tctcctcaaa gggactgcag 1800

gtgttgacat gagttggaaa gggaaccctg ggatacgtgg cgtcccctct attggaacag 1860

tctgaggact gaaggcattt gtccctggat ttattggaga cggcccagct cctccctctg 1920

aaggtggtca cattctgttg actctccata ctcagcctct cctccagaaa cagatctgtt 1980

ccagaacatt ccagcacttt ctatctggcc tccttgtccc cacactacgc ccccccaccc 2040

tcgccagggc ttcctctagt gacactgtta gagctaatct ctgagacagg gaaggcatta 2100

ctcacttaaa acccaggctg agtcctggcc acctgctgga ttgtgacata ggaggtggaa 2160

tccactgaac tgctacttct gcacaggctc cttctcctgg ggctgtaccc aggcccagcc 2220

ctgatggctc accctgtcag gcaccagctg ctccctcctg ggctctcacc cacctgcaca 2280

tcctccttcc tagcatcaca ttacctgcgt gtttccccag acaaaagcac ttcccattct 2340

tgaaccttgc ctaccctggg ctgagctgac ggcaatagat ttaatgacag tgactcccag 2400

gaagggggtc ctgtgacttt gcgccttaat aagaacaaaa ggtggaattg gtgacctagg 2460

aaaactgttg aattctaaaa agaatgaagt tagtttctaa ccctagttaa tgttcctttt 2520

ttattttttg agtcttgccc tgtcactcag ggtggagtgc ggtgttatga tctcagctca 2580

ctgcaacttc cgcctcccgg gtttaagcga ttctcctggg tagctgggat tacaggtgtg 2640

tcccaccaca cctagcacat gggcatattt gtaatagaga caaggttttg ctatgttggc 2700

caggctggtc tcgaactcct ggcttcaagt gatccaccca cctcggcctc ccaaagtgct 2760

gggattacag gcatgagcca ctgtgcctgg cccctttatt tgataattta cacatacatt 2820

tttgtccaaa actcttcttt atttcaagat gatgtttctg tggctatgtg tggtatgtgg 2880

tataaatctc aatctatggt caaaaaaaaa aaaaaaaaa 2919
<210> 4
   <211> 1657
   <212> DNA
   <213> Homo sapiens
<300>
   <308> NM_016539
   <309> 2010-07-17
   <313> (1)..(1657)
<400> 4 
gcttccggcg gaagcggcct caacaaggga aactttattg ttcccgtggg gcagtcgagg 60

atgtcggtga attacgcggc ggggctgtcg ccgtacgcgg acaagggcaa gtgcggcctc 120

ccggagatct tcgacccccc ggaggagctg gagcggaagg tgtgggaact ggcgaggctg 180

gtctggcagt cttccagtgt ggtgttccac acgggtgccg gcatcagcac tgcctctggc 240

atccccgact tcaggggtcc ccacggagtc tggaccatgg aggagcgagg tctggccccc 300

aagttcgaca ccacctttga gagcgcgcgg cccacgcaga cccacatggc gctggtgcag 360

ctggagcgcg tgggcctcct ccgcttcctg gtcagccaga acgtggacgg gctccatgtg 420

cgctcaggct tccccaggga caaactggca gagctccacg ggaacatgtt tgtggaagaa 480

tgtgccaagt gtaagacgca gtacgtccga gacacagtcg tgggcaccat gggcctgaag 540

gccacgggcc ggctctgcac cgtggctaag gcaagggggc tgcgagcctg caggggagag 600

ctgagggaca ccatcctaga ctgggaggac tccctgcccg accgggacct ggcactcgcc 660

gatgaggcca gcaggaacgc cgacctgtcc atcacgctgg gtacatcgct gcagatccgg 720

cccagcggga acctgccgct ggctaccaag cgccggggag gccgcctggt catcgtcaac 780

ctgcagccca ccaagcacga ccgccatgct gacctccgca tccatggcta cgttgacgag 840

gtcatgaccc ggctcatgaa gcacctgggg ctggagatcc ccgcctggga cggcccccgt 900

gtgctggaga gggcgctgcc acccctgccc cgcccgccca cccccaagct ggagcccaag 960

gaggaatctc ccacccggat caacggctct atccccgccg gccccaagca ggagccctgc 1020

gcccagcaca acggctcaga gcccgccagc cccaaacggg agcggcccac cagccctgcc 1080

ccccacagac cccccaaaag ggtgaaggcc aaggcggtcc ccagctgacc agggtgcttg 1140

gggagggtgg ggctttttgt agaaactgtg gattcttttt ctctcgtggt ctcactttgt 1200

tacttgtttc tgtccccggg agcctcaggg ctctgagagc tgtgctccag gccaggggtt 1260

acacctgccc tccgtggtcc ctccctgggc tccaggggcc tctggtgcgg ttccgggaag 1320

aagccacacc ccagaggtga caggtgagcc cctgccacac cccagcctct gacttgctgt 1380

gttgtccaga ggtgaggctg ggccctccct ggtctccagc ttaaacagga gtgaactccc 1440

tctgtcccca gggcctccct tctgggcccc ctacagccca ccctacccct cctccatggg 1500

ccctgcagga ggggagaccc accttgaagt gggggatcag tagaggcttg cactgccttt 1560

ggggctggag ggagacgtgg gtccaccagg cttctggaaa agtcctcaat gcaataaaaa 1620

caatttcttt cttgcaaaaa aaaaaaaaaa aaaaaaa 1657
<210> 5
   <211> 1028
   <212> DNA
   <213> Homo sapiens
<400> 5 
cttttcactt gtgaatacca attaggtttc cagtttctca taaagatcta acaaataccc 60

aatttctcca tcagactgac atcccttaac aaaagcagag tttcaattcc ctgcatctcc 120

tttaggagct atgatataat gtaggtagaa atcttgcctt aactccattt acccactgtg 180

ctataaataa gcagaagcaa atattttttt aaggctggag aggttttaaa aatctgaact 240

aatttagcaa ctgctgctgc actcagtttt tggcagttcc caaacatcca ttatcatgta 300

aggataaatc cttctaaacc agaaaaatgt ttcctacttg gaaaaggcat aagaaaatac 360

atatacgacc tccccatgta ctagtcttac ataccccagc tccagttaga actataattt 420

ttttgcactc ttgcagtaat ttcacagcat cttcaattgt attaatatct tttctttttt 480

tcctttttgg tggttctgaa aggatattaa taacaatctg ccacagtgtc atatcatcca 540

actcaggtgg aggtattgtt tccggcaata aatctttaag aattgttcga ggatctgtgc 600

caatcataag atgttgctga acaaaagtat atggacctac aataaggggg aaaaggctta 660

aagtcaactt atcaagtaat tcaaaatctc atttattttc tgaagtaatg agttagcatt 720

ctgtgagggt tttttgcaaa gtaagaaaat gcaatttaat ggtatttcat tctcggtaca 780

ctcagaatta atgctatatc ccaatgagat taggaagatc taatgaagag ttgggaagac 840

ccccttcagc tgtaagtata tatttcaaga gtctaattaa ttaacaacca gaattaagtt 900

cttatggtta atatctagaa acacacacca taataccaaa agtatttaca aaagggttct 960

acgacataga aaaatcgtac cagtcctaaa agcctgtact acttatcatt aaaaccacac 1020

aggaaaaa 1028
<210> 6
   <211> 429
   <212> DNA
   <213> Homo sapiens
<220>
   <221> misc_feature
   <222> (7)..(7)
   <223> n is a, c, g, or t
<220>
   <221> misc_feature
   <222> (373)..(373)
   <223> n is a, c, g, or t
<220>
   <221> misc_feature
   <222> (400)..(400)
   <223> n is a, c, g, or t
<400> 6 
cgacganaac ataagcactt ttaatttcct ctctattatg ctacagacaa ggccgaagac 60

tgggtttttt aggttgttta aggctgtaaa gaaaacaaag aacatatgac agagacccta 120

tgcagtctgc aaagcatact acatatttac taccaggccc taccttacta cagaaagttt 180

gctgatccag ctgtgaacat ataccccgat gcagatgaaa acaaatacaa aacaaaccta 240

acttgccatt ttggtcacaa gagcaagtaa gtagcagagc cctgttttga tatgaaaatc 300

cagcactgga ctgggcaaca tggcgagacc ccatctctac caaaaatact aaaaaaatag 360

ccgggcatgg tgnggcacat ctgtagtact agctacttgn gaggctgaga caggagaatc 420

atttgagcc 429
<210> 7
   <211> 156
   <212> DNA
   <213> Homo sapiens
<400> 7 
cctgtatata cacacactat gcaatagtct taggtaacta attagctgca accctaaggt 60

agatcaaata gaaaatgtca agtcgccaca atcacatcat cttaattaat atggagggaa 120

ggtaggaatc tgttactctt cccaacacta agcttt 156
<210> 8
   <211> 593
   <212> DNA
   <213> Homo sapiens
<220>
   <221> misc_feature
   <222> (460)..(460)
   <223> n is a, c, g, or t
<220>
   <221> misc_feature
   <222> (571)..(571)
   <223> n is a, c, g, or t
<400> 8 
tctcactgtc tcccaggctg gagcgcaatg gtgcaatctt ggctcgctgc aacctccaac 60

tcccaggttc aagtgattct cgtgcctcag cctcctgagt agctgggatt acaggcgcct 120

gccaccatgc ccggctaatt ttagtatttc tagtagagtt gggatttcac catgttggcc 180

aagctgttct cgaactcttg gcttcatggg atctgcccgc ctcggcttcc caaagtgcta 240

ggattacagg cgtgagccac tgcactcagc cagaaaagat tatttaaaat aattgaatgc 300

atgcaactgc agcatctttt gaaatcaaaa gcaaattaat atctgaggtt ttctataatt 360

aactgtcaag gccaatactt ctggttttat tatttttggt ttctactttt ctgaggttat 420

cgataaatgg agaaacatga ttaaataaat gctttatctn cacttctcga tggcagtcag 480
 
ctttaatgga aaatattttc ctataaacct aaattaattt ccggaaaccc cttttgaggt 540

taactaccat tgcactggaa taatctttgg natcccggaa ccctgttcaa ggg 593
<210> 9
   <211> 373
   <212> DNA
   <213> Homo sapiens
<400> 9 
ggcacttcat ggggtatgga acctaaaata gggtgaaaaa taagagttag aaaaagggag 60

gaggaagtaa gctaataaaa ccacaacaga tacattatgc aaaaatccaa caagggcttg 120

agctccaaat tcccaaaaaa gacccatgca ctaaatactt aaccaagcca ttacatttca 180

tcaagcaagc agatgggcag ttaagtgtct tttataaaac gctcaagttc tgctcctatc 240

acctggttac ccctgagcaa tcttcccaga ctttcccact ctgaacctca aatttccttg 300

tctgcagaat ggggatgata atagtacttc tccacaagac tgtggtgagg attaaatgag 360

ttagtcaagt gca 373
<210> 10
   <211> 1713
   <212> DNA
   <213> Mus musculus
<400> 10 
gggctcccct cagcggcctc tggcgcctcc cgcccgcccg acccgttcgc tcgctcgctc 60

gctcgctcgc ttgctcgtcc gggatcgccg cggtggttca agtttgcgat ggcgccgcca 120

cttcccacct gggcctcacg cgtgcacctt gcctgcctgc gcctcttcgc ctcaagtcgg 180

cttttacctc aggggctctg gagagcccaa cctggccgac gccggccttc ctgaggagaa 240

ctcctccacc tgccttgccc ttgctctgtg acagctcttc ctcaggttac ccctgtggtc 300

tctcctcagg aagtttgcgc tctctcccaa tctcccttct caagtgcaat ggaatgccca 360

agccagccct cggggcctgt tgccctcctg gaaagatctg gcgattgagg acccgcccta 420

tctgctctct ggacccacca ggtcctctgt acctcgcttt agtctttggt aaaattcatc 480

tcttggggca gcaagagaga ggacagaagg gagagtggtt ggttctccac aaacttctgt 540

gttaagagtc agattgggcc tgggctcttg tgacttgggc gattgactga acctttctta 600

agcccagttt ttaatcatct ctaaaatgac agggccagga ccgaaagaga ctgtagctca 660

gttgtaaagt cacgcttgcc agacaacccc gaagccctag agagagggag gaaggagggt 720

aagttgaagg taatctccaa ctacttagga agttcaaaaa aggcctggaa tacataagac 780

ctcgtctcaa aaacgaaatt taaaacgata gaccatgaga aatcagctag tcaggtttaa 840

agtaaatgac attagtttta aaatcctagg cagttgatgg tggcacaggc ctttaatccc 900

agcaagctgg aggagacagg aggaggttca ctaggacagc caaggctaca caaagaaacc 960
 
ctgtctcgaa aaaataatct tacttctaga attgtagaaa tggctctgta gttaacagca 1020

cttgttgctc ctgcagaggc cctaggtttg actcccatca tccacatgac agctcatacc 1080

ttcagatctg acacctgctt ttggtaaaca cagacatgta tggagccaaa agacccaaac 1140

acataaaaat cctctttgtt gttgttttat gagttagggt ttctctgtgt agccctggct 1200

gtccaggaac tctgtagatc aggctgtcct tgaactcaga ggccacctgc ctctgcttct 1260

tgaactgctg ggattaaaga tgtacaccag caagcccagc ataaaaatac atatttaaat 1320

aattttttaa ataatcctta gttccttcac aactctaagc cccttcactt tctagttacc 1380

atgaaattct gagcacctgt atccatttgg atcattaggg ctcaattgca catggttcaa 1440

ttacagtggg gtttccccag attttagagt tagaggcagc aggatcagaa aattaaatcc 1500

atttgcacta ggtaataaat ttgatcccac cctatctcaa aaacaaaaca ctagccacac 1560

gtggcagcac acacctttta caacaggact caggagcctg gcatgatggg acagaccttt 1620

actccctgca cttgaggcag atgcaggcaa atctaggcat cctggtgtac atatgaagtt 1680

caggcaagcc agggccacgt aggctcaaag acg 1713
<210> 11
   <211> 660
   <212> DNA
   <213> Homo sapiens
<220>
   <221> misc_feature
   <222> (587)..(587)
   <223> n is a, c, g, or t
<220>
   <221> misc_feature
   <222> (628)..(628)
   <223> n is a, c, g, or t
<400> 11 
tttttttttt tttttttccc acaggtagat aagagtcttg ctctgtcacc caggctggag 60

tgcagtggct caatcatagc tcactgcagc cttgaactcc tggactcaag ggatcctctt 120

gccttagtct caggagtaac tgggacaaca ggcttgcgcc accatgcccg gctaattttt 180

aaatttttgg tagagatggg ggtctcgtta tgttgcccag gctgctcttg aactcctggc 240

ctcgagcgat ccccgtctca gcctccgaaa gtgctggtat tacaagtgtg agctaccact 300

tcgagactca cttttcacca acattttcag cagttgtgtc agacagcaag tcaatgtgcc 360

attatttact taaatattca tccattatag ggcatctgat acctaacaac catggacctt 420

aagatttttt cctatgtagc ctcagttctt agatgcaatt actatggaga cgggtacgat 480

cacatgccag tgggagtatt aaccgcacaa catttatgag ggatcattaa agcgttaaat 540
 
gcatatactt ttggacctag caatcccagt actaggaatt tatgcanaca gtgaggtgcc 600

aacaaggtta ttcaccacag catgatgnca caatactaaa ggttagaacg tatttcaatg 660
<210> 12
   <211> 589
   <212> DNA
   <213> Homo sapiens
<400> 12 
agtctggtgt ctcatctctc gccccggagg tcaaacctgg agccagcatt tctgtaccct 60

gcgctcacac ctttttcaac ccggatggcg gtgatagtta atactttctg agcgctttta 120

ctatggtcag gcattatatt tacacctaat cctcgcaaaa cactactggg aagatcctca 180

tttgacagga gcaattccgg gtcacaaagg ctgacaccac aaagctagtc cgtagcgggt 240

tcgaccacag gcgcccacac tctttgacgc ctcaatggca cagccaagtg cgcgggaagt 300

gggctgcaaa cgccggagag ttttgtccgg agcgcagaga cgcgctgtaa ccgagcaacc 360

agcggggccc gcccccggcc tgctacggcg ctcccagcct gccccgcgcc gctcggcgcc 420

ggaagtgagt gagcatttcc ggcagccatc cccgcggtgc tgacatcccg gttgttcttc 480

tgtgccgggg gtcttcctgc tgtcatgaag gacgtaccgg gcttcctaca gcagagccag 540

aactccgggc ccgggcagcc cgctgtgtgg caccgtctgg aggagctct 589
<210> 13
   <211> 428
   <212> DNA
   <213> Homo sapiens
<400> 13 
agggtgagtt gcaagacagg aagaggggga tagaacaaga cacagacaga gagataaaga 60

gttaaagaca aagcaagtca gagacagaat tggagagaga cagagagagg gagaggcaga 120

gagagagaga tgaattcaca cgaaggcaaa aatgacttat tctattcaac caacactgct 180

gctgcaatga gcccgacgat gctcacatgg gcccaagccc tgccctccag aagcccttga 240

tcttggggga gacaggtgga cacagatcct ctggccccaa gagtcaagcc tggatcagag 300

gaggtacctg ggcctgggga agccccgtgg aggtgggggc cctggggcac gtggaagggc 360

ttcctggagg aggctaagca gtttcttgaa gggtgagagg gaagatcaga cagaagggat 420

cctctaga 428
<210> 14
   <211> 4041
   <212> DNA
   <213> Homo sapiens
<400> 14 gtggcctgga ggactcattt aagaatcatg tgttgagtac ctactttgta ctgggcacac 60 
tggagagcaa aggtggtacc atctatgccc tggtggctgc gggtggacac aaacatgtga 120
ccaggggcca ggactggtca ggaaggagga agcggtcctt ttgaggaggg cgaggcaacg 180
gaccctgccg cccaccagga ctggtgtccc tcaccttacc cccacccttg ccctcctcag 240
gtggcctgtg gagaggagaa acacagggca ccaactatga agactctcag ggcgcgattt 300
aagaagacag agctgcggct cagccccact gaccttggct cctgcccgcc ctgcggcccc 360
tgccccatcc cgaagccggc agccagaggc aggcgccaga gtcaagactg gggcaagagt 420
gacgagaggc tgctacaagc cgtggaaaac aacgatgcac ctcgggtggc cgccctcatc 480
gcccgcaagg ggctggtgcc cacgaagcta gaccccgagg gcaagtccgc gttccacctg 540
gcggccatgc ggggtgcggc cagctgtctg gaggtgatga tagctcatgg cagcaatgtc 600
atgagcgcgg acggggcagg ttacaatgcc ctccacctgg ccgccaaata cgggcaccca 660
cagtgcttga agcaactact gcaggcttcc tgcgtggtgg acgtcgtgga cagcagcggg 720
tggactgccc tacaccatgc agcggctggt ggctgtctct cctgctcaga ggtgctctgc 780
tcctttaagg cacatctaaa cccccaagat cggtcaggcg caacacccct cattatagca 840
gctcagatgt gtcacacaga cctgtgccgt ctcctactgc agcaaggggc tgccgcgaac 900
gatcaggacc tgcaaggcag gacggccctg atgctggcct gtgagggggc cagccccgaa 960
acagtggagg tcctgctgca gggcggagcc cagccgggca tcaccgatgc gctggggcag 1020
gacgcggctc actatggcgc cctggcgggg gacaaactca tcctgcacct tctgcaagag 1080
gcggcccagc gcccctcccc acccagcgcc ctcacagagg atgattcagg cgaggcgtca 1140
tctcagaact ctatgtccag ccatggaaag cagggggccc ccaagaagcg gaaggcgcct 1200
ccacctcccg ccagcattcc catgccggat gatcgagatg cctatgagga gatcgtgagg 1260
ctgcggcagg agaggggccg cctcctgcag aagatccggg gcctggaaca gcacaaggaa 1320
cggaggcagc aggagtcccc ggaggccagc tccctgcaca tcctggagag acaggtgcaa 1380
gagctacagc agttgctggt ggagagacaa gaggagaagg agagcctggg acgggaggtg 1440
gagagtttgc agagccggct gtccctgctg gagaacgagc gggagaatac tagctatgac 1500
gtaaccaccc tgcaggatga ggagggtgag ctgcctgacc ttccaggggc cgaggtgctg 1560
ctgtccagac aactcagtcc gtcggcccag gaacacctgg cctcgctgca ggaacaggtg 1620
gctgtgctca ccagacagaa ccaggaactg atggagaagg tccagatcct ggagaacttt 1680
gagaaggacg agacacagat ggaagtggaa gctttggcag aggtcatccc tcttgccctc 1740
tatgactctc tccgggccga gtttgaccag ctacgcaggc agcacgctga ggccctgcag 1800
gccctgaggc agcaggagac acgagaggtc cccagagaag agggggcagc ctgtggggag 1860
agtgaggttg ctggagccac ggccaccaaa aacgggccaa cccacatgga gctaaatggc 1920
tcagtggctc cagaaaccaa agttaacgga gccgagacca tagatgagga ggctgcagga 1980
 
gatgaaacca tggaagccag gactatggaa gctgaggcca cgggagccga ggccacggga 2040

gctgaggcca caggagccaa ggtcacagaa acaaaaccca caggggctga ggtcagagaa 2100

atggagacca cagaagaaga agcaaacatg gaaactaagc ccacaggagc tcaggccaca 2160

gacacagaga ccacgggagt ggaggccatg ggggtggagg ccacaaaaac aaaagcagag 2220

gaagcagaaa tgcaggccta cggagtgggt gctgggcaag cagagccccc agtcacaggg 2280

accacaaaca tggaggccac gggctctagg gccacaggga tggaatccac aggagtcagt 2340

gccacaggtg tggagaaccc aggggtagag gccacggtcc cggggatctc tgctggcccc 2400

atcctacatc ctggtgccgc agaggcctcg gaaaagcttc aagtagagct ggagaccagg 2460

atccgtggct tggaggaggc tctccggcag cgggagcggg aggcagctgc ggagctggag 2520

gcggccctgg ggaagtgcga ggccgcggag gccgaggcag gccggctgcg agagcgtgtc 2580

cgcgaggccg agggcagcgg ggccagcggg ggcggtggcg gtgacaccac acagctgcgg 2640

gcggccctgg agcaggcccg ggaggacctc cgagaccggg actcccgcct gcgggagctg 2700

gaggcggcct cggcctgcct ggatgaggct cgggccagcc ggctgctggc ggaggaggag 2760

gcgcggggcc tgcgggccga gctggcccag cgggaggagg cgcggctgga gcagagccgg 2820

gagctggagg ttctgcggga gcagctggcc acggccaggg ccacggggga gcagcagcgc 2880

acggcggccg cggaactggg ccgggcacgg gacgccgctg aggcccgagt ggctgagctg 2940

cctgcggcct gcgaggaggc gcggcagggc ctggccgagc tgcgggaggc ctccgaggcc 3000

ctccgccagt ccgtggtgcc ggcctctgag caccgccggc tgcaggagga ggccctggag 3060

ctgcggggcc gggcagccag tctggagcag gaggtggtgg ccacgggcaa ggaggccgcc 3120

cggctgcgcg cggagctgga gcgggagcgt gtgtgcagcg tggcgctctc ggagcacgaa 3180

cgcatcgtgg gcaccctgca ggccaacgtg gcccagctgg aggggcagct ggaggagctg 3240

ggacggcggc atgagaagac cagcgcagag gtcttccagg tgcagcgtga ggccctgttc 3300

atgaagagtg agcgacacgc agccgaggca cagctggcca cagcagagca gcagctacgg 3360

gggctacgga ccgaggcgga aagggctcgc caggcccaga gccgggccca ggaggctctg 3420

gacaaggcca aggagaagga caagaagatc acagaactct ccaaagaagt cttcaatctt 3480

aaggaagcct tgaaggagca gccggccgcc ctcgccaccc ctgaggtgga ggctctccgt 3540

gaccaggtga aggatttaca gcagcagctg caggaagctg ccagggacca ctccagcgtg 3600

gtggctttgt acagaagcca cctcctatat gccattcagg gccagatgga tgaagatgtg 3660

cagcggattc tcagccagat tctgcagatg cagagactcc aggctcaggg ccgctgagaa 3720

aggccaggcc cagtggctac actgaccaca cccacgcagg gacctcaccc ccctgcaggc 3780

cccttgcaga ccggcttcac ttggcttcac ttggccctat ccaggcccat gcacttggag 3840
 
accagcctgg ttccctgccc gaccaccccc agctggctcc atcaccccac ctggtctctg 3900

cacgcacaca ctggtcagtc tggacccggg ccgtgactgc ccctccccca ccaccggaga 3960

ctgtgattcc ctgtgtcctc cacatccaga cgccagccca ggaataaagg cattctgtgc 4020

acagggaaaa aaaaaaaaaa a 4041
<210> 15
   <211> 14
   <212> DNA
   <213> Artificial Sequence
<220>
   <223> Antisense oligonucleotide
<400> 15
   ttggtattca caag 14
<210> 16
   <211> 14
   <212> DNA
   <213> Artificial Sequence
<220>
   <223> Antisense oligonucleotide
<400> 16
   aaactggaaa ccta 14
<210> 17
   <211> 14
   <212> DNA
   <213> Artificial Sequence
<220>
   <223> Antisense Oligonucleotide
<400> 17
   gatctttatg agaa 14
<210> 18
   <211> 14
   <212> DNA
   <213> Artificial Sequence
<220>
   <223> Antisense oligonucleotide
<400> 18
   gatggagaaa ttgg 14
<210> 19
   <211> 14
   <212> DNA
   <213> Artificial Sequence
<220>
   <223> Antisense oligonucleotide
<400> 19
   agtctgatgg agaa 14
<210> 20
   <211> 14
   <212> DNA
   <213> Artificial Sequence
<220>
   <223> Antisense oligonucleotide
<400> 20
   tgttaaggga tgtc 14
<210> 21
   <211> 14
   <212> DNA
   <213> Artificial Sequence
<220>
   <223> Antisense oligonucleotide
<400> 21
   aatctgcttt tgtt 14
<210> 22
   <211> 14
   <212> DNA
   <213> Artificial Sequence
<220>
   <223> Antisense oligonucleotide
<400> 22
   agggaattga aatc 14
<210> 23
   <211> 14
   <212> DNA
   <213> Artificial Sequence
<220>
   <223> Antisense oligonucleotide
<400> 23
   taaggcaaga tttc 14
<210> 24
   <211> 14
   <212> DNA
   <213> Artificial Sequence
<220>
   <223> Antisense oligonucleotide
<400> 24
   taaatggagt taag 14
<210> 25
   <211> 14
   <212> DNA
   <213> Artificial Sequence
<220>
   <223> Antisense oligonucleotide
<400> 25
   ttatttatag caca 14
<210> 26
   <211> 14
   <212> DNA
   <213> Artificial Sequence
<220>
   <223> Antisense oligonucleotide
<400> 26
   ttgcttctgc ttat 14
<210> 27
   <211> 14
   <212> DNA
   <213> Artificial Sequence
<220>
   <223> Antisense oligonucleotide
<400> 27
   aaaaaaatat ttgc 14
<210> 28
   <211> 14
   <212> DNA
   <213> Artificial Sequence
<220>
   <223> Antisense oligonucleotide
<400> 28
   cagccttaaa aaaa 14
<210> 29
   <211> 14
   <212> DNA
   <213> Artificial Sequence
<220>
   <223> Antisense oligonucleotide
<400> 29
   ttttaaaacc tctc 14
<210> 30
   <211> 14
   <212> DNA
   <213> Artificial Sequence
<220>
   <223> Antisense oligonucleotide
<400> 30
   tagttcagat tttt 14
<210> 31
   <211> 14
   <212> DNA
   <213> Artificial Sequence
<220>
   <223> Antisense oligonucleotide
<400> 31
   agcagttgct aaat 14
<210> 32
   <211> 14
   <212> DNA
   <213> Artificial Sequence
<220>
   <223> Antisense oligonucleotide
<400> 32
   ctgagtgcag cagc 14
<210> 33
   <211> 12
   <212> DNA
   <213> Artificial Sequence
<220>
   <223> Antisense oligonucleotide
<400> 33
   gtctgatgga ga 12
<210> 34
   <211> 12
   <212> DNA
   <213> Artificial Sequence
<220>
   <223> Antisense oligonucleotide
<400> 34
   gtctgatgga ga 12
<210> 35
   <211> 12
   <212> DNA
   <213> Artificial Sequence
<220>
   <223> Antisense oligonucleotide
<400> 35
   gtctgatgga ga 12
<210> 36
   <211> 20
   <212> DNA
   <213> Artificial Sequence
<220>
   <223> Antisense oligonucleotide
<400> 36
   gmumctmgma tmgmgamgma 20
<210> 37
   <211> 24
   <212> DNA
   <213> Artificial Sequence
<220>
   <223> Antisense oligonucleotide
<400> 37
   mgmumcmumg mamumgmgma mgma 24
<210> 38
   <211> 27
   <212> DNA
   <213> Artificial Sequence
<220>
   <223> Antisense oligonucleotide
<400> 38
   actgacacct aattgtattc acatgaa 27
<210> 39
   <211> 26
   <212> DNA
   <213> Artificial Sequence
<220>
   <223> Antisense oligonucleotide
<400> 39
   tgagcagcag ttgctaaatt agttca 26
<210> 40
   <211> 27
   <212> DNA
   <213> Artificial Sequence
<220>
   <223> Antisense oligonucleotide
<400> 40
   tctacctaca ttatatcata gctccta 27
<210> 41
   <211> 19
   <212> DNA
   <213> Artificial Sequence
<220>
   <223> Antisense oligonucleotide
<400> 41
   ttggtattca caagtgaaa 19
<210> 42
   <211> 19
   <212> DNA
   <213> Artificial Sequence
<220>
   <223> Antisense oligonucleotide
<400> 42
   ttgctaaatt agttcagat 19
<210> 43
   <211> 19
   <212> DNA
   <213> Artificial Sequence
<220>
   <223> Antisense oligonucleotide
<400> 43
   gcagcagcag ttgctaaat 19
<210> 44
   <211> 19
   <212> DNA
   <213> Artificial Sequence
<220>
   <223> Antisense oligonucleotide
<400> 44
   gcagttgcta aattagttc 19
<210> 45
   <211> 20
   <212> DNA
   <213> Artificial Sequence
<220>
   <223> Antisense oligonucleotide
<400> 45
   gccatgttgc ccagtccagt 20
<210> 46
   <211> 20
   <212> DNA
   <213> Artificial Sequence
<220>
   <223> Antisense oligonucleotide
<400> 46
   gggctctgct acttacttgc 20
<210> 47
   <211> 20
   <212> DNA
   <213> Artificial Sequence
<220>
   <223> Antisense oligonucleotide
<400> 47
   cccagtcttc agccttgtct 20
<210> 48
   <211> 21
   <212> DNA
   <213> Artificial Sequence
<220>
   <223> Antisense oligonucleotide
<400> 48
   gggtctctgt catatgttct t 21
<210> 49
   <211> 19
   <212> DNA
   <213> Artificial Sequence
<220>
   <223> Antisense oligonucleotide
<400> 49
   ttcctacctt ccctccata 19
<210> 50
   <211> 19
   <212> DNA
   <213> Artificial Sequence
<220>
   <223> Antisense oligonucleotide
<400> 50
   attcctacct tccctccat 19
<210> 51
   <211> 20
   <212> DNA
   <213> Artificial Sequence
<220>
   <223> Antisense oligonucleotide
<400> 51
   ccttagggtt gcagctaatt 20
<210> 52
   <211> 20
   <212> DNA
   <213> Artificial Sequence
<220>
   <223> Antisense oligonucleotide
<400> 52
   atcccagcta ctcaggaggc 20
<210> 53
   <211> 20
   <212> DNA
   <213> Artificial Sequence
<220>
   <223> Antisense oligonucleotide
<400> 53
   tctggctgag tgcagtggct 20
<210> 54
   <211> 20
   <212> DNA
   <213> Artificial Sequence
<220>
   <223> Antisense oligonucleotide
<400> 54
   cctgggagtt ggaggttgca 20
<210> 55
   <211> 21
   <212> DNA
   <213> Artificial Sequence
<220>
   <223> Antisense oligonucleotide
<400> 55
   cagatcccat gaagccaaga g 21
<210> 56
   <211> 21
   <212> DNA
   <213> Artificial Sequence
<220>
   <223> Antisense oligonucleotide
<400> 56
   ctgactgcca tcgagaagtg g 21
<210> 57
   <211> 19
   <212> DNA
   <213> Artificial Sequence
<220>
   <223> Antisense oligonucleotide
<400> 57
   gcccatctgc ttgcttgat 19
<210> 58
   <211> 19
   <212> DNA
   <213> Artificial Sequence
<220>
   <223> Antisense oligonucleotide
<400> 58
   atcctcacca cagtcttgt 19
<210> 59
   <211> 20
   <212> DNA
   <213> Artificial Sequence
<220>
   <223> Antisense oligonucleotide
<400> 59
   gcttacttcc tcctcccttt 20
<210> 60
   <211> 21
   <212> DNA
   <213> Artificial Sequence
<220>
   <223> Antisense oligonucleotide
<400> 60
   ccaggtgata ggagcagaac t 21
<210> 61
   <211> 20
   <212> DNA
   <213> Artificial Sequence
<220>
   <223> Antisense oligonucleotide
<400> 61
   accctccttc ctccctctct 20
<210> 62
   <211> 21
   <212> DNA
   <213> Artificial Sequence
<220>
   <223> Antisense oligonucleotide
<400> 62
   ccactctccc ttctgtcctc t 21
<210> 63
   <211> 20
   <212> DNA
   <213> Artificial Sequence
<220>
   <223> Antisense oligonucleotide
<400> 63
   cctccttcct ccctctctct 20
<210> 64
   <211> 20
   <212> DNA
   <213> Artificial Sequence
<220>
   <223> Antisense oligonucleotide
<400> 64
   gtctgtccca tcatgccagg 20
<210> 65
   <211> 20
   <212> DNA
   <213> Artificial Sequence
<220>
   <223> Antisense oligonucleotide
<400> 65
   tttctgatcc tgctgcctct 20
<210> 66
   <211> 15
   <212> DNA
   <213> Artificial Sequence
<220>
   <223> Antisense oligonucleotide
<400> 66
   accctccttc ctccc 15
<210> 67
   <211> 14
   <212> DNA
   <213> Artificial Sequence
<220>
   <223> Antisense oligonucleotide
<400> 67
   ctccttcctc cctc 14
<210> 68
   <211> 11
   <212> DNA
   <213> Artificial Sequence
<220>
   <223> Antisense oligonucleotide
<400> 68
   ctccttcctc c 11
<210> 69
   <211> 15
   <212> DNA
   <213> Artificial Sequence
<220>
   <223> Antisense oligonucleotide
<400> 69
   cttcctccct ctctc 15
<210> 70
   <211> 14
   <212> DNA
   <213> Artificial Sequence
<220>
   <223> Antisense oligonucleotide
<400> 70
   atcctgctgc ctct 14
<210> 71
   <211> 14
   <212> DNA
   <213> Artificial Sequence
<220>
   <223> Antisense oligonucleotide
<400> 71
   ctccttcctc cctc 14
<210> 72
   <211> 15
   <212> DNA
   <213> Artificial Sequence
<220>
   <223> Antisense oligonucleotide
<400> 72
   accctccttc ctccc 15
<210> 73
   <211> 20
   <212> DNA
   <213> Artificial Sequence
<220>
   <223> Antisense oligonucleotide
<400> 73
   gttctggctc tgctgtagga 20
<210> 74
   <211> 20
   <212> DNA
   <213> Artificial Sequence
<220>
   <223> Antisense oligonucleotide
<400> 74
   atgctcactc acttccggcg 20
<210> 75
   <211> 21
   <212> DNA
   <213> Artificial Sequence
<220>
   <223> Antisense oligonucleotide
<400> 75
   ctttgtggtg tcagcctttg t 21
<210> 76
   <211> 20
   <212> DNA
   <213> Artificial Sequence
<220>
   <223> Antisense oligonucleotide
<400> 76
   aggtgtgagc gcagggtaca 20
<210> 77
   <211> 20
   <212> DNA
   <213> Artificial Sequence
<220>
   <223> Antisense oligonucleotide
<400> 77
   ctcggttaca gcgcgtctct 20
<210> 78
   <211> 19
   <212> DNA
   <213> Artificial Sequence
<220>
   <223> Antisense oligonucleotide
<400> 78
   aactctccgg cgtttgcag 19
<210> 79
   <211> 19
   <212> DNA
   <213> Artificial Sequence
<220>
   <223> Antisense oligonucleotide
<400> 79
   tgttgtccca gttactcct 19
<210> 80
   <211> 20
   <212> DNA
   <213> Artificial Sequence
<220>
   <223> Antisense oligonucleotide
<400> 80
   gccaggagtt caagagcagc 20
<210> 81
   <211> 20
   <212> DNA
   <213> Artificial Sequence
<220>
   <223> Antisense oligonucleotide
<400> 81
   ccttgttggc acctcactgt 20
<210> 82
   <211> 20
   <212> DNA
   <213> Artificial Sequence
<220>
   <223> Antisense oligonucleotide
<400> 82
   gttgtgcggt taatactccc 20
<210> 83
   <211> 21
   <212> DNA
   <213> Artificial Sequence
<220>
   <223> Antisense oligonucleotide
<400> 83
   gtgagtctcg aagtggtagc t 21
<210> 84
   <211> 21
   <212> DNA
   <213> Artificial Sequence
<220>
   <223> Antisense oligonucleotide
<400> 84
   gactcttatc tacctgtggg a 21
<210> 85
   <211> 21
   <212> DNA
   <213> Artificial Sequence
<220>
   <223> Antisense oligonucleotide
<400> 85
   tccttctcct cttgtctctc c 21
<210> 86
   <211> 20
   <212> DNA
   <213> Artificial Sequence
<220>
   <223> Antisense oligonucleotide
<400> 86
   gccctgtgtt tctcctctcc 20
<210> 87
   <211> 21
   <212> DNA
   <213> Artificial Sequence
<220>
   <223> Antisense oligonucleotide
<400> 87
   actcccgtgg tctctgtgtc t 21
<210> 88
   <211> 20
   <212> DNA
   <213> Artificial Sequence
<220>
   <223> Antisense oligonucleotide
<400> 88
   tccagcaacc tcactctccc 20
<210> 89
   <211> 20
   <212> DNA
   <213> Artificial Sequence
<220>
   <223> Antisense oligonucleotide
<400> 89
   gcgtgggtgt ggtcagtgta 20
<210> 90
   <211> 20
   <212> DNA
   <213> Artificial Sequence
<220>
   <223> Antisense oligonucleotide
<400> 90
   accacctcct gctccagact 20
<210> 91
   <211> 21
   <212> DNA
   <213> Artificial Sequence
<220>
   <223> Antisense oligonucleotide
<400> 91
   cctctccctc tctctgtctc t 21
<210> 92
   <211> 19
   <212> DNA
   <213> Artificial Sequence
<220>
   <223> Antisense oligonucleotide
<400> 92
   atcttccctc tcacccttc 19
<210> 93
   <211> 21
   <212> DNA
   <213> Artificial Sequence
<220>
   <223> Antisense oligonucleotide
<400> 93
   cccttctgtc tgatcttccc t 21
<210> 94
   <211> 20
   <212> DNA
   <213> Artificial Sequence
<220>
   <223> Antisense Oligonucleotide
<400> 94
   tctgtgtcca cctgtctccc 20
<210> 95
   <211> 25
   <212> DNA
   <213> Homo sapiens
<400> 95
   aactggagct ggggtgtctg tttca 25
<210> 96
   <211> 25
   <212> DNA
   <213> Artificial Sequence
<220>
   <223> Forward Primer Sequence
<400> 96
   ccatcagacg acatccctta acaaa 25
<210> 97
   <211> 31
   <212> DNA
   <213> Artificial Sequence
<220>
   <223> Reverse Primer Sequence
<400> 97
   acattatatc atagctccta aaggagatgc a 31
<210> 98
   <211> 16
   <212> DNA
   <213> Artificial Sequence
<220>
   <223> Reporter Sequence
<400> 98
   cagagtttca attccc 16
<210> 99
   <211> 12
   <212> DNA
   <213> Artificial Sequence
<220>
   <223> Antisense Oligonucleotide
<400> 99
   gctagtctgt tg 12

## Description

Instances of the disclosure comprise oligonucleotides modulating expression and/or function of a Sirtuin (SIRT) and associated molecules.

### BACKGROUND

DNA-RNA and RNA-RNA hybridization are important to many aspects of nucleic acid function including DNA implication, transcription, and translation. Hybridization is also central to a variety of technologies that either detect a particular nucleic acid or alter its expression. Antisense nucleotides, for example, disrupt gene expression by hybridizing to target RNA, thereby interfering with RNA splicing, transcription, translation, and replication. Antisense DNA has the added feature that DNA-RNA hybrids serve as a substrate for digestion by ribonuclease H, an activity that is present in most cell types. Antisense molecules can be delivered into cells, as is the case for oligodeoxynucleotides (ODNs), or they can be expressed from endogenous genes as RNA molecules. The FDA recently approved an antisense drug, VTTRAVENE™ (for treatment of cytomegalovirus retinitis), reflecting that antisense has therapeutic utility.
DATABASE GENBANK [Online] NCBI; 26 June 2007, anonymous: "Homo sapiens ankyrin repeat domain 24 (ANKRD24), mRNA", retrieved from http://ncbi.nlm.nih.gov/genbank/, database accession no NM_133475.
DATABASE ENA [Online] EMBL; 26 February 2004, anonymous: "CM4-IT0042-131200-611-h08 IT0042 Homo sapiens cDNA, mRNA sequence.", retrieved from EBI accession no. EMBL:BF772662, database accession no. BF772662.
Mostoslavsky et al. (Cell, vol. 124, no. 2, 27 January 2006, pages 315-329) relates to genomic instability and aging-like phenotype in the absence of mammalian SIRT6.

### SUMMARY

The invention is defined by the claims. Those aspects/instances of the present disclosure which constitute the invention are defined by the claims.

In one instance, the disclosure provides methods for inhibiting the action of a natural antisense transcript by using antisense oligonucleotide(s) targeted to any region of the natural antisense transcript resulting in up-regulation of the corresponding sense gene. It is also contemplated herein that inhibition of the natural antisense transcript can be achieved by siRNA, ribozymes and small molecules, which are considered to be within the scope of the present disclosure.

One instance provides a method of modulating function and/or expression of a Sirtuin (SIRT) polynucleotide in patient cells or tissues in vivo or in vitro comprising contacting said cells or tissues with an antisense oligonucleotide 5 to 30 nucleotides in length wherein said oligonucleotide has at least 50% sequence identity to a reverse complement of a polynucleotide comprising 5 to 30 consecutive nucleotides within nucleotides 1 to 1028 of SEQ ID NO: 5 or nucleotides 1 to 429 of SEQ ID NO: 6, or nucleotides 1 to 156 of SEQ ID NO: 7 or nucleotides 1 to 593 of SEQ ID NO:8, 1 to 373 of SEQ ID NO: 9, 1 to 1713 of SEQ ID NO: 10, 1 to 660 of SEQ ID NO: 11, 1 to 589 of SEQ ID NO: 12, 1 to 428 of SEQ ID NO: 13 and 1 to 4041 of SEQ ID NO: 14 thereby modulating function and/or expression of the Sirtuin (SIRT) polynucleotide in patient cells or tissues *in vivo* or *in vitro.*

In another instance, an oligonucleotide targets a natural antisense sequence of a Sirtuin (SIRT) polynucleotide, for example, nucleotides set forth in SEQ ID NO: 5 to 14, and any variants, alleles, homologs, mutants, derivatives, fragments and complementary sequences thereto. Examples of antisense oligonucleotides are set forth as SEQ ID NOS: 15 to 94.

Another instance, provides a method of modulating function and/or expression of a Sirtuin (SIRT) polynucleotide in patient cells or tissues in vivo or in vitro comprising contacting said cells or tissues with an antisense oligonucleotide 5 to 30 nucleotides in length wherein said oligonucleotide has at least 50% sequence identity to a reverse complement of the an antisense of the Sirtuin (SIRT) polynucleotide; thereby modulating function and/or expression of the Sirtuin (SIRT) polynucleotide in patient cells or tissues in vivo or in vitro.

Another instance, provides a method of modulating function and/or expression of a Sirtuin (SIRT) polynucleotide in patient cells or tissues in vivo or in vitro comprising contacting said cells or tissues with an antisense oligonucleotide 5 to 30 nucleotides in length wherein said oligonucleotide has at least 50% sequence identity to an antisense oligonucleotide to a Sirtuin (SIRT) antisense polynucleotide; thereby modulating function and/or expression of the Sirtuin (SIRT) polynucleotide in patient cells or tissues in vivo or in vitro.

In one instance, a composition comprises one or more antisense oligonucleotides which bind to sense and/or antisense Sirtuin (SIRT) polynucleotides.

In another instance, the oligonucleotides comprise one or more modified or substituted nucleotides.

In another instance, the oligonucleotides comprise one or more modified bonds.

In yet another instance, the modified nucleotides comprise modified bases comprising phosphorothioate, methylphosphonate, peptide nucleic acids, 2'-O-methyl, fluoro- or carbon, methylene or other locked nucleic acid (LNA) molecules. Preferably, the modified nucleotides are locked nucleic acid molecules, including α-L-LNA.

In another instance, the oligonucleotides are administered to a patient subcutaneously, intramuscularly, intravenously or intraperitoneally.

In another instance, the oligonucleotides are administered in a pharmaceutical composition. A treatment regimen comprises administering the antisense compounds at least once to patient; however, this treatment can be modified to include multiple doses over a period of time. The treatment can be combined with one or more other types of therapies.

In another instance, the oligonucleotides are encapsulated in a liposome or attached to a carrier molecule (e.g. cholesterol, TAT peptide).

Other aspects are described *infra,*

### BRIEF DESCRIPTION OF THE DRAWINGS

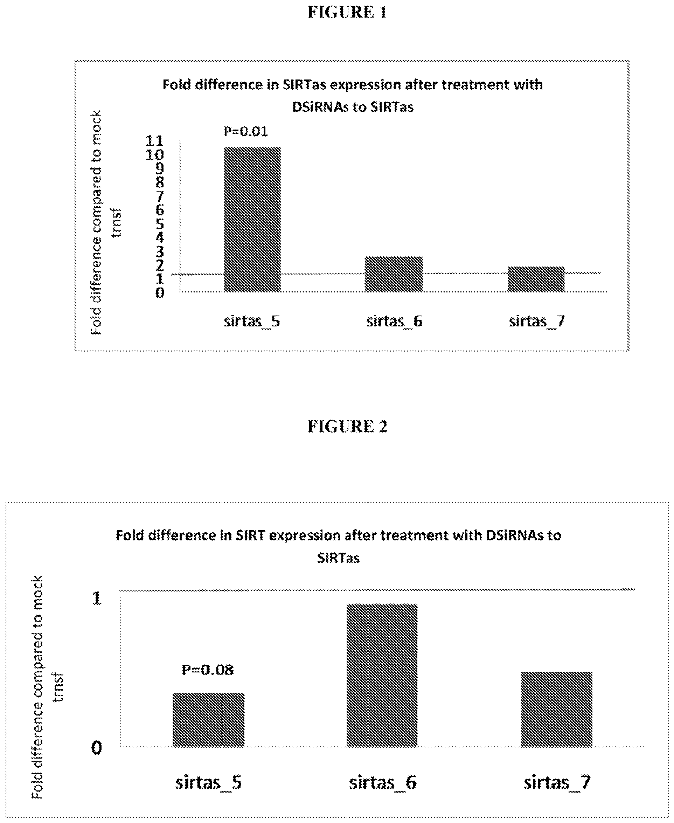

Figure 1 and 2 show Real time PCR results of oligonucleotides designed to SIRT antisense CV396200. The results show that the levels of the SIRT1 mRNA in HepG2 cells are significantly increased 48 h after treatment with one of the siRNAs designed to sirtas (sirtas_5, P=0.01). In the same samples the levels of sirtas RNA were significantly decreased after treatment with sirtas_5, but unchanged after treatment with sirtas_6 and sirtas_7, which also had no effect on the SIRT1 mRNA levels (Fig. 2). sirtas_5, sirtas_6 and sirtas_7 correspond to SEQ ID NOs: 38, 39 and 40 respectively.

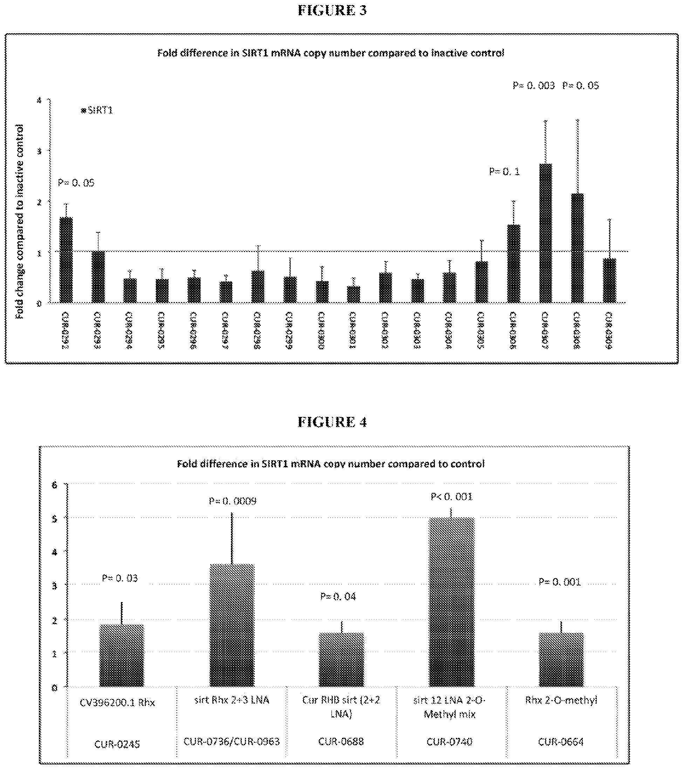

Figure 3 shows results for the oligonucleotide walk across the SIRT antisense. Real time PCR results show that the levels of the SIRT1 mRNA in HepG2 cells are significantly increased 48 h after treatment with three of the antisense oligonucleotides designed to sirtas. CUR-0392 to CUR-0309 correspond to SEQ ID NOs: 15 to 32 respectively.

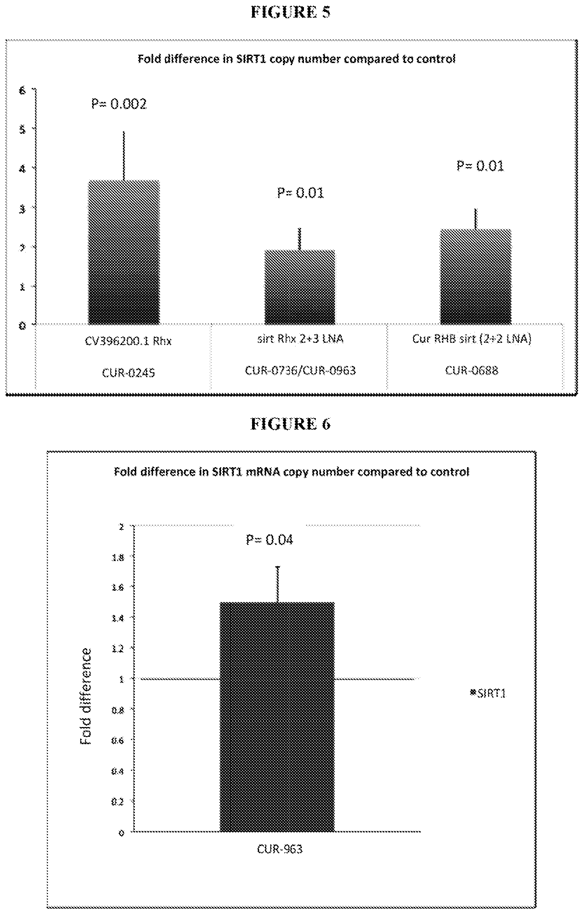

Figure 4 and 5 show results for PS, LNA and 2'O Me Modified oligonucleotides in HepG2 (Fig. 4) and Vero76 (Fig. 5) cells. Real time PCR results show that the levels of the SIRT1 mRNA in HepG2 cells are significantly increased 48 h after treatment with PS, LNA, 2'O Me and 2'O Me mixmer designed antisense oligonucleotides to SIRT1 antisense. Levels of SIRT1 mRNA in Vero cells also increased 48 hours after treatment with PS and LNA modified antisense oligonucleotides to SIRT1 antisense. Bars denoted as CUR-0245, CUR-0736, CUR 0688, CUR-0740 and CUR-0664 correspond to SEQ ID NOs: 33 to 37 respectively.
Figure 6 shows PCR results of Monkey Fat Biopsies. Real time PCR results show an increase in SIRT1 mRNA levels in fat biopsies from monkeys dosed with CUR-963, an oligonucleotide designed to SIRT1 antisense CV396200.1. CUR-963 corresponds to SEQ ID NO: 34,

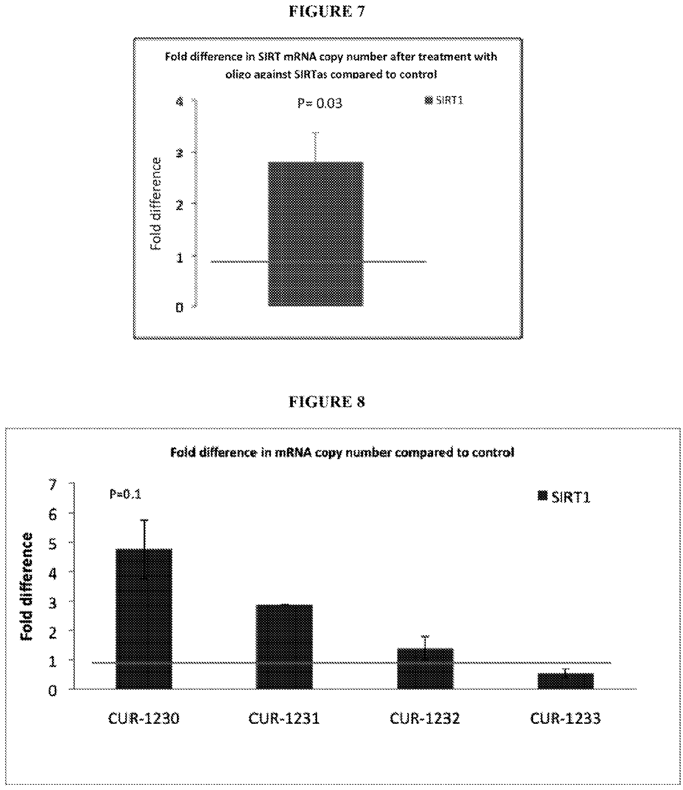

Figure 7 shows PCR results of primary monkey liver hepatocytes. Real time PCR results show an increase in SIRT1 mRNA levels after treatment with an oligonucleotide against SIRT1 antisense. Bar denoted as CUR-0245 corresponds to SEQ ID NO: 33.
Figure 8 shows results for oligonucleotides designed to SIRT antisense CV396200. Real Time PCR results show that levels of SIRT1 mRNA in HepG2 cells are significantly increased in one of the oligonucleotides designed to SIRT1 antisense CV396200. The bars denoted as CUR-1230, CUR-1231, CUR-1232 and CUR-1233 correspond to SEQ ID NOs: 41 to 44.

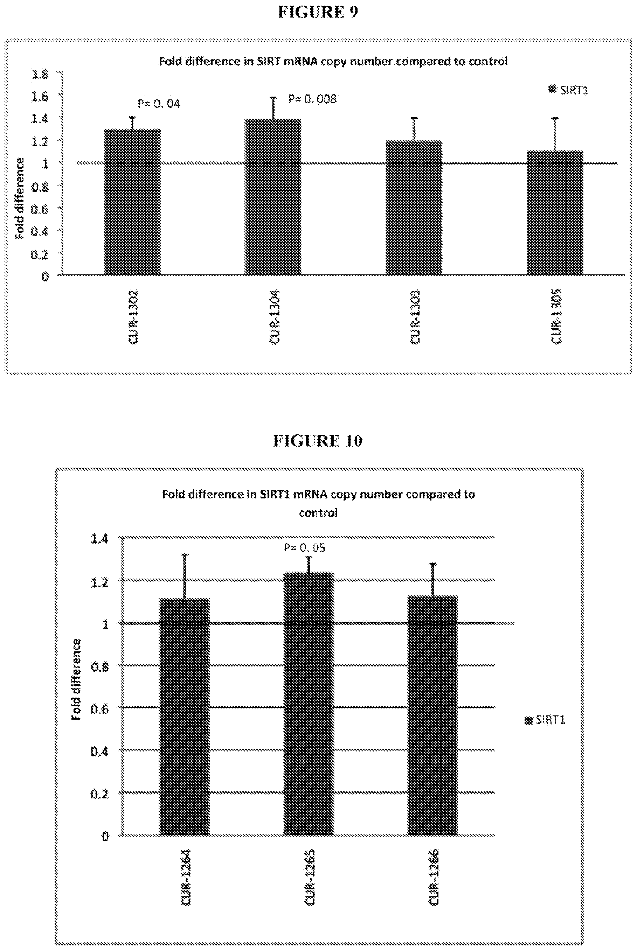

Figure 9 shows results for oligonucleotides designed to SIRT antisense CV428275. Real Time PCR results show that levels of SIRT1 mRNA in HepG2 cells are significantly increased in two of the oligonucleotides designed to SIRT1 antisense CV428275. The bars denoted as CUR-1302, CUR-1304, CUR-1303 and CUR-1305 correspond to SEQ ID NOs: 45 to 48.
Figure 10 shows Real time PCR results. The results show that a significant increase in SIRT1 mRNA levels in HepG2 cells 48 hours after treatment with one of the oligonucleotides designed to SIRT antisense BE717453. The bars denoted as CUR-1264, CUR1265 and CUR-1266 correspond to SEQ ID NOs: 49 to 51 respectively.

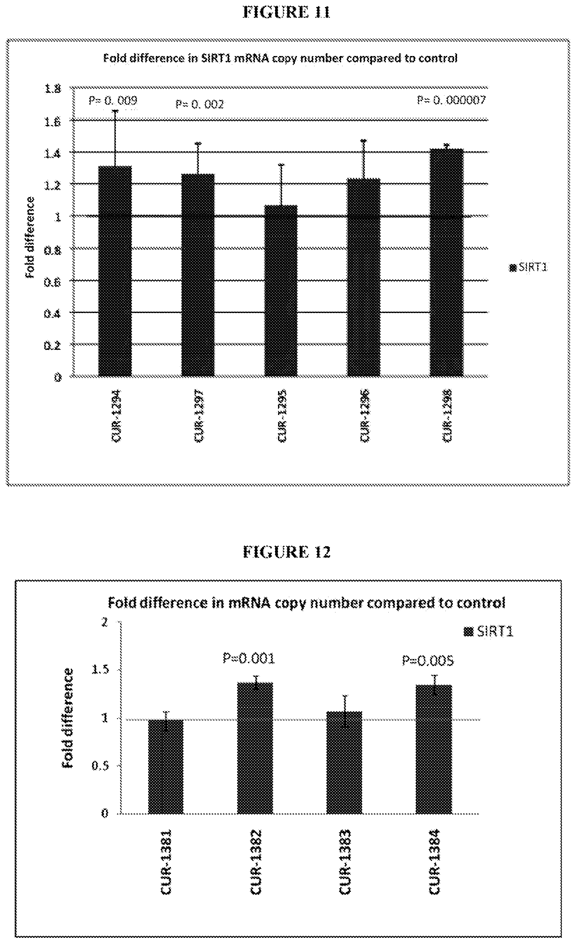

Figure 11 shows Real time PCR results. The results show that show that the levels of the SIRT1 mRNA in HepG2 cells are significantly increased 48 h after treatment with three of the oligonucleotides designed to SIRT1 antisense AV718812. The bars denoted as CUR-1294, CUR-1297, CUR-1295, CUR-1296 and CUR-1298 correspond to SEQ ID NOs: 52 to 56 respectively.
Figure 12 is a graph of real time PCR results showing the fold change + standard deviation in SIRT1 mRNA after treatment of HepG2 cells with phosphorothioate oligonucleotides introduced using Lipofectamine 2000, as compared to control. Real time PCR results show that the levels of SIRT1 mRNA are significantly increased in HepG2 cells 48 h after treatment with two of the oligos designed to SIRT1 antisense AW169958. Bars denoted as CUR-1381, CUR-1382, CUR-1383 and CUR-1384 correspond to samples treated with SEQ ID NOS: 57 to 60 respectively.

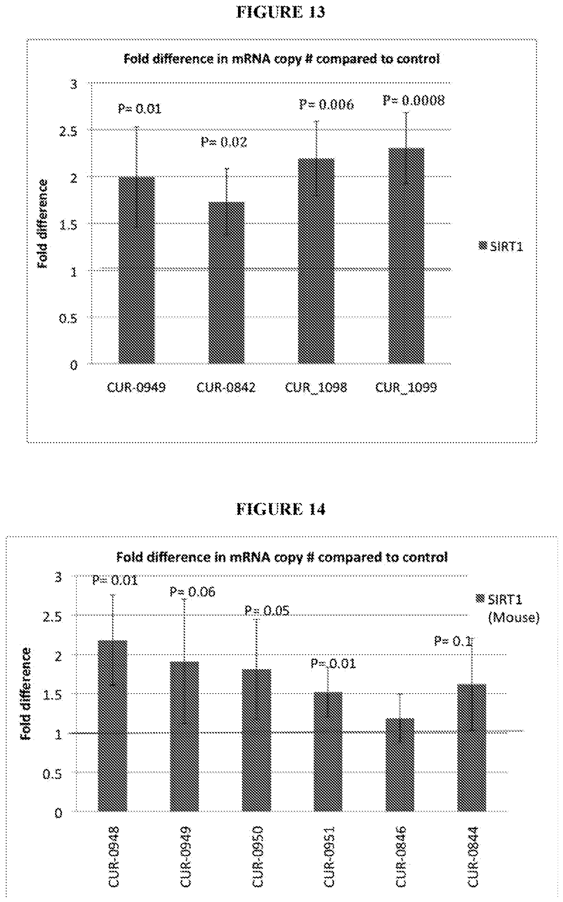

Figure 13 is a graph of real time PCR results showing the fold change + standard deviation in SIRT1 mRNA after treatment of 3T3 cells with phosphorothioate oligonucleotides introduced using Lipofectamine 2000, as compared to control Real time PCR results show that the levels of SIRT1 mRNA are significantly increased in 3T3 cells 48 h after treatment with three of the oligonucleotides designed to SIRT1 mouse antisense AK044604. Bars denoted as CUR-0949, CUR-0842, CUR-1098 and CUR-1099 correspond to samples treated with SEQ ID NOS: 67, 61, 71 and 72 respectively.
Figure 14 is a graph of real time PCR results showing the fold change + standard deviation in SIRT1 mRNA after treatment of 3T3 cells with phosphorothioate oligonucleotides introduced using Lipofectamine 2000, as compared to control. Real time PCR results show that the levels of SIRT1 mRNA are significantly increased in 3T3 cells 48 h after treatment with five of the oligonucleotides designed to SIRT1 mouse antisense AK044604. Bars denoted as CUR-0948, CUR-0949, CUR-C950, CUR-0951, CUR-0846, and CUR-0844 correspond to samples treated with SEQ ID NOS: 66 to 69, 65 and 63 respectively.

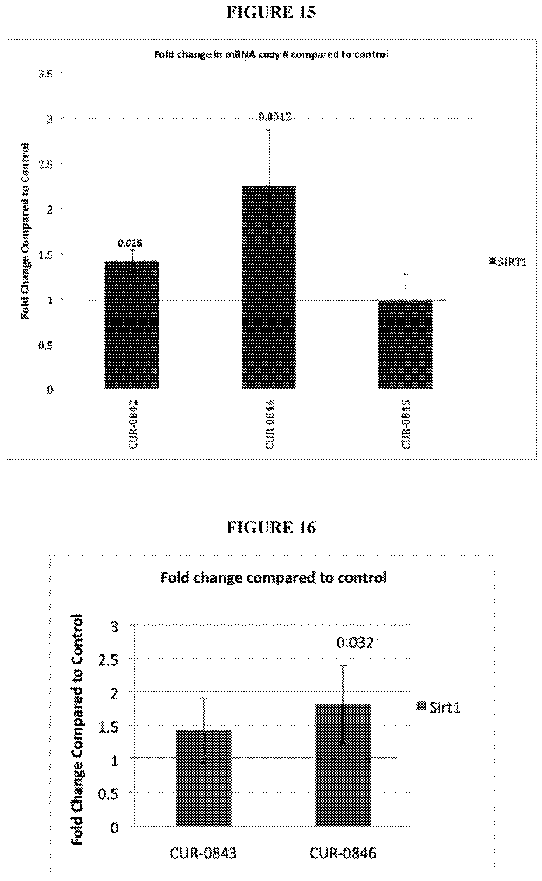

Figure 15 is a graph of real time PCR results showing the fold change + standard deviation in SIRT1 mRNA after treatment of 3T3 cells with phosphorothioate oligonucleotides introduced using Lipofectamine 2000, as compared to control. Real time PCR results show that the levels of SIRT1 mRNA are significantly increased in HepG2 cells 48 h after treatment with two of the oligonucleotides designed to SIRT1 mouse antisense AK044604. Bars denoted as CUR-0842, CUR-0844, and CUR-0845 correspond to samples treated with SEQ ID NOS: 61, 63 and 64 respectively.
Figure 16 is a graph of real time PCR results showing the fold change + standard deviation in SIRT1 mRNA after treatment of 3T3 cells with phosphorothioate oligonucleotides introduced using Lipofectamine 2000, as compared to control Real time PCR results show that the levels of SIRT1 mRNA are significantly increased in HepG2 cells 48 h after treatment with two of the oligonucleotides designed to SIRT1 mouse antisense AK044604, Bars denoted as CUR-0843, CUR-0846 correspond to samples treated with SEQ ID NOS: 62 and 65 respectively.

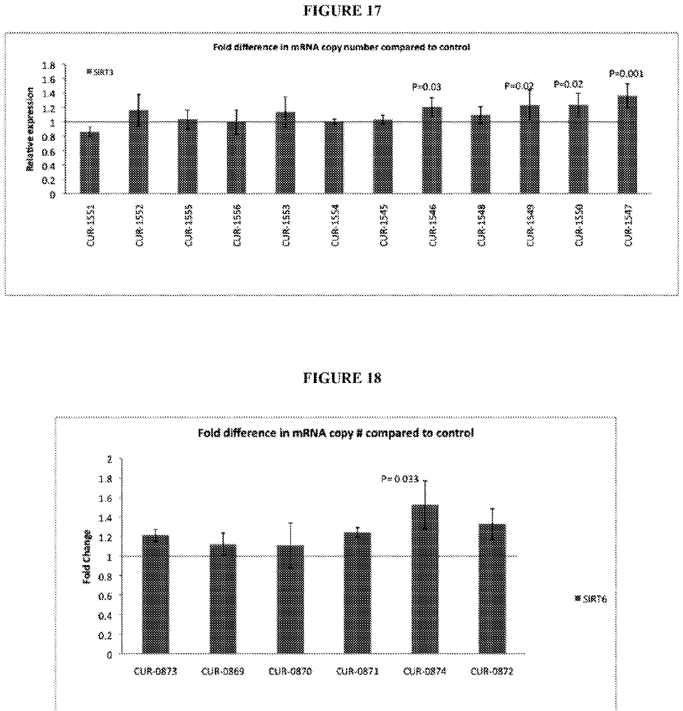

Figure 17 is a graph of real time PCR results showing the fold change + standard deviation in Sirtuin3 mRNA after treatment of HepG2 cells with phosphorothioate oligonucleotides introduced using Lipofectamine 2000, as compared to control. RT PCR results show that sirt3 levels in HepG2 cells are increased 48 hours after treatment with phosphorothioate antisense oligonucleotides designed to sirt3 antisense Hs.683117 (CUR-1545-1550). Bars denoted as CUR-0551, CUR-1552, CUR-1555, CUR-1556, CUR-1553, CUR-1554, CUR-1545, CUR-1546, CUR-1548, CUR-1549, CUR-1550 and CUR-1547, correspond to samples treated with SEQ ID NOS: 73 to 84 respectively.
Figure 18 is a graph of real time PCR results showing the fold change + standard deviation in SIRT6 mRNA after treatment of HepG2 cells with phosphorothioate oligonucleotides introduced using Lipofectamine 2000, as compared to control. Real time PCR results show that the levels of SIRT6 mRNA in HepG2 cells are significantly increased 48h after treatment with one of the oliogs designed to SIRT6 antisense NM_133475. Bars denoted as CUR-0873, CUR-0869 to CUR-0871, CUR-0874 and CUR-C872, correspond to samples treated with SEQ ID NOS: 85 to 90 respectively.

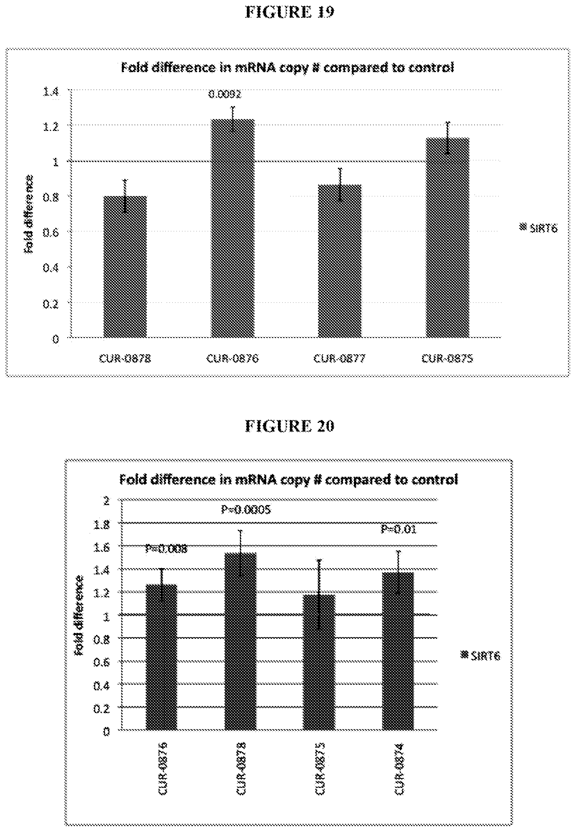

Figure 19 is a graph of real time PCR results showing the fold change + standard deviation in SIRT6 mRNA after treatment of HepG2 cells with phosphorothioate oligonucleotides introduced using Lipofectamine 2000, as compared to control. Real time PCR results show that the levels of SIRT6 mRNA in HepG2 cells are significantly increased 48h after treatment with one of the oliogs designed to SIRT6 antisense bf772662. Bars denoted as CUR-0878, CUR-0876, CUR-0877 and CUR-0875, correspond to samples treated with SEQ ID NOS: 91 to 94 respectively.
Figure 20 is a graph of real time PCR results showing the fold change + standard deviation in SIRT6 mRNA after treatment of DBS-FCL-1 cells with phosphorothioate oligonucleotides introduced using Lipofectamine 2000, as compared to control. Real time PCR results show that the levels of SIRT6 mRNA in DBS-FCL-1 cells are significantly increased 48h after treatment with two of the oliogs designed to SIRT6 antisense bf772662 and one oligo designed to NM_133475. Bars denoted as CUR-0876, CUR-0878, CUR-0875 and CUR-0874, correspond to samples treated with SEQ ID NOS: 92, 91, 94 and 89 respectively.

### Sequence Listing Description

SEQ ID NO: 1: Homo sapiens sirtuin (silent mating type information regulation 2 homolog) 1 (S. cerevisiae) (SIRT1), mRNA (NCBI Accession Number: NM_012238.3)
SEQ ID NO: 2: Mas musculus sirtuin 1 (silent mating type information regulation 2, homolog) 1 (S. cerevisiae) (SIRT1) mRNA (NCBI Accession Number: NM_001159589)
SEQ ID NO: 3: Homo sapiens sirtuin (silent mating type information regulation 2 homolog) 3 (S. cerevisiae) (SIRT3), transcript variant 1, mRNA (NCBI Accession No.: NM_012239.5).
SEQ ID NO: 4: Homo sapiens sirtuin 6 (SIRT6), transcript variant 1, mRNA (NCBI Accession No.: NM_016539).
SEQ ID NO: 5: Expanded natural antisense sequence (CV396200 - expanded)
SEQ ID NO: 6: Natural Antisense sequence (CV428275)
SEQ ID NO: 7: Natural Antisense Sequence (BE717453)
SEQ ID NO: 8: Natural Antisense Sequence (AV718812)
SEQ ID NO: 9: Natural SIRT1 antisense sequence (AW169958)
SEQ ID NO: 10 Natural SIRT1 mouse antisense sequence (AK044604)
SEQ ID NO: 11: Natural SIRT3 antisense sequence (Hs.6831117)
SEQ ID NO: 12: Natural SIRT3 antisense sequence (DA645474)
SEQ ID NO: 13: Natural SIRT6 antisense sequence (BF772662)
SEQ ID NO: 14: Natural SIRT6 antisense sequence (ANKRD24)
SEQ ID NOs: 15 to 94: Antisense oligonucleotides.* indicates phosphothioate bond, + indicates LNA and m indicates 2'O Me
SEQ ID NO: 95 to 98- SEQ ID NO: 95 correspond to the exon 4 of the SIRT1 natural antisense CV396200, SEQ ID NO: 96, 97 and 98 correspond to the forward primer sequence, reverse primer sequence and the reporter sequence respectively.
SEQ ID NO: 99 corresponds to CUR 962, * indicates phosphothioate bond and + indicates LNA.

### DETAILED DESCRIPTION

Several aspects of the disclosure are described below with reference to example applications for illustration. It should be understood that numerous specific details, relationships, and methods are set forth to provide a full understanding of the disclosure. One having ordinary skill in the relevant art, however, will readily recognize that the disclosure can be practiced without one or more of the specific details or with other methods. The present disclosure is not limited by the ordering of acts or events, as some acts may occur in different orders and/or concurrently with other acts or events. Furthermore, not all illustrated acts or events are required to implement a methodology in accordance with the present disclosure.

All genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes disclosed herein, which in some instances relate to mammalian nucleic acid and amino acid sequences are intended to encompass homologous and/or orthologous genes and gene products from other animals including, but not limited to other mammals, fish, amphibians, reptiles, and birds. In instances, the genes or nucleic acid sequences are human.

### Definitions

The terminology used herein is for the purpose of describing particular instances only and is not intended to be limiting of the disclosure. As used herein, the singular forms "a", "an" and "the" are intended to include the plural forms as well, unless the context clearly indicates otherwise. Furthermore, to the extent that the terms "including", "includes", "having", "has", "with", or variants thereof arc used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably with in 2-fold, of a value. Where particular values are described in the application and claims, unless otherwise stated the term "about" meaning within an acceptable error range for the particular value should be assumed.

As used herein, the term "mRNA" means the presently known mRNA transcript(s) of a targeted gene, and any further transcripts which may be elucidated.

By "antisense oligonucleotides" or "antisense compound" is meant an RNA or DNA molecule that binds to another RNA or DNA (target RNA, DNA). For example, if it is an RNA oligonucleotide it binds to another RNA target by means of RNA-RNA interactions and alters the activity of the target RNA. An antisense oligonucleotide can upregulate or downregulate expression and/or function of a particular polynucleotide. The definition is meant to include any foreign RNA or DNA molecule which is useful from a therapeutic, diagnostic, or other viewpoint. Such molecules include, for example, antisense RNA or DNA molecules, interference RNA (RNAi), micro RNA, decoy RNA molecules, siRNA, enzymatic RNA, therapeutic editing RNA and agonist and antagonist RNA, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds may be introduced in the form of single-stranded, double-stranded, partially single-stranded, or circular oligomeric compounds.

In the context of this disclosure, the term "oligonucleotide" refers to an oligomer or polymer of ribonucleic acid (RNA) or deoxyribonucleic acid (DNA) or mimetics thereof. The term "oligonucleotide", also includes linear or circular oligomers of natural and/or modified monomers or linkages, including deoxyribomicleosides, ribonucleosides, substituted and alpha-anomeric forms thereof, peptide nucleic acids (PNA), locked nucleic acids (LNA), phosphorothioate, methylphosphonate, and the like. Oligonucleotides are capable of specifically binding to a target polynucleotide by way of a regular pattern of monomer-to-monomer interactions, such as Watson-Crick type of base pairing, Hoögsteen or reverse Hoögsteen types of base pairing, or the like.

The oligonucleotide may be "chimeric", that is, composed of different regions. In the context of this disclosure 'chimeric" compounds are oligonucleotides, which contain two or more chemical regions, for example, DNA region(s), RNA region(s), PNA region(s) etc. Each chemical region is made up of at least one monomer unit, i.e., a nucleotide in the case of an oligonucleotides compound. These oligonucleotides typically comprise at least one region wherein the oligonucleotide is modified in order to exhibit one or more desired properties. The desired properties of the oligonucleotide include, but are not limited, for example, to increased resistance to nuclease degradation, increased cellular uptake, and/or increased binding affinity for the target nucleic acid. Different regions of the oligonucleotide may therefore have different properties. The chimeric oligonucleotides of the present disclosure can be formed as mixed structures of two or more oligonucleotides, modified oligonucleotides, oligonucleosides and/or oligonucleotide analogs as described above.

The oligonucleotide can be composed of regions that can be linked in "register" that is, when the monomers are linked consecutively, as in native DNA, or linked via spacers. The spacers are intended to constitute a covalent "bridge" between the regions and have in cases a length not exceeding about 100 carbon atoms. The spacers may carry different functionalities, for example, having positive or negative charge, carry special nucleic acid binding properties (intercalators, groove binders, toxins, fluorophors etc.), being lipophilic, inducing special secondary structures like, for example, alanine containing peptides that induce alpha-helices.

As used herein "Sirtuins (SIRT)s" are inclusive of all family members, mutants, alleles, fragments, species, coding and noncoding sequences, sense and antisense polynucleotide strands, etc.

As used herein, the words Sirtuin1, SIRT1, sirtuin, silent mating type information regulation 2 homolog 1, hSIR2, hSIRT1, NAD-dependent deacetylase sirtuin-1, SIR2L1, SIR2-like protein 1, are considered the same in the literature and are used interchangeably in the present application.

As used herein, the words 'Sirtuin 3', Sirtuin3, Sirtuin-3, SIRT3, SIRT-3, hSIRT3, NAD-dependent deacetylase sirtuin-3, mitochondrial, SIR2L3, SIR2-like protein 3 are used interchangeably in the present application.

As used herein, the words 'Sirtuin 6', Sirtuin6, Sirtuin-6, SIRT6, SIRT-6, NAD-dependent deacetylase sirtuin-6, SIR2L6, SIR2-like protein 6 are considered the same in the literature and are used interchangeably in the present application.

As used herein, the term "oligonucleotide specific for" or "oligonucleotide which targets" refers to an oligonucleotide having a sequence (i) capable of forming a stable complex with a portion of the targeted gene, or (ii) capable of forming a stable duplex with a portion of a mRNA transcript of the targeted gene. Stability of the complexes and duplexes can be determined by theoretical calculations and/or in vitro assays. Exemplary assays for determining stability of hybridization complexes and duplexes are described in the Examples below.

As used herein, the term "target nucleic acid" encompasses DNA, RNA (comprising premRNA and mRNA) transcribed from such DNA, and also cDNA derived from such RNA, coding, noncoding sequences, sense or antisense polynucleotides. The specific hybridization of an oligomeric compound with its target nucleic acid interferes with the normal function of the nucleic acid. This modulation of function of a target nucleic acid by compounds, which specifically hybridize to it, is generally referred to as "antisense". The functions of DNA to be interfered include, for example, replication and transcription. The functions of RNA to be interfered, include all vital functions such as, for example, translocation of the RNA to the site of protein translation, translation of protein from the RNA, splicing of the RNA to yield one or more mRNA species, and catalytic activity which may be engaged in or facilitated by the RNA. The overall effect of such interference with target nucleic acid function is modulation of the expression of an encoded product or oligonucleotides.

RNA interference "RNAi" is mediated by double stranded RNA (dsRNA) molecules that have sequence-specific homology to their "target" nucleic acid sequences. In certain instances of the present disclosure, the mediators are 5-25 nucleotide "small interfering" RNA duplexes (siRNAs). The siRNAs are derived from the processing of dsRNA by an RNase enzyme known as Dicer. siRNA duplex products are recruited into a multi-protein siRNA complex termed RISC (RNA Induced Silencing Complex). Without wishing to be bound by any particular theory, a RISC is then believed to be guided to a target nucleic acid (suitably mRNA), where the siRNA duplex interacts in a sequence-specific way to mediate cleavage in a catalytic fashion. Small interfering RNAs that can be used in accordance with the present disclosure can be synthesized and used according to procedures that are well known in the art and that will be familiar to the ordinarily skilled artisan. Small interfering RNAs for use in the methods of the present disclosure suitably comprise between about 1 to about 50 nucleotides (nt). In examples of non limiting instances, siRNAs can comprise about 5 to about 40 nt, about 5 to about 30 nt, about 10 to about 30 nt, about 15 to about 25 nt, or about 20-25 nucleotides.

Selection of appropriate oligonucleotides is facilitated by using computer programs that automatically align nucleic acid sequences and indicate regions of identity or homology. Such programs are used to compare nucleic acid sequences obtained, for example, by searching databases such as GenBank or by sequencing PCR products. Comparison of nucleic acid sequences from a range of species allows the selection of nucleic acid sequences that display an appropriate degree of identity between species. In the case of genes that have not been sequenced, Southern blots are performed to allow a determination of the degree of identity between genes in target species and other species. By performing Southern blots at varying degrees of stringency, as is well known in the art, it is possible to obtain an approximate measure of identity. These procedures allow the selection of oligonucleotides that exhibit a high degree of complementarity to target nucleic acid sequences in a subject to be controlled and a lower degree of complementarity to corresponding nucleic acid sequences in other species. One skilled in the art will realize that there is considerable latitude in selecting appropriate regions of genes for use in the present disclosure.

By "enzymatic RNA" is meant an RNA molecule with enzymatic activity. Enzymatic nucleic acids (ribozymes) act by first binding to a target RNA Such binding occurs through the target binding portion of an enzymatic nucleic acid which is held in close proximity to an enzymatic portion of the molecule that acts to cleave the target RNA. Thus, the enzymatic nucleic acid first recognizes and then binds a target RNA through base pairing, and once bound to the correct site, acts enzymatically to cut the target RNA.

By "decoy RNA" is meant an RNA molecule that mimics the natural binding domain for a ligand. The decoy RNA therefore competes with natural binding target for the binding of a specific ligand. For example, it has been shown that over-expression of HIV trans-activation response (TAR) RNA can act as a "decoy" and efficiently binds HIV tat protein, thereby preventing it from binding to TAR sequences encoded in the HIV RNA. This is meant to be a specific example. Those in the art will recognize that this is but one example, and other instances can be readily generated using techniques generally known in the art.

As used herein, the term "monomers" typically indicates monomers linked by phosphodiester bonds or analogs thereof to form oligonucleotides ranging in size from a few monomeric units, e.g., from about 3-4, to about several hundreds of monomeric units. Analogs of phosphodiester linkages include: phosphorothioate, phosphorodithioate, methylphosphomates, phosphoroselenoate, phosphoramidate, and the like, as more fully described below.

The term "nucleotide" covers naturally occurring nucleotides as well as normaturally occurring nucleotides. It should be clear to the person skilled in the art that various nucleotides which previously have been considered "non-naturally" occurring" have subsequently been found in nature. Thus, "nucleotides" includes not only the known purine and pyrimidine heterocycles-containing molecules, but also heterocyclic analogues and tautomers thereof Illustrative examples of other types of nucleotides are molecules containing adenine, guanine, thymine, cytosine, uracil, purine, xanthine, diaminopurirte, 8-oxo- N6-methyladenine, 7-deazaxanthine, 7-deazaguanine, N4,N4-ethanocytosin, N6,N6-ethano-2,6- diaminopurine, 5-methylcytosine, 5-(C3-C6)-alkynylcytosine, 5-fluorouracil, 5-bromouracil, pseudoisocytosine, 2-hydroxy-5-methyl-4-triazolopyridin, isocytosine, isoguanin, inosine and the "non-naturally occurring" nucleotides described in U.S. Pat No. 5,432,272. The term "nucleotide" is intended to cover every and all of these examples as well as analogues and tautomers thereof. Especially interesting nucleotides are those containing adenine, guanine, thymine, cytosine, and uracil, which are considered as the naturally occurring nucleotides in relation to therapeutic and diagnostic application in humans. Nucleotides include the natural 2'-deoxy and 2'- hydroxyl sugars, e.g., as described in Kornberg and Baker, DNA Replication, 2nd Ed. (Freeman, San Francisco, 1992) as well as their analogs.

"Analogs" in reference to nucleotides includes synthetic nucleotides having modified base moieties and/or modified sugar moieties. Such analogs include synthetic nucleotides designed to enhance binding properties, e.g., duplex or triplex stability, specificity, or the like.

As used herein, "hybridization" means the pairing of substantially complementary strands of oligomeric compounds. One mechanism of pairing involves hydrogen bonding, which may be Watson-Crick, Hoögsteen or reversed Hoögsteen hydrogen bonding, between complementary nucleoside or nucleotide bases (nucleotides) of the strands of oligomeric compounds. For example, adenine and thymine are complementary nucleotides which pair through the formation of hydrogen bonds. Hybridization can occur under varying circumstances.

An antisense compound is "specifically hybridizable" when binding of the compound to the target nucleic acid interferes with the normal function of the target nucleic acid to cause a modulation of function and/or activity, and there is a sufficient degree of complementarity to avoid non-specific binding of the antisense compound to non-target nucleic acid sequences under conditions in which specific binding is desired, i.e., under physiological conditions in the case of in vivo assays or therapeutic treatment, and under conditions in which assays arc performed in the case of in vitro assays.

As used herein, the phrase "stringent hybridisation conditions" or "stringent conditions" refers to conditions under which a compound of the disclosure will hybridize to its target sequence, but to a minimal number of other sequences. Stringent conditions are sequence-dependent, and will be different in different circumstances and in the context of this disclosure, "stringent conditions" under which oligomeric compounds hybridize to a target sequence are determined by the nature and composition of the oligomeric compounds and the assays in which they are being investigated. In general, stringent hybridization conditions comprise low concentrations (<0.15M) of salts with inorganic cations such as Na++ or K++ (i.e., low ionic strength), temperature higher than 20°C - 25° C. below the Tm of the oligomeric compound target sequence complex, and the presence of denaturants such as formamide, dimethylformamide, dimethyl sulfoxide, or the detergent sodium dodecyl sulfate (SDS). For example, the hybridization rate decreases 1.1% for each 1% formamide. An example of a high stringency hybridization condition is 0.1X sodium chloride-sodium citrate buffer (SSC)/0.1% (w/v) SDS at 60° C, for 30 minutes.

"Complementary," as used herein, refers to the capacity for precise pairing between two nucleotides on one or two oligomeric strands. For example, if a nucleobase at a certain position of an antisense compound is capable of hydrogen bonding with a nucleobase at a certain position of a target nucleic acid, said target nucleic acid being a DNA, RNA, or oligonucleotide molecule, then the position of hydrogen bonding between the oligonucleotide and the target, nucleic acid is considered to be a complementary position. The oligomeric compound and the further DNA, RNA, or oligonucleotide molecule are complementary to each other when a sufficient number of complementary positions in each molecule are occupied by nucleotides which can hydrogen bond with each other. Thus, "specifically hybridizable" and "complementary" are terms which are used to indicate a sufficient degree of precise pairing or complementarity over a sufficient number of nucleotides such that stable and specific binding occurs between the oligomeric compound and a target nucleic acid.

It is understood in the art that the sequence of an oligomeric compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure, mismatch or hairpin structure). The oligomeric compounds of the present disclosure comprise at least about 70%, or at least about 75%, or at least about 80%, or at least about 85%, or at least about 90%, or at least about 95%, or at least about 99% sequence complementarity to a target region within the target nucleic acid sequence to which they are targeted. For example, an antisense compound in which 18 of 20 nucleotides of the antisense compound are complementary to a target region, and would therefore specifically hybridize, would represent 90 percent complementarity. In this example, the remaining noncomplementary nucleotides may be clustered or interspersed with complementary nucleotides and need not be contiguous to each other or to complementary nucleotides. As such, an antisense compound which is 18 nucleotides in length having 4 (four) noncomplementary nucleotides which are flanked by two regions of complete complementarity with the target nucleic acid would have 77.8% overall complementarity with the target nucleic acid and would thus fall within the scope of the present disclosure. Percent complementarity of an antisense compound with a region of a target nucleic acid can be determined routinely using BLAST programs (basic local alignment search tools) and PowerBLAST programs known in the art. Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl Math., (1981) 2, 482-489).

As used herein, the term "Thermal Melting Point (Tm)" refers to the temperature, under defined ionic strength, pH, and nucleic acid concentration, at which 50% of the oligonucleotides complementary to the target sequence hybridize to the target sequence at equilibrium. Typically, stringent conditions will be those in which the salt concentration is at least about 0.01 to 1.0 M Na ion concentration (or other salts) at pH 7.0 to 8.3 and the temperature is at least about 30°C for short oligonucleotides (e.g., 10 to 50 nucleotide). Stringent conditions may also be achieved with the addition of destabilizing agents such as formamide.

As used herein, "modulation" means either an increase (stimulation) or a decrease (inhibition) in the expression of a gene.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the disclosure are variants of wild type gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs,) or single base mutations in which the polynucleotide sequence varies by one base. The presence of SNPs may be indicative of, for example, a certain population with a propensity for a disease state, that is susceptibility versus resistance.

Derivative polynucleotides include nucleic acids subjected to chemical modification, for example, replacement of hydrogen by an alkyl, acyl, or amino group. Derivatives, e.g., derivative oligonucleotides, may comprise non-naturally-occurring portions, such as altered sugar moieties or inter-sugar linkages. Exemplary among these are phosphorothioate and other sulfur containing species which are known in the art. Derivative nucleic acids may also contain labels, including radionucleotides, enzymes, fluorescent agents, chemiluminescent agents, chromogenic agents, substrates, cofactors, inhibitors, magnetic particles, and the like.

A "derivative" polypeptide or peptide is one that is modified, for example, by glycosylation, pegylation, phosphorylation, sulfation, reduction/alkylation, acylation, chemical coupling, or mild formalin treatment. A derivative may also be modified to contain a detectable label, either directly or indirectly, including, but not limited to, a radioisotope, fluorescent, and enzyme label.

As used herein, the term "animal" or "patient" is meant to include, for example, humans, sheep, elks, deer, mule deer, minks, mammals, monkeys, horses, cattle, pigs, goats, dogs, cats, rats, mice, birds, chicken, reptiles, fish, insects and arachnids.

"Mammal" covers warm blooded mammals that are typically under medical care (e.g., humans and domesticated animals). Examples include feline, canine, equine, bovine, and human, as well as just human.

"Treating" or "treatment" covers the treatment of a disease-state in a mammal, and includes: (a) preventing the disease-state from occurring in a mammal, in particular, when such mammal is predisposed to the disease-state but has not yet been diagnosed as having it; (b) inhibiting the disease-state, e.g., arresting it development; and/or (c) relieving the disease-state, e.g., causing regression of the disease state until a desired endpoint is reached. Treating also includes the amelioration of a symptom of a disease (e.g., lessen the pain or discomfort), wherein such amelioration may or may not be directly affecting the disease (e.g., cause, transmission, expression, etc.).

As used herein, "cancer" refers to all types of cancer or neoplasm or malignant tumors found in mammals, including, but not limited to: leukemias, lymphomas, melanomas, carcinomas and sarcomas. The cancer manifests itself as a "tumor" or tissue comprising malignant cells of the cancer. Examples of tumors include sarcomas and carcinomas such as, but not limited to: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma. Additional cancers which can be treated by the disclosed composition according to the disclosure include but not limited to, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, cervical cancer, endometrial cancer, adrenal cortical cancer, and prostate cancer.

"Neurological disease or disorder" refers to any disease or disorder of the nervous system and/or visual system, "Neurological disease or disorder" include disease or disorders that involve the central nervous system (brain, brainstem and cerebellum), the peripheral nervous system (including cranial nerves), and the autonomic nervous system (parts of which are located in both central and peripheral nervous system). Examples of neurological disorders include but are not limited to, headache, stupor and coma, dementia, seizure, sleep disorders, trauma, infections, neoplasms, neuroopthalmology, movement disorders, demyelinating diseases, spinal cord disorders, and disorders of peripheral nerves, muscle and neuromuscular junctions. Addiction and mental illness, include, but are not limited to, bipolar disorder and schizophrenia, are also included in the definition of neurological disorder. The following is a list of several neurological disorders, symptoms, signs and syndromes that can be treated using compositions and methods according to the present disclosure: acquired epileptiform aphasia; acute disseminated encephalomyelitis; adrenoleukodystrophy; age-related macular degeneration; agenesis of the corpus callosum; agnosia; Aicardi syndrome; Alexander disease; Alpers' disease; alternating hemiplegia; Vascular dementia; amyotrophic lateral sclerosis; anencephaly: Angelman syndrome; angiomatosis; anoxia; aphasia; apraxia; arachnoid cysts; arachnoiditis; Anronl-Chiari malformation; arteriovenous malformation; Asperger syndrome; ataxia telegiectasia; attention deficit hyperactivity disorder; autism; autonomic dysfunction; back pain; Batten disease; Behcet's disease; Bell's palsy; benign essential blepharospasm; benign focal; amyotrophy; benign intracranial hypertension; Binswanger's disease; blepharospasm: Bloch Sulzberger syndrome; brachial plexus injury; brain abscess; brain injury; brain tumors (including glioblastoma multiforme); spinal tumor; Brown-Sequard syndrome; Canavan disease; carpal tunnel syndrome; causalgia; central pain syndrome; central pontine myelinolysis; cephalic disorder; cerebral aneurysm; cerebral arteriosclerosis; cerebral atrophy; cerebral gigantism; cerebral palsy; Charcot-Marie-Tooth disease; chemotherapy-induced neuropathy and neuropathic pain; Chiari malformation; chorea; chronic inflammatory demyelinating polyneuropathy; chronic pain; chronic regional pain syndrome; Coffin Lowry syndrome; coma, including persistent vegetative state; congenital facial diplegia; corticobasal degeneration; cranial arteritis; craniosynostosis; Creutzfeldt-Jakob disease; cumulative trauma disorders; Cushing's syndrome; cytomegalic inclusion body disease; cytomegalovirus infection; dancing eyes-dancing feet syndrome; DandyWalker syndrome; Dawson disease; De Morsier's syndrome; Dejerine-Klumke palsy; dementia; dermatomyositis; diabetic neuropathy; diffuse selerosis; dysautonomia; dysgraphia; dyslexia; dystonias; early infantile epileptic encephalopathy; empty sella syndrome; encephalitis; encephaloceles; encephalotrigeminal angiomatosis; epilepsy; Erb's palsy; essential tremor; Fabry's disease; Fahr's syndrome; fainting; familial spastic paralysis; febrile seizures; Fisher syndrome; Friedreich's ataxia; fronto-temporal dementia and other "tauopathies"; Gaucher's disease; Gerstmann's syndrome; giant cell arteritis; giant cell inclusion disease; globoid cell leukodystrophy; Guillain-Barre syndrome; HTLV-1-associated myelopathy; Hallervorden-Spatz disease; head injury; headache; hemifacial spasm; hereditary spastic paraplegia; heredopathia atactic a polyneuritiformis; herpes zoster oticus; herpes zoster; Hirayama syndrome; HIVassociated dementia and neuropathy (also neurological manifestations of AIDS); holoprosencephaly; Huntington's disease and other polyglutamine repeat diseases; hydranencephaly; hydrocephalus; hypercortisolism; hypoxia; immune-mediated encephalomyelitis; inclusion body myositis; incontinentia pigmenti; infantile phytanic acid storage disease; infantile refsum disease; infantile spasms; inflammatory myopathy; intracranial cyst; intracranial hypertension; Joubert syndrome; Keams-Sayre syndrome; Kennedy disease Kinsboume syndrome; Klippel Feil syndromes; Krabbe disease; Kugelberg-Welander disease; kuru; Lafora disease; Lambert-Eaton myasthenic syndrome; Landau-Kleffner syndrome; lateral medullary (Wallenberg) syndrome; learning disabilities; Leigh's disease; Lennox-Gustaut syndrome: Lesch-Nyhan syndrome; leukodystrophy; Lewy body dementia; Lissencephaly; locked-in syndrome; Lou Gehrig's disease (i.e., motor neuron disease or amyotrophic lateral sclerosis); lumbar disc disease; Lyme disease--neurological sequelae; Machado-Joseph disease; macrencephaly; megalencephaly; Melkersson-Rosenthal syndrome; Menieres disease; meningitis; Menkes disease, metachromatic leukodystrophy; microcephaly; migraine; Miller Fisher syndrome; mini-strokes; mitochondrial myopathies; Mobius syndrome; monomelic amyotrophy; motor neuron disease; Moyamoya disease; mucopolysaccharidoses; milti-infarct dementia; multifocal motor neuropathy; multiple sclerosis and other demyelinating disorders; multiple system atrophy with postural hypotension; p muscular dystrophy; myasthenia gravis; myelinoclastic diffuse sclerosis; myoclonic encephalopathy of infants; myoclonus; myopathy; myotonia congenital; narcolepsy; neurofibromatosis; neuroleptic malignant syndrome; neurological manifestations of AIDS; neurological sequelae oflupus; neuromyotonia; neuronal ceroid lipofuscinosis; neuronal migration disorders; Niemann-Pick disease; O'Sullivan-McLeod syndrome; occipital neuralgia; occult spinal dysraphism sequence; Ohtahara syndrome; olivopontocerebellar atrophy; opsoclonus myoclonus; optic neuritis; orthostatic hypotension; overuse syndrome; paresthesia; Neurodegenerative disease or disorder (Parkinson's disease, Huntington's disease, Alzheimer's disease, amyotrophic lateral sclerosis (ALS), dementia, multiple sclerosis and other diseases and disorders associated with neuronal cell death); paramyotonia congenital; paraneoplastic diseases; paroxysmal attacks; Parry Romberg syndrome; Pelizaeus-Merzbacher disease; periodic paralyses; peripheral neuropathy; painful neuropathy and neuropathic pain; persistent vegetative state; pervasive developmental disorders; photic sneeze reflex; phytanic acid storage disease; Pick's disease: pinched nerve; pituitary tumors; polymyositis; porencephaly; post-polio syndrome; postherpetic neuralgia; postinfectious encephalomyelitis; postural hypotension; Prader- Willi syndrome; primary lateral sclerosis; prion diseases; progressive hemifacial atrophy; progressive multifocalleukoencephalopathy; progressive sclerosing poliodystrophy; progressive supranuclear palsy; pseudotumor cerebri; Ramsay-Hunt syndrome (types I and 11); Rasmussen's encephalitis; reflex sympathetic dystrophy syndrome; Refsum disease; repetitive motion disorders; repetitive stress injuries; restless legs syndrome; retrovirus-associated myelopathy; Rett syndrome; Reye's syndrome; Saint Vitus dance; Sandhoff disease; Schilder's disease; schizencephaly; septo-optic dysplasia; shaken baby syndrome; shingles; Shy-Drager syndrome; Sjogren's syndrome; sleep apnea; Soto's syndrome; spasticity; spina bifida; spinal cord injury; spinal cord tumors; spinal muscular atrophy; Stiff-Person syndrome; stroke; Sturge-Weber syndrome, subacute sclerosing panencephalitis; subcortical arteriosclerotic encephalopathy; Sydenham chorea; syncope; syringomyelia; tardive dyskinesia; Tay-Sachs disease; temporal arteritis; tethered spinal cord syndrome; Thomson disease; thoracic outlet syndrome; Tic Douloureux; Todd's paralysis; Tourette syndrome; transient ischemic attack; transmissible spongiform encephalopathies; transverse myelitis; traumatic brain injury; tremor; trigeminal neuralgia; tropical spastic paraparesis; tuberous sclerosis; vascular dementia (multi-infarct dementia); vasculitis including temporal arteritis; Von Hippel-Lindau disease; Wallenberg's syndrome; Werdnig-Hoffman disease; West syndrome; whiplash; Williams syndrome; Wildon's disease; and Zellweger syndrome.

"Metabolic disease" refers to a wide range of diseases and disorders of the endocrine system including, for example, insulin resistance, diabetes, obesity, impaired glucose tolerance, high blood cholesterol, hyperglycemia, dyslipidemia and hyperlipidemia.

An "Inflammation" refers to systemic inflammatory conditions and conditions associated locally with migration and attraction of monocytes, leukocytes and/or neutrophils. Examples of inflammation include, but are not limited to, Inflammation resulting from infection with pathogenic organisms (including gram-positive bacteria, gram-negative bacteria, viruses, fungi, and parasites such as protozoa and helminths), transplant rejection (including rejection of solid organs such as kidney, liver, heart, lung or cornea, as well as rejection of bone marrow transplants including graft-versus-host disease (GVHD)), or from localized chronic or acute autoimmune or allergic reactions. Autoimmune diseases include acute glomerulonephritis; rheumatoid or reactive arthritis; chronic glomerulonephritis; inflammatory bowel diseases such as Crohn's disease, ulcerative colitis and necrotizing enterocolitis; granulocyte transfusion associated syndromes; inflammatory dermatoses such as contact dermatitis, atopic dermatitis, psoriasis; systemic lupus erythematosus (SLE), autoimmune thyroiditis, multiple sclerosis, and some forms of diabetes, or any other autoimmune state where attack by the subject's own immune system results in pathologic tissue destruction. Allergic reactions include allergic asthma, chronic bronchitis, acute and delayed hypersensitivity. Systemic inflammatory disease states include inflammation associated with trauma, bums, reperfusion following ischemic events (e.g. thrombotic events in heart, brain, intestines or peripheral vasculature, including myocardial infarction and stroke), sepsis, ARDS or multiple organ dysfunction syndrome. Inflammatory cell recruitment also occurs in atherosclerotic plaques, Inflammation includes, but is not limited to, Non-Hodgkin's lymphoma, Wegener's granulomatosis, Hashimoto's thyroiditis, hepatocellular carcinoma, thymus atrophy, chronic pancreatitis, rheumatoid arthritis, reactive lymphoid hyperplasia, osteoarthritis, ulcerative colitis, papillary carcinoma, Crohn's disease, ulcerative colitis, acute cholecystitis, chronic cholecystitis, cirrhosis, chronic sialadenitis, peritonitis, acute pancreatitis, chronic pancreatitis, chronic Gastritis, adenomyosis, endometriosis, acute cervicitis, chronic cervicitis, lymphoid hyperplasia, multiple sclerosis, hypertrophy secondary tο idiopathic thrombocytopenic purpura, primary IgA nephropathy, systemic lupus erythematosus, psoriasis, pulmonary emphysema, chronic pyelonephritis, and chronic cystitis.

A cardiovascular disease or disorder includes those disorders that can either cause ischemia or are caused by reperfusion of the heart. Examples include, but are not limited to, atherosclerosis, coronary artery disease, granulomatous myocarditis, chronic myocarditis (non-granulomatous), primary hypertrophic cardiomyopathy, peripheral artery disease (PAD), stroke, angina pectoris, myocardial infarction, cardiovascular tissue damage caused by cardiac arrest, cardiovascular tissue damage caused by cardiac bypass, cardiogenic shock, and related conditions that would be known by those of ordinary skill in the art or which involve dysfunction of or tissue damage to the heart or vasculature, especially, but not limited to, tissue damage related to Sirtuin3 activation. CVS diseases include, but are not limited to, atherosclerosis, granulomatous myocarditis, myocardial infarction, myocardial fibrosis secondary to valvular heart disease, myocardial fibrosis without infarction, primary hypertrophic cardiomyopathy, and chronic myocarditis (non-granulomatous).

### Polynucleotide and Oligonucleotide Compositions and Molecules

### Targets

In one instance, the targets comprise nucleic acid sequences of a Sirtuin (SlRT), including without limitation sense and/or antisense noncoding and/or coding sequences associated with a Sirtuin (SIRT).

In one instance, the targets comprise nucleic acid sequences of SIRTI, including without limitation sense and/or antisense noncoding and/or coding sequences associated with SRTI gene.

In one instance, the targets comprise nucleic acid sequences of SIRT3, including without limitation sense and/or antisense noncoding and/or coding sequences associated with SIRT3 gene.

In one instance, . the targets comprise nucleic acid sequences of SIRT6, including without limitation sense and or antisense noncoding and/or coding sequences associated with SIRT6 gene.

"SIRT1 protein" refers to a member of the sir2 family of sirtuin deacetylases. In one instance, a SIRT1 protein includes yeast Sir2 (GenBank Accession No. P53685), C, elegans Sir-2.I (GenBank Accession No. NP.sub.--501912), human SIRT1 (GenBank Accession No. NM.sub,--012238 and NP.sub.--036370 (or AF083106))

SIRT1 "Sirtuins" are proteins that include a SIR2 domain, a domain defined as amino acids sequences that are scored as hits in the Pfam family "SIR2"- PF02146 (attached to the Appendix). This family is referenced in the INTERPRO database as INTERPRO description (entry IPR003000). To identity the presence of a "SIR2" domain in a protein sequence, and make the determination that a polypeptide or protein of interest has a particular profile, the amino acid sequence of the protein can be searched against the Pfam database of HMMs (e.g., the Pfam database, release 9) using the default parameters (http://www.sanger.ac.uk/Software/Pfam/HMM_search). The SIR2 domain is indexed in Pfam as PF02146 and in INTERPRO as INTERPRO description (entry IPR003000). A description of the Pfam database can be found in "The Pfam Protein Families Database" Bateman A et al. (2002) Nucleic Acids Research 30(1):276-280 and Sonhammer et al. (1997) Proteins 28(3):405-420 and a detailed description of HMMs can be found, for example, in Gribskov et al. (1990) Meth. Enzymol. 183: 146-159; Gribskov et al. (1987) Proc. Natl. Acad. Sci. USA 84:4355-4358: Krogh et al. (1994) J. Mol. Biol. 235: 1501-1531:, and Stultz et al. (1993) Protein Sci. 2:305-314.

Among the mitochondrial sirtuins, SIRT3 possesses the most robust deacetylase activity. Indeed, significantly higher levels of mitochondrial protein acetylation were detected in the livers of SIRT3-null mice, compared to those of SIRT4 or SIRT5 knockout animals. However, little is known about the physiological role of SIRT3 despite the fact that a number of SIRT3 substrates and co-precipitating proteins have been identified: acetyl-CoA synthetase 2, Ku70, FOXO3a, subunit 9 of mitochondrial Complex I (NDUFA9), glutamate dehydrogenase and isocitrate dehydrogenase 2.

SIRT3 is a major mitochondrial deacetylase. Mitochondrial proteins show hyperacetylation in SIRT3 knockout mice, but not in SIRT4 or SIRT5 knockout mice. Acetyl-CoA synthetase 2 (AceCS2), a mitochondrial enzyme that converts acetate into acetyl-CoA, was the first mitochondrial substrate of SIRT3 identified. Deacetylation of AceCS2 at lysine 642 by SIRT3 activates acetyl-CoA synthetase activity, providing increased acetyl-CoA to feed into the tricarboxylic acid cycle. Glutamate dehydrogenase (GDH), another mitochondrial protein involved in energy production, is deacetylated by SIRT3. GDH can also be ADP-ribosylated by SIRT4 in turn to decrease. its enzyme activity. This indicates that SIRT3 could play an important role in cell metabolism. SIRT3 has also been shown to be involved in selective apoptosis pathways and cell growth control SIRT3 and SIRT4, but not SIRT5, have been implicated in the NAD+ salvage pathway that regulates the NAD+ level relating to cell survival. In addition, variability in the hSIRT3 gene has been linked to human longevity.

The Silent information Regulator-2 gene (Sir2) encodes an NAD-dependent histone deacetylase that links regulation of chromatin, genomic stability, and life span in S. cerevisiae. By promoting chromatin silencing, Sir2 inhibits transcription at several genetic loci and represses recombination at ribosomal DNA (rDNA) repeats. Yeast with mutations in Sir2 have increased genomic instability in the context of rDNA recombination, which in turn shortens replicative life span - a marker of reproductive aging in this organism. Conversely, extracopies of Sir2 that suppress rDNA recombination increase replicative life span. These effects of Sir2 suggest paradigms in which genes that promote genome stabilization through chromatin modulation may be important contributors to regulation of organismal life span, aging, and age-related pathology.

Consistent with a conserved role for Sir2 factors in life span regulation, increased activity of Sir2 proteins in the multicellular organisms C. elegans and D. melanogaster also increases life span. However, these Sir2 factors may operate through mechanisms that are independent of genome stabilization, and their physiologic molecular substrates are still unclear. In mammals, there are seven Sir2 family members, SIRT1-SIRT7. The SIRTs have been of great interest as candidate regulators of mammalian life span and aging-related processes. In this context, several mammalian SIRTs have functions that impact on aging-associated molecular pathways and disease. However, initial studies of mammalian SIRTs linked these enzymes to biochemical targets and cellular functions that are distinct from those of S. cerevisiae Sir2.

The generation of mice deficient for the mammalian SIRT6 gene revealed a potential role for SIRT6 in linking regulation of life span, chromatin, and genomic stability. In this context, SIRT6 deficiency in mice leads to dramatically shortened life span and acute degenerative phenotypes that overlap with pathologies of premature aging. Moreover, SIRT6 knockout mouse cells have genomic instability and DNA damage hypersensitivity. In biochemical fractionation assays, SIRT6 protein associates preferentially with a chromatin-enriched cellular fraction. Together, these observations suggested that SIRT6 might couple chromatin regulation with DNA repair. However, a physiologic role for SIRT6 in such a process has not yet been demonstrated.

In some instances, antisense oligonucleotides are used to prevent or treat diseases or disorders associated with Sirtuin (SIRT) family members. Exemplary Sirtuin (SIRT) mediated diseases and disorders which can be treated with cell/tissues regenerated from stern cells obtained using the antisense compounds comprise; cancer (e.g., breast cancer, colorectal cancer, CCL, CML, prostate cancer), a neurodegenerative disease or disorder (e.g., Alzheimer's Disease (AD), Huntington's disease, Parkinson's disease, Amyotrophic Lateral Sclerosis (ALS), Multiple Sclerosis., and disorders caused by polyglutamine aggregation); skeletal muscle disease (e.g., Duchene muscular dystrophy, skeletal muscle atrophy, Becker's dystrophy, or myotonic dystrophy); a metabolic disease or disorder (e.g., insulin resistance, diabetes, type 2 diabetes, obesity, impaired glucose tolerance, metabolic syndrome, adult-onset diabetes, diabetic nephropathy, hyperglycemia, diabetic nephropathy, Hypercholesterolemia, dyslipidemia hyperlipidemia and an age-related metabolic disease etc.), a disease or disorder associated with impaired regulation of insulin level,. neuropathy (e.g., sensory neuropathy, autonomic neuropathy, motor neuropathy, retinopathy), a disease or disorder associated with a ketogenic condition, a disease or disorder associated with impaired energy homeostasis, a disease or disorder associated with impaired Acetyl-CoA synthetase 2 activity, a disease or disorder associated with metabolic homeostasis, a lipid metabolism disease or disorder, a disease or disorder associated with impaired thermogenesis, a disease or disorder associated with mitochondrial dysfunction, neuropathy (e.g., sensory neuropathy, autonomic neuropathy, motor neuropathy, retinopathy), a liver disease (e.g., due to alcohol abuse or hepatitis, fatty liver disease etc.); age-related macular degeneration, bone disease (e.g., osteoporosis), a blood disease (e.g., a leukemia); liver disease (e.g., due to alcohol abuse or hepatitis); obesity; bone resorption, age-related macular degeneration, AIDS related dementia, ALS, Bells Palsy, atherosclerosis, a cardiac disease (e.g., cardiac dysrhymias, chronic congestive heart failure, ischemic stroke, coronary artery disease and cardiomyopathy), chronically degenerative disease (e.g., cardiac muscle disease), chronic renal failure, type 2 diabetes, ulceration, cataract, presbiopia, glomerulonephritis, Guillan-Barre syndrome, hemorrhagic stroke, rheumatoid arthritis, inflammatory bowel disease, SLE, Crohn's disease, osteoarthritis, osteoporosis, Chronic Obstructive Pulmonary Disease (COPD), pneumonia, skin aging, urinary incontinence, a disease or disorder associated with mitochondrial dysfunction (e.g., mitochondrial myopathy, encephalopathy, Leber's disease, Leigh encephalopathia, Pearson's disease, lactic acidosis, 'mitochondrial encephalopathy, lactic acidosis and stroke like symptoms' (MBLAS) etc.) and a disease or disorder associated with neuronal cell death, degenerative syndrome, aging, a disease or disorder associated with telomere dysfunction, a disease or disorder associated with impaired chromatin regulation, a disease or disorder associated with premature cellular senescence, a disease or disorder associated with impared SIRT6 mediated DNA repair and a condition characterized by unwanted cell loss.

In another instance, the antisense oligonucleotides modulate the normal expression and/or normal function of a Sirtuin (SIRT) in patients suffering from or at risk of developing diseases or disorders associated with Sirtuin (SIRT).

In instances of the present disclosure, therapeutic and/or cosmetic regimes and related tailored treatments are provided to subjects requiring skin treatments or at risk of developing conditions for which they would require skin treatments. Diagnosis can be made, e.g., based on the subject's SIRT status. A patient's SIRT expression levels in a given tissue such as skin can be determined by methods known to those of skill in the art and described elsewhere herein, e.g., by analyzing tissue using PCR or antibody-based detection methods.

A preferred instance of the present disclosure provides a composition for skin treatment and/or a cosmetic application comprising SIRT antisense oligonucleotides, e.g., to upregulate expression of SIRT in the skin. Examples of antisense oligonucleotides are set forth as SEQ ID NOS: 4 to 16. U.S. Pat. No. 7,544,497, "Compositions for manipulating the lifespan and stress response of cells and organisms,"
describes potential cosmetic use for agents that modulate Sirtuin activity by reducing the Kₘ of the Sirtuin protein for its substrate. In instances, cells are treated in vivo with the oligonucleotides of the present disclosure, to increase cell lifespan or prevent apoptosis. For example, skin can be protected from aging, e.g., developing wrinkles, by treating skin, e.g., epithelial cells, as described herein. In an exemplary instance, skin is contacted with a pharmaceutical or cosmetic composition composing a SIRT antisense compound as described herein. Exemplary skin afflictions or skin conditions include disorders or diseases associated with or caused by inflammation, sun damage or natural aging. For example, the compositions find utility in the prevention or treatment of contact dermatitis (including irritant contact dermatitis and allergic contact dermatitis), atopic dermatitis (also known as allergic eczema), actinic keratosis, keratinization disorders (including eczema), epidermolysis bullosa diseases (including penfigus), exfoliative dermatitis, seborrheic dermatitis, erythemas (including erythema multiforme and erythema nodosum), damage caused by the sun or other light sources, discoid lupus erythematosus, dermatomyositis, skin cancer and the effects of natural aging.

Sirtuin has been reported to interfere with dihydrotestosterone-induced androgen receptor signaling. (See, e.g., Fu, et al., 2006, "Hormonal Control of Androgen Receptor Function through SIRT1," Molecular and Cellular Biology 26(21): 8122-8135). In instances of the present disclosure, a composition comprising SIRT antisense oligonucleotides, e.g., to upregulate expression of SIRT in the scalp and inhibit androgen receptor signaling, thereby preventing androgenetic alopecia (hair loss). In instances, a patient suffering from alopecia is administered either a topical or systemic formulation.

In an instance, an antisense oligonucleotide described herein is incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like. Formulations may be colorless, odorless ointments, lotions, creams, microemulsions and gels.

Antisense oligonucleotides of the disclosure may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington's Pharmaceutical Sciences (Mack Pub. Co.), ointment bases may be grouped into four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Exemplary water-soluble ointment bases are prepared from polyethylene glycols (PEGs) of varying molecular weight (see, e.g.. Remington's, supra).

Antisense oligonucleotides of the disclosure may be incorporated into lotions, which generally are preparations to be applied to the skin surface without friction, and are typically liquid or semiliquid preparations in which solid particles, including the active agent, are present in a water or alcohol base. Lotions arc usually suspensions of solids, and may comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like. An exemplary lotion formulation for use in conjunction with the present method contains propylene glycol mixed with a hydrophilic petrolatum such as that which may be obtained under the trademark Aquaphor.sup.RTM from Beiersdorf, Inc. (Notwalk, Conn.).

Antisense oligonucleotides of the disclosure may be incorporated into creams, which generally are viscous liquid or semisolid emulsions, either oil-in-water or water-in-oil. Cream bases are water-washable, and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, supra, is generally a nonionic, anionic, cationic or amphoteric surfactant.

Antisense oligonucleotides of the disclosure may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier). co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of poly glycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono-di-and triglycerides, mono-and di-esters of PEG (e.g., oleoyl macrogol glycerides), etc.

Antisense oligonucleotides of the disclosure may be incorporated into gel formulations, which generally are semisolid systems consisting of either suspensions made up of small inorganic particles (two-phase systems) or large organic molecules distributed substantially uniformly throughout a carrier liquid (single phase gels). Single phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulose, polyoxyethylene-polyoxypropylene, hydroxyethylcellulose and gelatin. Although gels commonly employ aqueous carrier liquid, alcohols and oils can be used as the carrier liquid as well.

Various additives, known to those skilled in the art, may be included in formulations, e.g., topical formulations. Examples of additives include, but are not limited to, solubilizers, skin permeation enhancers, opacifiers, preservatives (e.g., anti-oxidants), gelling agents, buffering agents, surfactants (particularly nonionic and amphoteric surfactants), emulsifiers, emollients, thickening agents, stabilizers, humectants" colorants, fragrance, and the like. Inclusion of solubilizers and/or skin permeation enhancers is particularly preferred, along with emulsifiers, emollients and preservatives. An optimum topical formulation comprises approximately: 2 wt. % to 60 wt. %, preferably 2 wt. % to 50 wt. %, solubilizer and/or skin permeation enhancer: 2 wt. % to 50 wt. %, preferably 2 wt, %, to 20 wt. %, emulsifiers; 2 wt. % to 20 wt. % emollient; and 0.01 to 0.2 wt. % preservative, with the active agent and carrier (e.g., water) making of the remainder of the formulation.

A skin permeation enhancer serves to facilitate passage of therapeutic levels of active agent to pass through a reasonably sized area of unbroken skin. Suitable enhancers are well known in the art and include, for example: lower alkanols such as methanol ethanol and 2-propanol; alkyl methyl sulfoxides such as dimethylsulfoxide (DMSO), decylmethylsulfoxide (C.sub.10MSO) and tetradecylmethyl sulfboxide; pyrrolidones such as 2-pyrrolidone, N-methyl-2-pyrrolidone and N-(-hydroxyethyl)pyrrolidone: urea; N,N-diethyl-m-toluamide; C.sub.2-C.sub.6 alkanediols; miscellaneous solvents such as dimethyl formamide (DMF), N,N-dimtethylacetamide (DMA) and tetrahydrofurfuryl alcohol; and the 1-substituted azacycloheptan-2-ones, particularly 1-n-dodecylcyclazacycloheptan-2-one (laurocapram; available under the trademark Azone.sup.RTM from Whitby Research Incorporated, Richmond, Va.).

Examples of solubilizers include, but are not limited to, the following: hydrophilic ethers such as diethylene glycol monoethyl ether (ethoxydiglycol, available commercially as Transcutol.sup.RTM) and diethylene glycol monoethyl ether oleate (available commercially as Soficutol.sup.RTM); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol.sup.RTM); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate, polypropylene glycol-2 (PPG-2) myristyl ether propionate, and the like.

Other active agents may also be included in formulations, e.g., other anti-inflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (e.g., octyl methoxycinnamate), dibenzoyl methanes (e.g., butyl methoxydibenzoyl methane), p-aminobenzoic- acid (PABA) and derivatives thereof, and salicylates (e.g., octyl salicylate).

In one instance, the oligonucleotides are specific for polynucleotides of a Sirtuin (SIRT), which includes, without limitation noncoding regions. The Sirtuin (SIRT) targets comprise variants of a Sirtuin (SIRT); mutants of a Sirtuin (SIRT), including SNPs; noncoding sequences of a Sirtuin (SIRT); alleles, fragments and the like. Preferably the oligonucleotide is an antisense RNA molecule.

In accordance with instances of the disclosure, the target nucleic acid molecule is not limited to a Sirtuin (SIRT) polynucleotides alone but extends to any of the isotonns, receptors, homologs, non-coding regions and the like of a Sirtuin (SIRT).

In another instance, an oligonucleotide targets a natural antisense sequence (natural antisense to the coding and non-coding regions) of a Sirtuin (SIRT) targets, including, without limitation, variants, alleles, homologs, mutants, derivatives, fragments and complementary sequences thereto. Preferably the oligonucleotide is an antisense RNA or DNA molecule.

In another instance, the oligomeric compounds of the present disclosure also include variants in which a different base is present at one or more of the nucleotide positions in the compound. For example, if the first nucleotide is an adenine, variants may be produced which contain thymidine, guanosine, cytidine or other natural or unnatural nucleotides at this position. This may be done at any of the positions of the antisense compound.

In some instances, homology, sequence identity or complementarity, between the antisense compound and target is nom about 50% to about 60%. In some instances, homology, sequence identity or complementarity, is from about 60% to about 70%. In some instances, homology, sequence identity or complementarity, is from about 70% to about 80%. In some instances, homology, sequence identity or complementarity, is from about 80% to about 90%. In some instances, homology, sequence identity or complementarity, is about 90%, about 92%, about 94%, about 95%, about 96%, about 97%, about 98%, about 99% or about 100%.

An antisense compound is specifically hybridizable when binding of the compound to the target nucleic acid interferes with the normal function of the target nucleic acid to cause a loss of activity, and there is a sufficient degree of complementarity tο avoid non-specific binding of the antisense compound to non-target nucleic acid sequences under conditions in which specific binding is desired. Such conditions include, i.e., physiological conditions in the case of in vivo assays or therapeutic treatment, and conditions in which assays are performed in the case of in vitro assays.

An antisense compound, whether DNA, RNA, chimeric, substituted etc, is specifically hybridizable when binding of the compound to the target DNA or RNA molecule interferes with the normal function of the target DNA or RNA to cause a loss of utility, and there is a sufficient degree of complementarily to avoid non-specific binding of the antisense compound to non-target sequences under conditions in which specific binding is desired, i.e., under physiological conditions in the case of in vivo assays or therapeutic treatment, and in the case of in vitro assays, under conditions in which the assays arc performed.

In another instance, targeting of a Sirtuin (SIRT) including without limitation, antisense sequences which are identified and expanded, using for example, PCR, hybridization etc., one or more of the sequences set forth as SEQ ID NO: 5 to 14, and the like, modulate the expression or function of a Sirtuin (SIRT). In one instance, expression or function is up-regulated as compared to a control. In another instance, expression or function is down-regulated as compared to a control.

In another instance, oligonucleotides comprise nucleic acid sequences set forth as SEQ ID NOS: 15 to 94 including antisense sequences which are identified and expanded, using for example, PCR, hybridization etc. These oligonucleotides can comprise one or more modified nucleotides, shorter or longer fragments, modified bonds and the like. Examples of modified bonds or internucleotide linkages comprise phosphorothioate, phosphorodithioate or the like. In another instance, the nucleotides comprise a phosphorus derivative. The phosphorus derivative (or modified phosphate group) which may be attached to the sugar or sugar analog moiety in the modified oligonucleotides of the present disclosure may be a monophosphate, diphosphate, triphosphate, alkylphosphate, alkanephosphate, phosphorothioate and the like. The preparation of the above-noted phosphate analogs, and their incorporation into nucleotides, modified nucleotides and oligonucleotides, *per se,* is also known and need not be described here.

The specificity and sensitivity of antisense is also harnessed by those of skill in the art for therapeutic uses. Antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides can be useful therapeutic modalities that can be configured to be useful in treatment regimes for treatment of cells, tissues and animals, especially humans.

In instances of the present disclosure oligomeric antisense compounds, particularly oligonucleotides, bind to target nucleic acid molecules and modulate the expression and/or function of molecules encoded by a target gene. The functions of DNA to be interfered comprise, for example, replication and transcription. The functions of RNA to be interfered comprise all vital functions such as, for example, translocation of the RNA to the site of protein translation, translation of protein from the RNA, splicing of the RNA to yield one or more mRNA species, and catalytic activity which may be engaged in or facilitated by the RNA. The functions may be up-regulated or inhibited depending on the functions desired.

The antisense compounds, include, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds may be introduced in the form of single-stranded, double-stranded, partially single-stranded, or circular oligomeric compounds.

Targeting an antisense compound to a particular nucleic acid molecule, in the context of this disclosure, can be a multistep process. The process usually begins with the identification of a target nucleic acid whose function is to be modulated. This target nucleic acid may be, for example, a cellular gene (or mRNA transcribed from the gene) whose expression is associated with a particular disorder or disease state, or a nucleic acid molecule from an infectious agent. In the present disclosure, the target nucleic acid encodes a Sirtuin (SIRT).

The targeting process usually also includes determination of at least one target region, segment, or site within the target nucleic acid for the antisense interaction to occur such that the desired effect, e.g., modulation of expression, will result. Within the context of the present disclosure, the term "region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present disclosure, are defined as positions within a target nucleic acid.

In one instance, the antisense oligonucleotides bind to the natural antisense sequences of a Sirtuin (SIRT) and modulate the expression and/or function of a Sirtuin (SIRT) (SEQ ID NO: 1 to 3). Examples of antisense sequences include SEQ ID NOS: 4 to 29.

In another instance, the antisense oligonucleotides bind to one or more segments of a Sirtuin (SIRT) polynucleotide and modulate the expression and/or function of a Sirtuin (SIRT). The segments comprise at least five consecutive nucleotides of a Sirtuin (SIRT) sense or antisense polynucleotides.

In another instance, the antisense oligonucleotides are specific for natural antisense sequences of a Sirtuin (SIRT) wherein binding of the oligonucleotides to the natural antisense sequences of a Sirtuin (SIRT) modulate expression and/or function of a Sirtuin (SIRT).

In another instance, oligonucleotide compounds comprise sequences set forth as SEQ ID NOS: 15 to 94, antisense sequences which are identified and expanded, using for example, PCR, hybridization etc These oligonucleotides can comprise one or more modified nucleotides, shorter or longer fragments, modified bonds and the like. Examples of modified bonds or internucleotide linkages comprise phosphorothioate, phosphorodithioate or the like. In another instance, the nucleotides comprise a phosphorus derivative. The phosphorus derivative (or modified phosphate group) which may be attached to the sugar or sugar analog moiety in the modified oligonucleotides of the present disclosure may be a monophosphate, diphosphate, triphosphate, alkylphosphate, alkanephosphate, phosphorothioate and the like. The preparation of the above-noted phosphate analogs, and their incorporation into nucleotides, modified nucleotides and oligonucleotides, per se, is also known and need not be described here.

Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes has a translation initiation codon having the RNA sequence 5'-GUG, 5'-UUG or 5'-CUG; and 5'-AUA, 5'-ACG and 5'-CUG have been shown to function in vivo. Thus, the terms "translation initiation codon" and "start codon" can encompass many codon sequences, even though the initiator amino acid in each instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. In the context of the disclosure, "start codon" and "translation initiation codon" refer to the codon or codons that are used in vivo to initiate translation of an mRNA transcribed from a gene encoding a Sirtuin (SIRT), regardless of the sequence(s) of such codons. A translation termination codon (or "stop codon") of a gene may have one of three sequences, i.e., 5'-UAA, 5'-UAG and 5'-UGA (the corresponding DNA sequences are 5'-TAA, 5'-TAG and 5'-TGA, respectively).

The terms "start codon region" and "translation initiation codon region" refer to a portion of such an mRNA or gene that encompasses from about 25 to about 50 contiguous nucleotides in either direction (i.e., 5' or 3') from a translation initiation codon. Similarly, the terms "stop codon region" and "translation termination codon region" refer to a portion of such an mRNA or gene that encompasses from about 25 to about 50 contiguous nucleotides in either direction (i.e., 5' or 3') from a translation termination codon. Consequently, the "start codon region" (or "translation initiation codon region") and the "stop codon region" (or "translation termination codon region") are all regions that may be targeted effectively with the antisense compounds of the present disclosure.

The open reading frame (ORF) or "coding region," which is known in the art to refer to the region between the translation initiation codon and the translation termination codon, is also a region which may be targeted effectively. Within the context of the present disclosure, a targeted region is the intragenic region encompassing the translation initiation or termination codon of the open reading frame (ORF) of a gene.

Another target region includes the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene). Still another target region includes the 3' untranslated region (3'UTR), known in the art to refer to the portion of an mRNA in the 3' direction from the translation termination, codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. Another target region for this disclosure is the 5' cap region.

Although some cukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. In one instance, targeting splice sites, i.e., intron-exon junctions or exon-intron junctions, is particularly useful in situations where aberrant splicing is implicated in disease, or where an overproduction of a particular splice product is implicated in disease. An aberrant fusion junction due to rearrangement or deletion is another instance of a target site. mRNA transcripts produced via the process of splicing of two (or more) mRNAs from different gene sources are known as "fusion transcripts". Introns can be effectively targeted using antisense compounds targeted to, for example, DNA or pre-mRNA.

In another instance, the antisense oligonucleotides bind to coding and/or non-coding regions of a target polynucleotide and modulate the expression and/or function of the target molecule.

In another instance, the antisense oligonucleotides bind to natural antisense polynucleotides and modulate the expression and/or function of the target molecule.

In another instance, the antisense oligonucleotides bind to sense polynucleotides and modulate the expression and/or function of the target molecule.

Alternative RNA transcripts can be produced from the same genomic region of DNA. These alternative transcripts are generally known as "variants". More specifically, "pre-mRNA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing. These mRNA variants are also known as "alternative splice variants". If no splicing of the pre-mRNA variant occurs then the pre-mRNA variant is identical to the mRNA variant.

Variants can be produced through the use of alternative signals to start or stop transcription. Pre-mRNAs and mRNAs can possess more than one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Within the context of the disclosure, the types of variants described herein are also instances of target nucleic acids.

The locations on the target nucleic acid to which the antisense compounds hybridize are defined as at least a 5-nucleotide long portion of a target region to which an active antisense compound is targeted.

While the specific sequences of certain exemplary target segments are set forth herein, one of skill in the art will recognize that these serve to illustrate and describe particular instances within the scope of the present disclosure. Additional target segments are readily identifiable by one having ordinary skill in the art in view of this disclosure.

Target segments 5-100 nucleotides in length comprising a stretch of at least five (5) consecutive nucleotides selected from within the illustrative target segments are considered to be suitable for targeting as well.

Target segments can include DNA or RNA sequences that comprise at least the 5 consecutive nucleotides from the 5'-terminus of one of the illustrative target segments (the remaining nucleotides being a consecutive stretch of the same DNA or RNA beginning immediately upstream of the 5'-terminus of the target segment and continuing until the DNA or RNA contains about 5 to about 100 nucleotides). Similarly target segments are represented by DNA or RNA sequences that comprise at least the 5 consecutive nucleotides from the 3'-terminus of one of the illustrative target segments (the remaining nucleotides being a consecutive stretch of the same DNA or RNA beginning immediately downstream of the 3-terminus of the target segment and continuing until the DNA or RNA contains about 5 to about 100 nucleotides). One having skill in the art armed with the target segments illustrated herein will be able, without undue experimentation, to identify further target segments.

Once one or more target regions, segments or sites have been identified, antisense compounds are chosen which are sufficiently complementary to the target, i.e., hybridize sufficiently well and with sufficient specificity, to give the desired effect.

In instances of the disclosure the oligonucleotides bind to an antisense strand of a particular target. The oligonucleotides are at least 5 nucleotides in length and can be synthesized so each oligonucleotide targets overlapping sequences such that oligonucleotides are synthesized to cover the entire length of the target polynucleotide. The targets also include coding as well as non coding regions.

In one instance, specific nucleic acids are targeted by antisense oligonucleotides. Targeting an antisense compound to a particular nucleic acid, is a multistep process. The process usually begins with the identification of a nucleic acid sequence whose function is to be modulated. This may be, for example, a cellular gene (or mRNA transcribed from the gene) whose expression is associated with a particular disorder or disease state, or a non coding polynucleotide such as for example, non coding RNA (ncRNA).

RNAs can be classified into (1) messenger RNAs (mRNAs), which are translated into proteins, and (2) non-protein-coding RNAs (ncRNAs). ncRNAs comprise microRNAs, antisense transcripts and other Transcriptional Units (TU) containing a high density of stop codons and lacking any extensive "Open Reading Frame", Many ncRNAs appear to start from initiation sites in 3' untranslated regions (3'UTRs) of protein-coding loci. ncRNAs are often rare and at least half of the ncRNAs that have been sequenced by the FANTOM consortium seem not to be polyadenylated. Most researchers have for obvious reasons focused on polyadenylated mRNAs that are processed and exported to the cytoplasm. Recently, it was shown that the set of non-polyadenylated nuclear RNAs may be very large, and that many such transcripts arise from intergenic regions. The mechanism by which ncRNAs may regulate gene expression is by base pairing with target transcripts. The RNAs that function by base pairing can be grouped into (1) cis encoded RNAs that are encoded at the same genetic location, but on the opposite strand to the RNAs they act upon and therefore display perfect complementarity to their target, and (2) trans-encoded RNAs that are encoded at a chromosomal location distinct from the RNAs they act upon and generally do not exhibit perfect base-pairing potential with then targets.

Without wishing to be bound by theory, perturbation of an antisense polynucleotide by the antisense oligonucleotides described herein can alter the expression of the corresponding sense messenger RNAs. However, this regulation can either be discordant (antisense knockdown results in messenger RNA elevation) or concordant (antisense knockdown results in concomitant messenger RNA reduction). In these cases, antisense oligonucleotides can be targeted to overlapping or non-overlapping parts of the antisense transcript resulting in its knockdown or sequestration. Coding as well as non-coding antisense can be targeted in an identical manner and that either category is capable of regulating the corresponding sense transcripts - either in a concordant or disconcordant manner. The strategies that are employed in identifying new oligonucleotides for use against a target can be based on the knockdown of antisense RNA transcripts by antisense oligonucleotides or any other means of modulating the desired target.

*Strategy 1:* In the case of discordant regulation, knocking down the antisense transcript elevates the expression of the conventional (sense) gene. Should that latter gene encode for a known or putative drug target, then knockdown of its antisense counterpart could conceivably mimic the action of a receptor agonist or an enzyme stimulant.

*Strategy 2.* In the case of concordant regulation, one could concomitantly knock down both antisense and sense transcripts and thereby achieve synergistic reduction of the conventional (sense) gene expression. If for example, an antisense oligonucleotide is used to achieve knockdown, then this strategy can be used to apply one antisense oligonucleotide targeted to the sense transcript and another antisense oligonucleotide to the corresponding antisense transcript, or a single energetically symmetric antisense oligonucleotide that simultaneously targets overlapping sense and antisense transcripts.

According to the present disclosure, antisense compounds include antisense oligonucleotides, ribozymes, external guide sequence (EGS) oligonucleotides, siRN A compounds, single- or double-stranded RNA interference (RNAi) compounds such as siRNA compounds, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, doublestranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleotide positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines, however, in some instances, the gene expression or function is up regulated. When formed from two strands, or a single strand that takes the form of a self-complementary hairpin-type molecule doubled back oi3 itself to form a duplex, the two strands (or duplex-forming regions of a single strand) are complementary RNA strands that base pair in Watson-Crick fashion.

Once introduced to a system, the compounds of the disclosure may elicit the action of one or more enzymes or structural proteins to effect cleavage or other modification of the target nucleic acid or may work via occupancy-based mechanisms, in general, nucleic acids (including oligonucleotides) may be described as "DNA-like" (i.e., generally having one or more 2'-deoxy sugars and, generally, T rather than U bases) or "RNA-like" (i.e., generally having one or more 2'-hydroxyl or 2'-modified sugars and, generally U rather than T bases). Nucleic acid helices can adopt more than one type of structure, most commonly the A- and B-forms. It is believed that, in general, oligonucleotides which have β-form-like structure are "DNA-like" and those which have A-formlike structure are "RNA-like." In some (chimeric) instances, an antisense compound may contain both A- and B-form regions.

In another instance, the desired oligonucleotides or antisense compounds, comprise at least one of: antisense RNA, antisense DNA, chimeric antisense oligonucleotides, antisense oligonucleotides comprising modified linkages, interference RNA (RNAi), short interfering RNA (siRNA); a micro, interfering RNA (miRNA); a small, temporal RNA (stRNA); or a short, hairpin RNA (shRNA); small RNA-induced gene activation (RNAa); small activating RNAs (saRNAs), or combinations thereof.

dsRNA can also activate gene expression, a mechanism that has been termed "small RNA-induced gene activation" or RNAa. dsRNAs targeting gene promoters induce potent transcriptional activation of associated genes. RNAa was demonstrated in human cells using synthetic dsRNAs, termed "small activating RNAs" (saRNAs).

Small double-stranded RNA (dsRNA), such as small interfering RNA (siRNA) and microRNA (miRNA), have been found to be the trigger of an evolutionary conserved mechanism known as RNA interference (RNAi), RNAi invariably leads to gene silencing. However, in instances described in detail in the examples section which follows, oligonucleotides are shown to increase the expression and/or function of the Sirtuin (S1RT) polynucleotides and encoded products thereof dsRNAs may also act as small activating RNAs (saRNA). Without wishing to be bound by theory, by targeting sequences in gene promoters, saRNAs would induce target gene expression in a phenomenon referred to as dsRNA-induced transcriptional activation (RNAa).

In a further instance, the "target segments" identified herein may be employed in a screen for additional compounds that modulate the expression of a Sirtuin (SIRT) polynucleotide. "Modulators" are those compounds that decrease or increase the expression of a nucleic acid molecule encoding a Sirtuin (SIRT) and which comprise at least a 5-nucleotide portion that is complementary to a target segment. The screening method comprises the steps of contacting a target segment of a nucleic acid molecule encoding sense or natural antisense polynucleotides of a Sirtuin (SiRT) with one or more candidate modulators, and selecting for one or more candidate modulators which decrease or increase the expression of a nucleic acid molecule encoding a Sirtuin (SIRT) polynucleotide, e.g. SEQ ID NOS: 15 to 94. Once it is shown that the candidate modulator or modulators are capable of modulating (e.g. either decreasing or increasing) the expression of a nucleic acid molecule encoding a Sirtuin (SIRT) polynucleotide, the modulator may then be employed in further investigative studies of the function of a Sirtuin (SIRT) polynucleotide, or for use as a research, diagnostic, or therapeutic agent in accordance with the present disclosure.

Targeting the natural antisense sequence modulates the function of the target gene. For example, the Sirtuin (SIRT) (e.g. accession numbers NM_012238.3, NM_001159589, NM_012239, NM_016539), in a instance, the target is an antisense polynucleotide of the Sirtuin (SIRT). In a instance, an antisense oligonucleotide targets sense and/or natural antisense sequences of a Sirtuin (SIRT) polynucleotide (e.g. accession numbers NM_012238.3, NM_001159589, NM_012239, NM_016539), variants, alleles, isoforms, homologs, mutants, derivatives, fragments and complementary sequences thereto. Preferably the oligonucleotide is an antisense molecule and the targets include coding and noncoding regions of antisense and/or sense Sirtuin (SIRT) polynucleotides.

The target segments of the present disclosure may be also be combined with their respective complementary antisense compounds of the present disclosure to form stabilized double-stranded (duplexed) oligonucleotides.

Such double stranded oligonucleotide moieties have been shown in the art to modulate target expression and regulate translation as well as RNA processing via an antisense mechanism. Moreover, the double-stranded moieties may be subject to chemical modifications. For example, such double-stranded moieties have been shown to inhibit the target by the classical hybridization of antisense strand of the duplex to the target, thereby triggering enzymatic degradation of the target.

In a instance, an antisense oligonucleotide targets Sirtuin (SIRT) polynucleotides (e.g. accession numbers NM_012238.3, NM_001159589, NM_012239, NM_016539), variants, alleles, isoforms, homologs, mutants, derivatives, fragments and complementary sequences thereto. Preferably the oligonucleotide is an antisense molecule.

In accordance with instances of the disclosure, the target nucleic acid molecule is not limited to Sirtuin (SIRT) alone but extends to any of the isoforms, receptors, homologs and the like of a Sirtuin (SIRT) molecule.

In another instance, an oligonucleotide targets a natural antisense sequence of a Sirtuin (SIRT) polynucleotide, for example, polynucleotides set forth as SEQ ID NO: 5 to 14, and any variants, alleles, homologs, mutants, derivatives, fragments and complementary sequences thereto. Examples of antisense oligonucleotides are set forth as SEQ ID NOS: 15 to 94.

In one instance, the oligonucleotides are complementary to or bind to nucleic acid sequences of a Sirtuin (SIRT) antisense, including without limitation noncoding sense and/or antisense sequences associated with a Sirtuin (SIRT) polynucleotide and modulate expression and/or function of a Sirtuin (SIRT) molecule.

In another instance, the oligonucleotides are complementary to or bind to nucleic acid sequences of a Sirtuin (SIRT) natural antisense, set forth as SEQ ID NO: 5 to 14 and modulate expression and/or function of a Sirtuin (SIRT) molecule.

In a instance, oligonucleotides comprise sequences of at least 5 consecutive nucleotides of SEQ ID NOS: 15 to 94 and modulate expression and/or function of a Sirtuin (SIRT) molecule.

The polynucleotide targets comprise Sirtuin (SIRT), including family members thereof, variants of a Sirtuin (SIRT); mutants of a Sirtuin (SIRT), including SNPs; noncoding sequences of a Sirtuin (SIRT); alleles of a Sirtuin (SIRT); species variants, fragments and the like. Preferably the oligonucleotide is an antisense molecule.

In another instance, the oligonucleotide targeting Sirtuin (SIRT) polynucleotides, comprise: antisense RNA, interference RNA (RNAi), short interfering RNA (siRNA); micro interfering RNA (miRNA); a small, temporal RNA (stRNA); or a short, hairpin RNA (shRNA); small RNA-induced gene activation (RNAa); or, small activating RNA (saRNA).

In another instance, targeting of a Sirtuin (SIRT) polynucleotide, e.g. SEQ ID NO: 5 to 14 modulate the expression or function of these targets. In one instance, expression or function is up-regulated as compared to a control. In another instance, expression or function is down-regulated as compared to a control.

In another instance, antisense compounds comprise sequences set forth as SEQ ID NOS: 15 to 94. These oligonucleotides can comprise one or more modified nucleotides, shorter or longer fragments, modified bonds and the like.

In another instance, SEQ ID NOS: 15 to 94 comprise one or more LNA nucleotides.

The modulation of a desired target nucleic acid can be carried out in several ways known in the art. For example, antisense oligonucleotides, siRNA etc. Enzymatic nucleic acid molecules (e.g., ribozymes) are nucleic acid molecules capable of catalyzing one or more of a variety of reactions, including the ability to repeatedly cleave other separate nucleic acid molecules in a nucleotide base sequence-specific manner. Such enzymatic nucleic acid molecules can be used, for example, to target virtually any RNA transcript.

Because of their sequence-specificity, trans-cleaving enzymatic nucleic acid molecules show promise as therapeutic agents for human disease. Enzymatic nucleic acid molecules can be designed to cleave specific RNA targets within the background of cellular RNA. Such a cleavage event renders the mRNA non-functional and abrogates protein expression from that RNA. In this manner, synthesis of a protein associated with a disease state can be selectively inhibited.

In general, enzymatic nucleic acids with RNA cleaving activity act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

Several approaches such as in vitro selection (evolution) strategies have been used to evolve new nucleic acid catalysts capable of catalyzing a variety of reactions, such as cleavage and ligation of phosphodiester linkages and amide linkages.

The development of ribozymes that are optimal for catalytic activity would contribute significantly to any strategy that employs RNA-cleaving ribozymes for the purpose of regulating gene expression. The hammerhead ribozyme, for example, functions with a catalytic rate (kcat) of about 1 min-1 in the presence of saturating (10 mM) concentrations of Mg2+ cofactor. An artificial "RNA ligase" ribozyme has been shown to catalyze the corresponding self-modification reaction with a rate of about 100 min-1. In addition, it is known that certain modified hammerhead ribozymes that have substrate binding arms made of DNA catalyze RNA cleavage with multiple turn-over rates that approach 100 min-1. Finally, replacement of a specific residue within the catalytic core of the hammerhead with certain nucleotide analogues gives modified ribozymes that show as much as a 10-fold improvement in catalytic rate. These findings demonstrate that ribozymes can promote chemical transformations with catalytic rates that are significantly greater than those displayed in vitro by most natural self-cleaving ribozymes. It is then possible that the structures of certain selfcleaving ribozymes may be optimized to give maximal catalytic activity, or that entirely new RNA motifs can be made that display significantly faster rates for RNA phosphodiester cleavage.

Intemolecular cleavage of an RNA substrate by an RNA catalyst that fits the "hammerhead" model was first shown in 1987. The RNA catalyst was recovered and reacted with multiple RNA molecules, demonstrating that it was truly catalytic.

Catalytic RNAs designed based on the "hammerhead" motif have been used to cleave specific target sequences by making appropriate base changes in the catalytic RNA to maintain necessary base pairing with the target sequences. This has allowed use of the catalytic RNA to cleave specific target sequences and indicates that catalytic RNAs designed according to the "hammerhead" model may possibly cleave specific substrate RNAs in vivo.

RNA interference (RNAi) has become a powerful tool for modulating gene expression in mammals and mammalian cells. This approach requires the delivery of small interfering RNA (siRNA) either as RNA itself or as DNA, using an expression plasmid or virus and the coding sequence for small hairpin RNAs that are processed to siRNAs. This system enables efficient transport of the pre-siRNAs to the cytoplasm where they are active and permit the use of regulated and tissue specific promoters for gene expression.

In one instance, an oligonucleotide or antisense compound comprises an oligomer or polymer of ribonucleic acid (RNA) and/or deoxyribonucleic acid (DNA), or a mimetic, chimera, analog or homolog thereof. This term includes oligonucleotides composed of naturally occurring nucleotides, sugars and covalent internucleoside (backbone) linkages as well as oligonucleotides having non-naturally occurring portions which function similarly. Such modified or substituted oligonucleotides are often desired over native forms because of desirable properties such as, for example, enhanced cellular uptake, enhanced affinity for a target nucleic acid and increased stability in the presence of nucleases.

According to the present disclosure, the oligonucleotides or "antisense compounds" include antisense oligonucleotides (e.g. RNA, DNA, mimetic, chimera, analog or homolog thereof), ribozymes, external guide sequence (EGS) oligonucleotides, siRNA compounds, single- or double-stranded RNA interference (RNAi) compounds such as siRNA compounds, saRNA, aRNA, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, double-stranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleotide positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines. When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

Once introduced to a system, the compounds of the disclosure may elicit the action of one or more enzymes or structural proteins to effect cleavage or other modification of the target nucleic acid or may work via occupancy-based mechanisms. In general, nucleic acids (including oligonucleotides) may be described as "DNA-like" (i.e., generally having one or more 2'-deoxy sugars and, generally, T rather than U bases) or "RNA-like" (i.e., generally having one or more 2'-hydroxyl or 2'-modified sugars and, generally U rather than T bases). Nucleic acid helices can adopt more than one type of structure, most commonly the A- and B-forms. It is believed that, in general, oligonucleotides which have B-form-like structure are "DNA-like" and those which have A-formlike structure are "RNA-like." In some (chimeric) instances, an antisense compound may contain both A- and B-form regions.

The antisense compounds in accordance with this disclosure can comprise an antisense portion from about 5 to about 80 nucleotides (i.e. from about 5 to about 80 linked nucleosides) in length. This refers to the length of the antisense strand or portion of the antisense compound. In other words, a single-stranded antisense compound of the disclosure comprises from 5 to about 80 nucleotides, and a double-stranded antisense compound of the disclosure (such as a dsRNA, for example) comprises a sense and an antisense strand or portion of 5 to about 80 nucleotides in length. One of ordinary skill in the art will appreciate that this comprehends antisense portions of 5, 6, 7,8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides in length, or any range therewithin.

In one instance, the antisense compounds of the disclosure have antisense portions of 10 to 50 nucleotides in length. One having ordinary skill in the art will appreciate that this embodies oligonucleotides having antisense portions of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length, or any range therewithin. In some instances, the oligonucleotides are 15 nucleotides in length.

In one instance, the antisense or oligonucleotide compounds of the disclosure have antisense portions of 12 or 13 to 30 nucleotides in length. One having ordinary skill in the art will appreciate that this embodies antisense compounds having antisense portions of 12, 13, 14, 15, 16, 17, 18, 19, 20, 21, 22, 23, 24, 25, 26, 27, 28, 29 or 30 nucleotides in length, or any range the

In another instance, the oligomeric compounds of the present disclosure also include variants in which a different base is present at one or more of the nucleotide positions in the compound. For example, if the first nucleotide is an adenosine, variants may be produced which contain thymidine, guanosine or cytidine at this position. This may be done at any of the positions of the antisense or dsRNA compounds. These compounds are then tested using the methods described herein to determine their ability to inhibit expression of a target nucleic acid.

In some instances, homology, sequence identity or complementarity, between the antisense compound and target is from about 40% to about 60%. In some instances, homology, sequence identity or complementarity, is from about 60% to about 70%. In some instances, homology, sequence identity or complementarity, is from about 70% to about 80%). In some instances, . homology, sequence identity or complementarity, is from about 80% to about 90%. In some instances, homology, sequence identity or complementarity, is about 90%, about 92%, about 94%, about 95%, about 96%, about 97%, about 98%, about 99% or about 100%,

In another instance, the antisense oligonucleotides, such as for example, nucleic acid molecules set forth in SEQ ID NOS: 4 to 29 comprise one or more substitutions or modifications. In one instance, the nucleotides are substituted with locked nucleic acids (LNA).

In another instance, he oligonucleotides target one or more regions of the nucleic acid molecules sense and/or antisense of coding and/or non-coding sequences associated with Sirtain (SIRT) and the sequences set forth as SEQ ID NOS: 1 to 14. The oligonucleotides are also targeted to overlapping regions of SEQ ID NOS: 1 to 14.

Certain oligonucleotides of this disclosure are chimeric oligonucleotides. "Chimeric oligonucleotides" or "chimeras," in the context of this disclosure are oligonucleotides which contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of antisense modulation of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art. In one instance, a chimeric oligonucleotide comprises at least one region modified to increase target binding affinity, and, usually, a region that acts as a substrate for RNAse H. Affinity of an oligonucleotide for its target (in this case, a nucleic acid encoding ras) is routinely determined by measuring the Tm of an oligonucleotide/target pair, which is the temperature at which the oligonucleotide and target dissociate; dissociation is detected spectrophotometrically. The higher the Tm. the greater is the affinity of the oligonucleotide for the target.

Chimeric antisense compounds of the disclosure may be formed as composite structures of two or more oligonucleotides, modified oligonucleotides, oligonucleosides and/or oligonucleotides mimetics as described above. Such; compounds have also been referred to in the art as hybrids or gapmers. Representative United States patents that teach the preparation of such hybrid structures comprise, but are not limited to, US patent nos. 5,013,830; 5,149,797; 5,220,007; 5,256,775; 5,366,878; 5,403,711; 5,491,133; 5,565,350; 5,623,065; 5,652,355; 5,652,356; and 5,700,922.

In another instance, the region of the oligonucleotide which is modified comprises at least one nucleotide modified at the 2' position of the sugar, most preferably a 2'-Oalkyl, 2'-O-alkyl-O-alkyl or 2'-fluoro-modified nucleotide. In other instances, RNA modifications include 2'-fluoro, 2'-amino and 2' O-methyl modifications on the ribose of pyrimidines, abasic residues or an inverted base at the 3' end of the RNA. Such modifications are routinely incorporated into oligonucleotides and these oligonucleotides have been shown to have a higher Tm (i.e., higher target binding affinity) than; 2'-deoxyoligonucleotides against a given target. The effect of such increased affinity is to greatly enhance RNAi oligonucleotide inhibition of gene expression. RNAse H is a cellular endonuclease that cleaves the RNA strand of RNA:DNA duplexes; activation of this enzyme therefore results in cleavage of the RNA target, and thus can greatly enhance the efficiency of RNAi inhibition. Cleavage of the RNA target can be routinely demonstrated by gel electrophoresis. In another instance, the chimeric oligonucleotide is also modified to enhance nuclease resistance. Cells contain a variety of exo- and endo-nucleases which can degrade nucleic acids. A number of nucleotide and nucleoside modifications have been shown to make the oligonucleotide into which they are incorporated more resistant to nuclease digestion than the native oligodeoxynucleotide. Nuclease resistance is routinely measured by incubating oligonucleotides with cellular extracts or isolated nuclease solutions and measuring the extent of intact oligonucleotide remaining over time, usually by gel electrophoresis. Oligonucleotides which have been modified to enhance their nuclease resistance survive intact for a longer time than unmodified oligonucleotides. A variety of oligonucleotide modifications have been demonstrated to enhance or confer nuclease resistance. Oligonucleotides which contain at least one phosphorothioate modification are presently more preferred. In some cases, oligonucleotide modifications which enhance target binding affinity are also, independently, able to enhance nuclease resistance. Some desirable modifications can be found in De Mesmaeker et al. (1995) Acc. Chem. Res., 28:366-374.

Specific examples of some oligonucleotides envisioned for this disclosure include those comprising modified backbones, for example, phosphorothioates, phosphotriesters, methyl phosphonates, short chain alkyl or cycloalkyl intersugar linkages or short chain heteroatomic or heterocyclic intersugar linkages. Most are oligonucleotides with phosphorothioate backbones and those with heteroatom backbones, particularly CH2 -NH-O--CH2, CH,--N(CH3)--O--CH2 [known as a methylelene(methylimino) or MMI backbone], CH2 --O--N (CH3)-CH2, CH2 --N (CH3)--N (CH3)--CH2 and O--N (CH3)--CH2 --CH2 backbones, wherein the native phosphodiester backbone is represented as O--P--O--CH,). The amide backbones disclosed by De Mesmaeker et al. (1995) Acc. Chem. Res. 28:366-374 are also preferred. Also are oligonucleotides having morpholino backbone structures (Suramerton and Weller, U.S. Pat. No. 5,034,506). In other instances, such as the peptide nucleic acid (PNA) backbone, the phosphodiester backbone of the oligonucleotide is replaced with a polyamide backbone, the nucleotides being bound, directly or indirectly to the aza nitrogen atoms of the polyamide backbone. Oligonucleotides may also comprise one or more substituted sugar moieties. oligonucleotides comprise one of the following at the 2' position: OH, SH, SCH3, F, OCN, OCH3 OCH3, OCH3 O(CH2)n CH3, O(CH2)n NH2 or O(CH2)n CH3 where n is from 1 to about 10; C1 to C10 lower alkyl, alkoxyalkoxy, substituted lower alkyl, alkaryl or aralkyl; Cl; Br; CN; CF3 ; OCF3; O--, S--, or N-alkyl; O--, S--, or N-alkenyl; SOCH3; SO2 CH3; ONO2; NO2; N3: NH2; heterocycloalkyl; heterocycloalkaryl; aminoalkylamino; polyalkylamino; substituted silyl; an RNA cleaving group: a reporter group; an intercalator; a group for improving the pharmacokinetic properties of an oligonucleotide; or a group for improving the pharmacodynamic properties of an oligonucleotide and other substituents having similar properties. A modification includes 2'-methoxyethoxy [2'-O-CH2 CH2 OCH3, also known as 2'-O-(2~methoxyethyl)]. Other modifications include 2'-methoxy (2'-O--CH3), 2'- propoxy (2'-OCH2 CH2CH3) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group.

Oligonucleotides may also include, additionally or alternatively, nucleobase (often referred to in the art simply as "base") modifications or substitutions. As used herein, "unmodified" or "natural" nucleotides include adenine (A), guanine (G), thymine (T), cytosine (C) and uracil (U). Modified nucleotides include nucleotides found only infrequently or transiently in natural nucleic acids, e.g., hypoxanthine, 6-methyladenine, 5-Me pyrimidines, particularly 5-methylcytosine (also referred to as 5-methyl-2' deoxycytosine and often referred to in the art as 5-Me-C), 5- hydroxymethylcytosine (HMC), glycosyl HMC and gentobiosyl HMC, as well as synthetic nucleotides, e.g., 2-aminoadenine, 2-(methylamino)adenine, 2-(imidazolylalkyl)adenine, 2- (aminoalklyamino)adenine or other heterosubstituted alkyladenines, 2-thiouracil, 2-thiothymine, 5- bromouracil, 5-hydroxymethyluracil, β-azaguanine, 7-deazaguanine, N6 (6-aminohexyl)adenine and 2,6-diaminopurine. A "universal" base known in the art, e.g., inosine, may be included. 5-Me-C substitutions have been shown to increase nucleic acid duplex stability by 0.6-1.2°C. (Sanghvi, Y. S., in Crooke, S. T. and Lebleu, B., eds., Antisense Research and Applications, CRC Press, Boca Raton, 1993, pp. 276-278) and are presently base substitutions.

Another modification of the oligonucleotides of the disclosure involves chemically linking to the oligonucleotide one or more moieties or conjugates which enhance the activity or cellular uptake of the oligonucleotide. Such moieties include but are not limited to lipid moieties such as a cholesterol moiety, a cholesteryl moiety, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero- 3-H-phosphonate, a polyamine or a polyethylene glycol chain, or adamantane acetic acid . Oligonucleotides comprising lipophilic moieties, and methods for preparing such oligonucleotides are known in the art, for example, U.S. Pat. Nos. 5,138,045,5,218,105 and 5,459,255.

It is not necessary for all positions in a given oligonucleotide to be uniformly modified, and in fact more than one of the aforementioned modifications may be incorporated in a single oligonucleotide or even at within a single nucleoside within an oligonucleotide. The present disclosure also includes oligonucleotides which are chimeric oligonucleotides as hereinbefore defined.

In another instance, the nucleic acid molecule of the present disclosure is conjugated with another moiety including but not limited to abasic nucleotides, polyether, polyamine, polyamides, peptides, carbohydrates, lipid, or polyhydrocarbon compounds. Those skilled in the art will recognize that these molecules can be linked to one or more of any nucleotides comprising the nucleic acid molecule at several positions on the sugar, base or phosphate group.

The oligonucleotides used in accordance with this disclosure may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of one of ordinary skill in the art. It is also well known to use similar techniques to prepare other oligonucleotides such as the phosphorothioates and alkylated derivatives. It is also well known to use similar techniques and commercially available modified amidites and controlled-pore glass (CPG) products such as biotin, fluorescein, acridine or psoralen-modified amidites and/or CPG (available from Glen Research, Sterling VA) to synthesize fluorescently labeled, biotinylated or other modified oligonucleotides such as cholesterol-modified oligonucleotides.

In accordance with the disclosure, use of modifications such as the use of LNA monomers to enhance the potency, specificity and duration of action and broaden the routes of administration of oligonucleotides comprised of current chemistries such as MOE, ANA, FANA, PS etc. This can be achieved by substituting some of the monomers in the current oligonucleotides by LNA monomers. The LNA modified oligonucleotide may have a size similar to the parent compound or may be larger or preferably smaller. It is that such LNA-modified oligonucleotides contain less than about 70%, more preferably less than about 60%, most preferably less than about 50% LNA monomers and that their sizes are between about 5 and 25 nucleotides, more preferably between about 12 and 20 nucleotides.

Modified oligonucleotide backbones comprise, but are not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Representative United States patents that teach the preparation of the above phosphorus containing linkages comprise, but are not limited to, US patent nos. 3,687,808; 4,469,863; 4,476,301; 5,023,243; 5, 177,196; 5,188,897; 5,264,423; 5,276,019; 5,278,302; 5,286,717; 5,321,131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050.

Modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones: methyleneimino and methylenehydrazino backbones; sulfonate and sulfonamide backbones; amide backbones; and others having mixed N, O, S and CH2 component parts.

Representative United States patents that teach the preparation of the above oligonucleosides comprise, but are not limited to, US patent nos. 5,034,506; 5,166,315; 5,185,444: 5,214,134; 5,216,141; 5,235,033; 5,264, 562; 5, 264,564; 5,405,938; 5,434,257; 5,466,677; 5,470,967; 5,489,677; 5,541,307; 5,561,225; 5,596, 086; 5,602,240; 5,610,289; 5,602,240; 5,608,046; 5,610,289; 5,618,704; 5,623,070; 5,663,312; 5,633,360; 5,677,437; and 5,677,439.

In other oligonucleotide mimetics, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds comprise, but are not limited to, US patent nos. 5,539,082; 5,714,331; and 5,719,262.

Further teaching of PNA compounds can be found in Nielsen, et al. (1991) Science 254, 1497-1500.

In another instance of the disclosure the oligonucleotides with phosphorothioate backbones and oligonucleosides with heteroatom backbones, and in particular- CH2-NH-O-CH2-,-CH2-N (CH3K)-CH2-known as a methylene (methylimino) or MMI backbone,- CH2-O-N(CH3)-CH2-,-CH2N(CH3)-N(CH3) CH2-and-ON(CH3)-CH2-CH2- wherein the native phosphodiester backbone is represented as -O-P-O-CH2- of the above referenced US patent no. 5,489,677, and the amide backbones of the above referenced US patent no. 5,602,240. Also are oligonucleotides having morpholino backbone structures of the above-referenced US patent no. 5,034,506.

Modified oligonucleotides may also contain one or more substituted sugar moieties. oligonucleotides comprise one of the following at the 2' position: OH; F; O-, S-, or N-alkyl; O-, S-, or N-alkenyl; O-, S-or N-alkynyl; or O alkyl-O-alkyl, wherein the alkyl, alkenyl and alkynyl may be substituted or unsubstituted C to CO alkyl or C2 to CO alkenyl and alkynyl. Particularly are O (CH2)n OmCH3, O(CH2)n,OCH3, O(CH2)nNH2, O(CH2)nCH3, O(CH2)nONH2, and O(CH2nON(CH2)nCH3)2 where n and m can be from 1 to about 10. Other oligonucleotides comprise one of the following at the 2' position: C to CO, (lower alkyl, substituted lower alkyl, alkaryl aralkyl, O-alkaryl or O-aralkyl, SH, SCH3, OCN, Cl, Br, CN, CF3, OCF3, SOCH3, SO2CH3, ONO2, NO2, N3, NH2, heterocycloalkyl, heterocycloalkaryl aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A modification comprises 2'-methoxyethoxy (2'-O-CH2CH2OCH3, also known as 2'O-(2-methoxyethyl) or 2-MOE) i.e., an alkoxyalkoxy group. A further modification comprises 2'-dimethylaminooxyethoxy, i.e. , a O(CH2)2ON(CH3)2 group, also known as 2-DMAOE, as described in examples herein below, and 2'-dimethylaminoethoxyethoxy (also known in the art as 2'-O-dimethylaminoethoxyethyl or 2'-DMAEOE), i.e., 2-O-CH2-O-CH2-N(CH2)2.

Other modifications comprise 2'-methoxy (2'-OCH3), 2'-aminopropoxy (2'-OCH2CH2CH2NH2) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures comprise, but are not limited to, US patent nos. 4,981,957; 5,118,800; 5,319,080; 5,359,044; 5,393,878; 5,446,137; 5,466,786; 5,514, 785; 5,519,134; 5,567,811; 5,576,427; 5,591,722; 5,597,909; 5,610,300; 5,627,053; 5,639,873; 5,646,265; 5,658,873; 5,670,633; and 5,700,920 .

Oligonucleotides may also comprise nucleobase (often referred to in the art simply as "base") modifications or substitutions. As used herein, "unmodified" or "natural" nucleotides comprise the purine bases adenine (A) and guanine (G), and the pyrimidine bases thymine (T), cytosine (C) and uracil (U). Modified nucleotides comprise other synthetic and natural nucleotides such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylquanine and 7-methyladenine, 8-azaguanine and 8-azaadenine, 7-deazaguanine and 7-deazaadenine and 3-deazaguanine and 3-deazaadenine.

Further, nucleotides comprise those disclosed in United States Patent No. 3.687,808, those disclosed in 'The Concise Encyclopedia of Polymer Science And Engineering', pages 858-859, Kroschwitz, J.I., ed. John Wiley & Sons, 1990, those disclosed by Englisch et al., 'Angewandle Chemie, International Edition', 1991, 30, page 613, and those disclosed by Sanghvi, Y.S., Chapter 15, 'Antisense Research and Applications', pages 289-302, Crooke, S.T. and Lebleu, B. ca., CRC Press, 1993. Certain of these nucleotides are particularly useful for increasing the binding affinity of the oligomeric compounds of the disclosure. These comprise 5-substituted pyrimidines, 6-azapyrimidines and N-2, N-6 and 0-6 substituted purines, comprising 2-aminopropyladenine, 5-propynyluracil and 5-propynylcytosine. 5-methylcytosine substitutions have been shown to increase nucleic acid duplex stability by 0.6-1,2°C (Sanghvi, Y.S., Crooke, S.T. and Lebleu, B., eds, 'Antisense Research and Applications', CRC Press, Boca Raton, 1993, pp. 276-278) and are presently base substitutions, even more particularly when combined with 2'-Omethoxyethyl sugar modifications.

Representative United States patents that teach the preparation of the above noted modified nucleotides as well as other modified nucleotides comprise, but are not limited to, US patent nos. 3,687,808, as well as 4,845,205; 5,130,302; 5,134,066; 5,175,273; 5,367,066, 5,432,272; 5,457,187; 5,459,255; 5,484,908; 5,502,177; 5,525,711; 5,552,540; 5,587,469; 5,596,091; 5,614,617; 5,750,692, and 5,681,941.

Another modification of the oligonucleotides of the disclosure involves chemically linking to the oligonucleotide one or more moieties or conjugates, which enhance the activity, cellular distribution, or cellular uptake of the oligonucleotide.

Such moieties comprise but are not limited to, lipid moieties such as a cholesterol moiety, cholic acid, a thioether, e.g., hexyl-S-tritylthiol, a thiocholesterol, an aliphatic chain, e.g., dodecandiol or undecyl residues , a phospholipid, e.g., di-hexadecyl-rac-glycerol or triethylammonium 1,2-di-O-hexadecyl-rac-glycero-3-H-phosphonate , a polyamine or a polyethylene glycol chain, or adamantane acetic acid, a palmityl moiety , or an octadecylamine or hexylamino-carbonyl-toxycholesterol moiety.

Representative United States patents that teach the preparation of such oligonucleotides conjugates comprise, but are not limited to, US patent nos. 4,828,979; 4,948,882; 5,218,105; 5,525,465; 5,541,313; 5,545,730; 5,552, 538; 5,578,717, 5,580,731; 5,580,731; 5,591,584; 5,109,124; 5,118,802; 5,138,045; 5,414,077; 5,486, 603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5, 245,022; 5,254,469; 5,258,506; 5,262,536; 5,272,250; 5,292,873, 5,317,098; 5,317,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

*Drug discovery:* The compounds of the present disclosure can also be applied in the areas of drug discovery and target validation. The present disclosure comprehends the use of the compounds and target segments identified herein in drug discovery efforts to elucidate relationships that exist between a Sirtuin (SIRT) polynucleotide and a disease state, phenotype, or condition. These methods include detecting or modulating a Sirtuin (SIRT) polynucleotide comprising contacting a sample, tissue, cell, or organism with the compounds of the present disclosure, measuring the nucleic acid or protein level of a Sirtutn (SIRT) polynucleotide and/or a related phenotypic or chemical endpoint at some time after treatment, and optionally comparing the measured value to a non-treated sample or sample treated with a further compound of the disclosure. These methods can also be performed in parallel or in combination with other experiments to determine the function of unknown genes for the process of target validation or to determine the validity of a particular gene product as a target for treatment or prevention of a particular disease, condition, or phenotype.

### Assessing Up-regulation or Inhibition of Gene Expression:

Transfer of an exogenous nucleic acid into a host cell or organism can be assessed by directly detecting the presence of the nucleic acid in the cell or organism. Such detection can be achieved by several methods well known in the art. For example, the presence of the exogenous nucleic acid can be detected by Southern blot or by a polymerase chain reaction (PCR) technique using primers that specifically amplify nucleotide sequences associated with the nucleic acid. Expression of the exogenous nucleic acids can also be measured using conventional methods including gene expression analysis. For instance, mRNA produced from an exogenous nucleic acid can be detected and quantified using a Northern blot and reverse transcription PCR (RT-PCR).

Expression of RNA from the exogenous nucleic acid can also be detected by measuring an enzymatic activity or a reporter protein activity. For example, antisense modulatory activity can be measured indirectly as a decrease οr increase in target nucleic acid expression as an indication that the exogenous nucleic acid is producing the effector RNA. Based on sequence conservation, primers can be designed and used to amplify coding regions of the target genes. Initially, the most highly expressed coding region from each gene can be used to build a model control gene, although any coding or non coding region can be used. Each control gene is assembled by inserting each coding region between a reporter coding region and its poly(A) signal. These plasmids would produce an mRNA with a reporter gene in the upstream portion of the gene and a potential RNAi target in the 3' non-coding region. The effectiveness of individual antisense oligonucleotides would be assayed by modulation of the reporter gene. Reporter genes useful in the methods of the present disclosure include acetohydroxyacid synthase (AHAS), alkaline phosphatase (AP), beta galactosidase (LacZ), beta glucoronidase (GUS), chloramphenicol acetyltransferase (CAT), green fluorescent protein (GFP), red fluorescent protein (RFP), yellow fluorescent protein (YFP), cyan fluorescent protein (CFP), horseradish peroxidase (HRP), luciferase (Luc), nopaline synthase (NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracycline. Methods to determine modulation of a reporter gene are well known in the art, and include, but are not limited to, fluorometric methods (e.g. fluorescence spectroscopy, Fluorescence Activated Cell Sorting (FACS), fluorescence microscopy), antibiotic resistance determination.

SIRT1, SIRT3 and SIRT6 proteins and mRNA expression can be assayed using methods known to those of skill in the art and described elsewhere herein. For example, immunoassays such as the ELISA can be used to measure protein levels. Sirtuin (SIRT) antibodies for ELISAs are available commercially, e.g., from R&D Systems (Minneapolis, MN), Abcam, Cambridge, MA.

In instances, SIRT1, SIRT3 and SIRT6 expression (e.g., mRNA or protein) in a sample (e.g., cells or tissues in vivo or in vitro) treated using an antisense oligonucleotide of the disclosure is evaluated by comparison with Sirtuin (SIRT) expression in a control sample. For example, expression of the protein or nucleic acid can be compared using methods known to those of skill in the art with that in a mock-treated or untreated sample. Alternatively, comparison with a sample treated with a control antisense oligonucleotide (e.g., one having an altered or different sequence) can be made depending on the information desired. In another instance, a difference in the expression of the Sirtuin (SIRT) protein or nucleic acid in a treated vs. an untreated sample can be compared with the difference in expression of a different nucleic acid (including any standard deemed appropriate by the researcher, e.g., a housekeeping gene) in a treated sample vs. an untreated sample.

Observed differences can be expressed as desired, e.g., in the form of a ratio or fraction, for use in a comparison with control. In instances, the level of a Sirtuin (SIRT) mRNA or protein, in a sample treated with an antisense oligonucleotide of the present disclosure, is increased or decreased by about 1.25-fold to about 10-fold or more relative to an untreated sample or a sample treated with a control nucleic acid. In instances, the level of a Sirtuin (SIRT) mRNA or protein is increased or decreased by at least about 1.25-fold, at least about 1.3-fold, at least about 1.4-fold, at least about 1.5-fold, at least about 1.6-fold, at least about 1.7-fold, at least about 1.8-fold, at least about 2-fold, at least about 2.5-fold, at least about 3-fold, at least about 3.5-fold, at least about 4-fold, at least about 4.5-fold, at least about 5-fold, at least about 5.5-fold, at least about 6-fold, at least about 6.5-fold, at least about 7-fold, at least about 7.5-fold, at least about 8-fold, at least about 8.5-fold, at least about 9-fold, at least about 9.5-fold, or at least about 10-fold or more.

### Kits, Research Reagents, Diagnostics, and Therapeutics

The compounds of the present disclosure can be utilized for diagnostics, therapeutics, and prophylaxis, and as research reagents and components of kits. Furthermore, antisense oligonucleotides, which are able to inhibit gene expression with exquisite specificity, are often used by those of ordinary skill to elucidate the function of particular genes or to distinguish between functions of various members of a biological pathway.

For use in kits and diagnostics and in various biological systems, the compounds of the present disclosure. either alone or in combination with other compounds or therapeutics, are useful as tools in differential and/or combinatorial analyses to elucidate expression patterns of a portion or the entire complement of genes expressed within cells and tissues.

As used herein the term "biological system" or "system" is defined as any organism, cell, cell culture or tissue that expresses, or is made competent to express products of the Sirtuin (SIRT). These include, but are not limited to, humans, transgenic animals, cells, cell cultures, tissues, xenografts, transplants and combinations thereof.

As one non limiting example, expression patterns within cells or tissues treated with one or more antisense compounds are compared to control cells or tissues not treated with antisense compounds and the patterns produced are analyzed for differential levels of gene expression as they pertain, for example, to disease association, signaling pathway, cellular localization, expression level, size, structure or function of the genes examined. These analyses can be performed on stimulated or unstimulated cells and in the presence or absence of other compounds that affect expression patterns.

Examples of methods of gene expression analysis known in the art include DNA arrays or microarrays (Brazma and Vilo, (2000) FEBS Lett., 480, 17-24; Celis, et al., (2000) FEBS Lett., 480, 2-16), SAGE (serial analysis of gene expression) (Madden, et al., (2000) Drug Discov. Today, 5, 415- 425), READS (restriction enzyme amplification of digested cDNAs) (Prashar and Weissman, (1999) Methods Enzymol., 303, 258-72), TOGA (total gene expression analysis) (Sutcliffe, et al., (2000) Proc. Natl. Acad. Sci. U.S.A., 97, 1976-81), protein arrays and proteomics (Celis, et al., (2000) FEBS Lett., 480, 2-16; Jungblut, et al., Electrophoresis, 1999, 20, 2100-10), expressed sequence tag (EST) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., (2000) Anal. Biochem. 286, 91-98; Larson, et al., (2000) Cytometry 41, 203-208), subtractive cloning, differential display (DD) (Jurecic and Belmont, (2000) Curr. Opin. Microbiol. 3, 316-21), comparative genomic hybridization (Carulli, et al., (1998) J. Cell Biochem. Suppl., 31, 286-96), FISH (fluorescent in situ hybridization) techniques (Going and Gusterson, (1999) Eur. J. Cancer, 35, 1895-904) and mass spectrometry methods (To, Comb. (2000) Chem. High Throughput Screen, 3, 235-41).

The compounds of the disclosure are useful for research and diagnostics, because these compounds hybridize to nucleic acids encoding a Sirtuin (SIRT). For example, oligonucleotides that hybridize with such efficiency and under such conditions as disclosed herein as to be effective Sirtuin (SIRT) modulators are effective primers or probes under conditions favoring gene amplification or detection, respectively. These primers and probes are useful in methods requiring the specific detection of nucleic acid molecules encoding a Sirtuin (SIRT) and in the amplification of said nucleic acid molecules for detection or for use in further studies of a Sirtuin (SIRT). Hybridization of the antisense oligonucleotides, particularly the primers and probes, of the disclosure with a nucleic acid encoding a Sirtuin (SIRT) can be detected by means known in the art. Such means may include conjugation of an enzyme to the oligonucleotide, radiolabeling of the oligonucleotide, or any other suitable detection means. Kits using such detection means for detecting the level of a Sirtuin (SIRT) in a sample may also be prepared.

The specificity and sensitivity of antisense are also harnessed by those of skill in the art for therapeutic uses. Antisense compounds have been employed as therapeutic moieties in the treatment of disease states in animals, including humans. Antisense oligonucleotide drugs have been safely and effectively administered to humans and numerous clinical trials are presently underway. It is thus established that antisense compounds can be useful therapeutic modalities that can be configured to be useful in treatment regimes for the treatment of cells, tissues and animals, especially humans.

For therapeutics, an animal, preferably a human, suspected of having a disease or disorder which can be treated by modulating the expression of a Sirtuin (SIRT) polynucleotide is treated by administering antisense compounds in accordance with this disclosure. For example, in one non-limiting instance, the methods comprise the step of administering to the animal in need of treatment, a therapeutically effective amount of a Sirtuin (SIRT) modulator. The Sirtuin (SIRT) modulators of the present disclosure effectively modulate the activity of a Sirtuin (SIRT) or modulate the expression of a Sirtuin (SIRT) protein. In one instance, the activity or expression of a Sirtuin (SIRT) in an animal is inhibited by about 10% as compared to a control. Preferably, the activity or expression of a Sirtuin (SIRT) in an animal is inhibited by about 30%. More preferably, the activity or expression of a Sirtuin (SIRT) in an animal is inhibited by 50% or more. Thus, the oligomeric compounds modulate expression of a Sirtuin (SIRT) mRNA by at least 10%, by at least 50%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as compared to a control.

In one instance, the activity or expression of a Sirtuin (SIRT) and/or in an animal is increased by about 10% as compared to a control. Preferably, the activity or expression of a Sirtuin (SIRT) in an animal is increased by about 30%. More preferably, the activity or expression of a Sirtuin (SIRT) in an animal is increased by 50% or more. Thus, the oligomeric compounds modulate expression of a Sirtuin (SIRT) mRNA by at least 10%, by at least 50%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100% as compared to a control.

For example, the reduction of the expression of a Sirtuin (SIRT) may be measured in serum, blood, adipose tissue, liver or any other body fluid, tissue or organ of the animal. Preferably, the cells contained within said fluids, tissues or organs being analyzed contain a nucleic acid molecule encoding Sirtuin (SIRT) peptides and/or the Sirtuin (SIRT) protein itself.

The compounds of the disclosure can be utilized in pharmaceutical compositions by adding an effective amount of a compound to a suitable pharmaceutically acceptable diluent or carrier. Use of the compounds and methods of the disclosure may also be useful prophylactically.

*Conjugates:* Another modification of the oligonucleotides of the disclosure involves chemically linking to the oligonucleotide one or more moieties or conjugates that enhance the activity, cellular distribution or cellular uptake of the oligonucleotide. These moieties or conjugates can include conjugate groups covalently bound to functional groups such as primary or secondary hydroxyl groups. Conjugate groups of the disclosure include intercalators, reporter molecules, polyamines, polyamides, polyethylene glycols, polyethers, groups that enhance the pharmacodynamic properties of oligomers, and groups that enhance the pharmacokinetic properties of oligomers. Typicalconjugatc groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this disclosure, include groups that improve uptake, enhance resistance to degradation, and/or strengthen sequence-specific hybridization with the target nucleic acid. Groups that enhance the pharmacokinetic properties, in the context of this disclosure include groups that improve uptake, distribution, metabolism or excretion of the compounds of the present disclosure. Representative conjugate groups are disclosed in International Patent Application No. PCT/US92/09196, filed Oct. 23, 1992, and U.S. Pat. No. 6,287,860. Conjugate moieties include, but are not limited to, lipid moieties such as a cholesterol moiety, cholic acid, a thioether, e.g., hexyl-5-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-Hphosphonate, a polyamine or a polyethylene glycol chain, or adamantane acetic acid, a palmityl moiety, or an octadecylamine or hexylamino-carbonyl-oxycholesterol moiety. Oligonucleotides of the disclosure may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic.

Representative United States patents that teach the preparation of such oligonucleotides conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

*formulations:* The compounds of the disclosure may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption. Representative United States patents that teach the preparation of such uptake, distribution and/or absorption-assisting formulations include, but are not limited to, U.S. Pat. Nos. 5,108,921; 5,354,844; 5,416,016; 5,459,127; 5,521,291; 5,543,165; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

Although, the antisense oligonucleotides do not need to be administered in the context of a vector in order to modulate a target expression and/or function, instances of the disclosure relates to expression vector constructs for the expression of antisense oligonucleotides, comprising promoters, hybrid promoter gene sequences and possess a strong constitutive promoter activity, or a promoter activity which can be induced in the desired case.

In an instance, disclosure practice involves administering at least one of the foregoing antisense oligonucleotides with a suitable nucleic acid delivery system. In one instance, that system includes a non-viral vector operably linked to the polynucleotide. Examples of such nonviral vectors include the oligonucleotide alone (e.g. any one or more of SEQ ID NOS: 15 to 94) or in combination with a suitable protein, polysaccharide or lipid formulation.

Additionally suitable nucleic acid delivery systems include viral vector, typically sequence from at least one of an adenovirus, adenovirus-associated virus (AAV), helper-dependent adenovirus, retrovirus, or hemagglutinatin virus of Japan-liposome (HVJ) complex. Preferably, the vital vector comprises a strong eukaryotic promoter operably linked to the polynucleotide e.g., a cytomegalovirus (CMV) promoter.

Additionally vectors include viral vectors, fusion proteins and chemical conjugates. Retroviral vectors include Moloney murine leukemia viruses and HIV-based viruses. One HIV-based viral vector comprises at least two vectors wherein the gag and pol genes are from an HIV genome and the env gene is from another virus. DNA viral vectors are preferred. These vectors include pox vectors such as orthopox or avipox vectors, herpesvirus vectors such as a herpes simplex 1 virus (HSV) vector, Adenovirus Vectors and Adeno-associatcd Virus Vectors).

The antisense compounds of the disclosure encompass any pharmaceutically acceptable salts, esters, or salts of such esters, or any other compound which, upon administration to an animal, including a human, is capable of providing (directly or indirectly) the biologically active metabolite or residue thereof.

The term "pharmaceutically acceptable salts" refers to physiologically and pharmaceutically acceptable salts of the compounds of the disclosure: i.e., salts that retain the desired biological activity of the parent compound and do not impart undesired toxicological effects thereto. For oligonucleotides, examples of pharmaceutically acceptable salts and their uses are further described in U.S. Pat. No. 6,287,860.

The present disclosure also includes pharmaceutical compositions and formulations that include the antisense compounds of the disclosure. The pharmaceutical compositions of the present disclosure may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral Parenteral administration includes intravenous, intraarterial, subcutaneous, intraperitoneal or intramuscular injection or infusion; or intracranial, e.g., intrathecal or intraventricular, administration.

For treating tissues in the central nervous system, administration can be made by, e.g., injection or infusion into the cerebrospinal fluid. Administration of antisense RNA into cerebrospinal fluid is described, e.g., in U.S. Pat. App. Pub. No. 2007/0117772, "Methods for slowing familial ALS disease progression," .

When it is intended that the antisense oligonucleotide of the present disclosure be administered to cells in the central nervous system, administration can be with one or more agents capable of promoting penetration of the subject antisense oligonucleotide across the blood-brain barrier. Injection can be made, e.g., in the entorhinal cortex or hippocampus. Delivery of neurotrophic factors by administration of an adenovirus vector to motor neurons in muscle tissue is described in, e.g., U.S. Pat. No. 6,632,427, "Adenoviral-vector-mediated gene transfer into medullary motor neurons,". Delivery of vectors directly to the brain, e.g., the striatum, the thalamus, the hippocampus, or the substantia nigra, is known in the art and described, e.g., in U.S. Pat. No. 6,756,523, "Adenovirus vectors for the transfer of foreign genes into cells of the central nervous system particularly in brain,". Administration can be rapid as by injection or made over a period of time as by slow infusion or administration of slow release formulations,

The subject antisense oligonucleotides can also be linked or conjugated with agents that provide desirable pharmaceutical or pharmacodynamic properties. For example, the antisense oligonucleotide can be coupled to any substance, known in the art to promote penetration or transport across the blood-brain barrier, such as an antibody to the transferrin receptor, and administered by intravenous injection. The antisense compound can be linked with a viral vector, for example, that makes the antisense compound more effective and/or increases the transport of the antisense compound across the blood-brain barrier. Osmotic blood brain barrier disruption can also be accomplished by, e.g., infusion of sugars including, but not limited to, meso erythritol, xylitol, D(+) galactose, D(+) lactose, D(+) xylose, dulcitol, myo-inositol, L(-) fructose, D(-) mannitol, D(+) glucose, D(+) arabinose, D(-) arabinose, cellobiose, D(+) maltose, D(+) raffinose, L(+) rhamnose, D(+) melibiose, D(-) ribose, adonitol, D(+) arabitol, L(-) arabitol, D(+) fucose, L(-) fucose, D(-) lyxose, L(+) lyxose, and L(-) lyxose, or amino acids including, but not limited to, glutamine, lysine, arginine, asparagine, aspartic acid, cysteine, glutamic acid, glycine, histidine, leucine, methionine, phenylalanine, proline, serine, threonine, tyrosine, valine, and taurine. Methods and materials for enhancing blood brain barrier penetration are described, e.g., in U. S. Patent No. 4,866,042, "Method for the delivery of genetic material across the blood brain barrier," 6,294,520, "Material for passage through the blood-brain barrier," and 6,936,589, "Parenteral delivery systems," .

The subject antisense compounds may be admixed, encapsulated, conjugated or otherwise associated with other molecules, molecule structures or mixtures of compounds, for example, liposomes, receptor-targeted molecules, oral, rectal, topical or other formulations, for assisting in uptake, distribution and/or absorption. For example, cationic lipids may be included in the formulation to facilitate oligonucleotide uptake, One such composition shown to facilitate uptake is LIPOFECTIN (available from GIBCO-BRL, Bethesda, MD).

Oligonucleotides with at least one 2'-O-methoxyethyl modification are believed to be particularly useful for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

The pharmaceutical formulations of the present disclosure which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present disclosure may be formulated into any of many possible dosage forms such as, but not limited to, tablets, capsules, gel capsules, liquid syrups, soft gels, suppositories, and enemas. The compositions of the present disclosure may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

Pharmaceutical compositions of the present disclosure include, but are not limited to, solutions, emulsions, foams and liposome-containing formulations. The pharmaceutical compositions and formulations of the present disclosure may comprise one or more penetration enhancers, carriers, excipients or other active or inactive ingredients.

Emulsions are typically heterogeneous systems of one liquid dispersed in another in the form of droplets usually exceeding 0.1 µm in diameter. Emulsions may contain additional components in addition to the dispersed phases, and the active drug that may be present as a solution in either the aqueous phase, oily phase or itself as a separate phase. Microemulsions are included as an instance of the present disclosure. Emulsions and their uses are well known in the art and are further described in U.S. Pat. No. 6,287,860.

Formulations of the present disclosure include liposomal formulations. As used in the present disclosure, the term "liposome" means a vesicle composed of amphiphilic lipids arranged in a spherical bilayer or bilayers. Liposomes are unilamellar or multilamellar vesicles which have a membrane formed from a lipophilic material and an aqueous interior that contains the composition to be delivered. Cationic liposomes are positively charged liposomes that are believed to interact with negatively charged DNA molecules to form a stable complex. Liposomes that are pH-sensitive or negatively-charged are believed to entrap DNA rather than complex with it. Both cationic and noncationic liposomes have been used to deliver DNA to cells.

Liposomes also include "sterically stabilized" liposomes, a term which, as used herein, refers to liposomes comprising one or more specialized lipids. When incorporated into liposomes, these specialized lipids result in liposomes with enhanced circulation lifetimes relative to liposomes lacking such specialized lipids. Examples of sterically stabilized liposomes are those in which part of the vesicle-forming lipid portion of the liposome comprises one or more glycolipids or is derivatized with one or more hydrophilic polymers, such as a polyethylene glycol (PEG) moiety. Liposomes and their uses are further described in U.S. Pat. No. 6,287,860.

The pharmaceutical formulations and compositions of the present disclosure may also include surfactants. The use of surfactants in drug products, formulations and in emulsions is well known in the art. Surfactants and their uses are further described in U.S. Pat. No. 6,287,860.

In one instance, , the present disclosure employs various penetration enhancers to effect the efficient delivery of nucleic acids, particularly oligonucleotides. In addition to aiding the diffusion of non-lipophilic drugs across cell membranes, penetration enhancers also enhance the permeability of lipophilic drugs. Penetration enhancers may be classified as belonging to one of five broad categories, i.e., surfactants, fatty acids, bile salts, chelating agents, and non-chelating nonsurfactants. Penetration enhancers and their uses are further described in U.S. Pat. No. 6,287,860.

One of skill in the art will recognize that formulations are routinely designed according to their intended use, i.e. route of administration.

formulations for topical administration include those in which the oligonucleotides of the disclosure are in admixture with a topical delivery agent such as lipids, liposomes, fatty acids, fatty acid esters, steroids, chelating agents and surfactants, lipids and liposomes include neutral (e.g. dioleoyl-phosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoyl-pliosphatidyl ethanolamine DOTMA).

For topical or other administration, oligonucleotides of the disclosure may be encapsulated within liposomes or may form complexes thereto, in particular to cationic liposomes. Alternatively, oligonucleotides may be completed to lipids, in particular to cationic lipids, fatty acids and esters, pharmaceutically acceptable salts thereof, and their uses are further described in U.S. Pat. No. 6,287,860.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable, oral formulations are those in which oligonucleotides of the disclosure are administered in conjunction with one or more penetration enhancers surfactants and chelators, surfactants include fatty acids and/or esters or salts thereof, bile acids and/or salts thereof bile acids/salts and fatty acids and their uses are further described in U.S. Pat. No. 6,287,860. Also are combinations of penetration enhancers, for example, fatty acids/salts in combination with bile acids/salts. A particularly combination is the sodium salt of lauric acid, capric acid and UDCA, Further penetration enhancers include polyoxyethylene-9-lauryl ether, polyoxyethylene-20-cetyl ether. Oligonucleotides of the disclosure may be delivered orally, in granular form including sprayed dried particles, or completed to form micro or nanoparticles. Oligonucleotide complexing agents and their uses are further described in U.S. Pat. No. 6,287,860.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Certain instances of the disclosure provide pharmaceutical compositions containing one or more oligomeric compounds and one or more other chemotherapeutic agents that function by a non-antisense mechanism. Examples of such chemotherapeutic agents include but are not limited to cancer chemotherapeutic drugs such as daunorubicin, daunomycin, dactinomycin, doxorubicin, epirubicin, idarubicin, esorubicin, bleomycin, mafosfamide, ifosfamide, cytosine arabinoside, bischloroethyl- nitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5- azacytidine, hydroxyurea, deoxycoformycin, 4-hydroxyperoxycyclo-phosphoramide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin and diethylstilbestrol (DES). When used with the compounds of the disclosure such chemotherapeutic agents may be used individually (e.g., 5-FU and oligonucleotide), sequentially (e.g., 5-FU and oligonucleotide for a period of time followed by MTX and oligonucleotide), or in combination with one or more other such chemotherapeutic agents (e.g., 5-FU, MTX and oligonucleotide, or 5-FU, radiotherapy and oligonucleotide). Anti-inflammatory drugs, including but not limited to nonsteroidal anti-inflammatory drugs and corticosteroids, and antiviral drugs, including but not limited to ribavirin, vidarabine, acyclovir and ganciclovir, may also be combined in compositions of the disclosure. Combinations of antisense compounds and other non-antisense drugs are also within the scope of this disclosure. Two or more combined compounds may be used together or sequentially.

In another related instance, compositions of the disclosure may contain one or more antisense compounds, particularly oligonucleotides, targeted to a first nucleic acid and one or more additional antisense compounds targeted to a second nucleic acid target. For example, the first target may be a particular antisense sequence of a Sirtuin (SIRT), and the second target may be a region from another nucleotide sequence. Alternatively, compositions of the disclosure may contain two or more antisense compounds targeted to different regions of the same Sirtuin (SIRT) nucleic acid target. Numerous examples of antisense compounds are illustrated herein and others may be selected from among suitable compounds known in the art. Two or more combined compounds may be used together or sequentially.

### Dosing:

The formulation of therapeutic compositions and their subsequent administration (dosing) is believed to be within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skin can easily determine optimum dosages, dosing methodologies and repetition rates. Optimum dosages may vary depending on the relative potency of individual oligonucleotides, and can generally be estimated based on EC50s found to be effective in in vitro and in vivo animal models. In general, dosage is from 0.01 µg to 100 g per kg of body weight, and may be given once or more daily, weekly, monthly or yearly, or even once every 2 to 20 years. Persons of ordinary skill in the art. can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the oligonucleotide is administered in maintenance doses, ranging from 0.01 µg to 100 g per kg of body weight, once or more daily, to once every 20 years,

In instances, a patient is treated with a dosage of drug that is at least about 1, at least about 2, at least about 3, at least about 4, at least about 5, at least about 6, at least about 7, at least about 8, at least about 9, at least about 10, at least about 15, at least about 20, at least about 25, at least about 30, at least about 35, at least about 40, at least about 45, at least about 50, at least about 60, at least about 70, at least about 80, at least about 90, or at least about 100 mg/kg body weight. Certain injected dosages of antisense oligonucleotides are described, e.g., in U.S. Pat. No. 7,563,884, "Antisense modulation of PTP1B expression,".

By their citation of various references in this document, Applicants do not admit any particular reference is "prior art" to their invention. Embodiments of inventive compositions and methods are illustrated in the following examples.

### EXAMPLES

The following non-limiting Examples serve to illustrate selected embodiments of the invention. It will be appreciated that variations in proportions and alternatives in elements of the components shown will be apparent to those skilled in the art and are within the scope of instances of the present disclosure.

### Example 1: Design of antisense oligonucleotides specific for a nucleic acid molecule antisense to a Sirtuin (SIRT) and/or a sense strand of a Sirtuin (SIRT) polynucleotide

As indicated above the term "oligonucleotide specific for" or "oligonucleotide targets" refers to an oligonucleotide having a sequence (i) capable of forming a stable complex with a portion of the targeted gene, or (ii) capable of forming a stable duplex with a portion of an mRNA transcript of the targeted gene.

Selection of appropriate oligonucleotides is facilitated by using computer programs that automatically align nucleic acid sequences and indicate regions of identity or homology Such programs are used to compare nucleic acid sequences obtained, for example, by searching databases such as GenBank or by sequencing PCR products. Comparison of nucleic acid sequences from a range of species allows the selection of nucleic acid sequences that display an appropriate degree of identity between species. In the case of genes that have not been sequenced, Southern blots are performed to allow a determination of the degree of identity between genes in target species and other species. By performing Southern blots at varying degrees of stringency, as is well known in the art, it is possible to obtain an approximate measure of identity. These procedures allow the selection of oligonucleotides that exhibit a high degree of complementarity to target nucleic acid sequences in a subject to be controlled and a lower degree of complementarity to corresponding nucleic acid sequences in other species. One skilled in the art will realize that there is considerable latitude in selecting appropriate regions of genes for use in the present invention.

An antisense compound is "specifically hybridizable" when binding of the compound to the target nucleic acid interferes with the normal function of the target nucleic acid to cause a modulation of function and/or activity, and there is a sufficient degree of complementarity to avoid non-specific binding of the antisense compound to non-target nucleic acid sequences under conditions in which specific binding is desired, i.e., under physiological conditions in the case of in vivo assays or therapeutic treatment, and under conditions in which assays are performed in the case of in vitro assays

The hybridization properties of the oligonucleotides described herein can be determined by one or more in vitro assays as known in the art. For example, the properties of the oligonucleotides described herein can be obtained by determination of binding strength between the target natural antisense and a potential drug molecules using melting curve assay.

The binding strength between the target natural antisense and a potential drug molecule (Molecule) can be estimated using any of the established methods of measuring the strength of intermolecular interactions, for example, a melting curve assay.

Melting curve assay determines the temperature at which a rapid transition from double-stranded to single-stranded conformation occurs for the natural antisense/Molecule complex. This temperature is widely accepted as a reliable measure of the interaction strength between the two molecules.

A melting curve assay can be performed using a cDNA copy of the actual natural antisense RNA molecule or a synthetic DNA or RNA nucleotide corresponding to the binding site of the Molecule. Multiple kits containing all necessary reagents to perform this assay are available (e.g. Applied Biosystems Inc. MeltDoctor kit). These kits include a suitable buffer solution containing one of the double strand DNA (dsDNA) binding dyes (such as ABI HRM dyes, SYBR Green, SYTO, etc.). The properties of the dsDNA dyes are such that they emit almost no fluorescence in free form, but are highly fluorescent when bound to dsDNA.

To perform the assay the cDNA or a corresponding oligonucleotide are mixed with Molecule in concentrations defined by the particular manufacturer's protocols. The mixture is heated to 95 °C to dissociate all pre-formed dsDNA complexes, then slowly cooled to room temperature or other lower temperature defined by the kit manufacturer to allow the DMA molecules to anneal. The newly formed complexes are then slowly heated to 95 °C with simultaneous continuous collection of data on the amount of fluorescence that is produced by the reaction. The fluorescence intensity is inversely proportional to the amounts of dsDNA present in the reaction. The data can be collected using a real time PCR instrument compatible with the kit (e.g.ABI's StepOne Plus Real Time PCR System or LightTyper instrument, Roche Diagnostics, Lewes, UK).

Melting peaks are constructed by plotting the negative derivative of fluorescence with respect to temperature (-d(Fluorescence)/dT) on the y-axis) against temperature (x-axis) using appropriate software (for example LightTyper (Roche) or SDS Dissociation Curve, ABI). The data is analyzed to identify the temperature of the rapid transition from dsDNA complex to single strand molecules. This temperature is called Tm and is directly proportional to the strength of interaction between the two molecules. Typically, Tm will exceed 40 °C.

### Example 2: Modulation of SIRT polynucleotides

### Treatment of HepG2 cells with antisense oligonucleotides

HepG2 cells from ATCC (cat# HB-8065) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted to the concentration of 20 µM. Two µl of this solution was incubated with 400 µl of Opti-MEM media (Gibco cat#31985-(70) and 4 µl of Lipofectamine 2000 (Invitrogen cat# 11668019) at room temperature for 20 min and applied to each well of the 6 well plates with HepG2 cells. A Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) or High Capacity cDNA Reverse Transcription Kit (cat# 4368813) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_ml, Hs00202030_ml and Hs00213036_ml by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems).

Fold change in gene expression after treatment with antisense oligonucleotides was calculated based on the difference in 18S-normalized dCt values between treated and mock-transfected samples.

### Results:

Real time PCR results show that the levels of the SIRT1 mRNA in HepG2 cells significantly increased 48 h after treatment with some antisense oligonucleotides to SIRT1 antisense CV396200 (Figure 3,4).

Real Time PCR results show that levels of SIRT1 mRNA in HepG2 cells are significantly increased in one of the oligonucleotides designed to SIRT1 antisense CV396200 (Fig 8).

Real Time PCR results show that levels of SIRT1 mRNA in HepG2 cells are significantly increased in two of the oligonucleotides designed to SIRT1 antisense CV428275 (Fig 9).

The results show that a significant increase in SIRT1 mRNA levels in HepG2 cells 48 hours after treatment with one of the oligonucleotides designed to SIRT antisense BE717453. (Fig 10).

The results show that show that the levels of the SIRT1 mRNA in HepG2 cells are significantly increased 48 h after treatment with three of the oligonucleotides designed to SIRT1 antisense AV718812 respectively (Fig 11).

Real time PCR results show that the levels of SIRT1 mRNA in HepG2 cells are significantly increased 48 h after treatment with two of the oligos designed to SIRT1 antisense AW169958 (Fig 12).

RT PCR results show that sirt3 levels in HepG2 cells are increased 48 hours after treatment with phosphorothioate antisense oligonucleotides designed to sirt3 antisense Hs,683117 (CUR-1545-1550) (Fig 17).

Real time PCR results show that the levels of SIRT6 mRNA in HepG2 cells are significantly increased 48h after treatment with one of the oliogs designed to SIRT6 antisense NM_133475 (Fig 18).

Real time PCR results show that the levels of SIRT6 mRNA in HepG2 cells are significantly increased 48h after treatment with one of the oliogs designed to SIRT6 antisense bf772662 (Fig 19)

### Treatment of 3T3 cells with antisense oligonucleotides

3T3 cells from ATCC (cat# CRL-1658) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted to the concentration of 20 µM. Two µl of this solution was incubated with 400 µl of Opti-MEM media (Gibco cat#31985-070) and 4 µl of Lipofectamine 2000 (Invitrogen cat# 11668019) at room temperature for 20 min and applied to each well of the 6 well plates with 3T3 cells. A Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) or High Capacity cDNA Reverse Transcription Kit (cat# 4368813) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_ml by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems).

Fold change in gene expression after treatment with antisense oligonucleotides was calculated based on the difference in 18S-normalized dCt values between treated and mock-transfected samples.

### Results:

Real time PCR results show that the levels of SIRT1 mRNA are significantly increased in 3T3 cells 48 h after treatment with three of the oligonucleotides designed to SIRT1 mouse antisense AK044604 (Fig 13).

Real time PCR results show that the levels of SIRT1 mRNA are significantly increased in 3T3 cells 48 h after treatment with five of the oligonucleotides designed to SIRT1 mouse antisense AK044604 (Fig 14).

Real time PCR results show that the levels of SIRT1 mRNA are significantly increased in 3T3 cells 48 h after treatment with two of the oligonucleotides designed to SIRT1 mouse antisense AK044604 (Fig 15).

Real time PCR results show that the levels of SIRT1 mRNA are significantly increased in 3T3 cells 48 h after treatment with two of the oligonucleotides designed to SIRT1 mouse antisense AK044604 (Fig 16). *Treatment of Vero 76* cells *with antisense oligonucleotides:*

Vero76 cells from ATCC (cat# CRL-1587) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO2. One day before the experiment the cells were replated at the density of 1.5 × 10⁵/ml into 6 well plates and incubated at 37°C and 5% CO2. On the day of the experiment the media in the 6 well plates was changed to fresh growth media. All antisense oligonucleotides were diluted in water to the concentration of 20 µM. 2 µl of this solution was incubated with 400 µl of Opti-MEM media (Gibco cat#31985-070) and 4 ul of Lipofectamine 2000 (Invitrogen cat# 11668019) at room temperature for 20 min and applied to each well of the 6 well plates with Vero76 cells. Similar mixture including 2 µl of water instead of the oligonucleotide solution was used for the mock- transfected controls. After 3-18 h of incubation at 37°C and 5% CO2 the media was changed to fresh growth media. 48 h after addition of antisense oligonucleotides the media was removed and RNA was extracted from the cells using SV Total RNA Isolation System from. Promega (cat # Z3105) or RNeasy Total RNA Isolation kit from Qiagen (cat# 74181), following the manufacturers' instructions. 600 ng of RNA was added to the reverse transcription reaction performed using Verso cDNA kit from Thermo Scientific (cat#AB1453B) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_ml by Applied Biosystems Inc., Foster City CA). The following PCR cycle was used: 50°C for 2 min, 95°C for 1 0 min, 40 cycles of (95°C for 15 seconds, 60°C for 1 min) using StepOne Plus Real Time PCR Machine (Applied Biosystems). Fold change in gene expression after treatment with antisense oligonucleotides was calculated based on the difference in 18S-normalized dCt values between treated and mock-transfected samples.
*Results:* Real time PCR results show that the levels of the SIRT1 mRNA in Vero cells significantly increased 48 h after treatment with antisense oligonucleotides to SIRT1 antisense CV396-700 (Figure 5).

### Treatment of DBS cells with antisense oligonucleotides

DBS cells from ATCC (cat# CCL-161) were grown in growth media (MEM/EBSS (Hyclone cat #SH30024, or Mediatech cat # MT-10-010-CV) +10% FBS (Mediatech cat# MT35- 011-CV)+ penicillin/streptomycin (Mediatech cat# MT30-002-CI)) at 37°C and 5% CO₂. One day before the experiment the cells were replated at the density of 1.5 × 10⁵/ml into 6 well plates and incubated at 37°C and 5% CO₂. On the day of the experiment the media in the 6 well plates was changed to fresh growth media. All antisense oligonucleotides were diluted to the concentration of 20 µM. Two µl of this solution was incubated with 400 µl of Opti-MEM media (Gibco cat#31985-070) and 4 µl of Lipofectamine 2000 (Invitrogen cat# 11668019) at room temperature for 20 min and applied to each well of the 6 well plates with 3T3 cells. A Similar mixture including 2 µl of water instead of the oligonucleotide solution was used for the mock-transfected controls. After 3-18 h of incubation at 37°C and 5% CO₂ the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) or High Capacity cDNA Reverse Transcription Kit (cat# 4368813) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00213036_ml by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems).

Fold change in gene expression after treatment with antisense oligonucleotides was calculated based on the difference in 18S-normalized dCt values between treated and mock-transfected samples.
*Results:* Real time PCR results show that the levels of SIRT6 mRNA in DBS cells are significantly increased 48h after treatment with two of the oliogs designed to SIRT6 antisense bf772662 and one oligo designed to NM_133475 (Fig 20).

### Example 3: Modulation of SIRT Gene Expression

### Materials and Methods

### Treatment of HepG2 cells with naked antisense oligonucleotides:

HepG2 cells from ATCC (cat# HB-8065) were grown in growth media (MEM/EBSS (Hyclone cat #SH30024, or Mediatech cat # MT-10-010-CV) +10% FBS (Mediatech cat# MT35- 011-CV) + penicillin/streptomycin (Mediatech cat# MT30-002-CI)) at 37°C and 5% CO2. One day before the experiment the cells were replated at the density of 0.5 × 10⁵/ml into 6 well plates and incubated at 37°C and 5% CO2. On the day of the experiment the media in the 6 well plates was replaced with 1.5 ml/well of fresh growth media. All antisense oligonucleotides were diluted in water to the concentration of 20 µM. 2 µl of this solution was incubated with 400 µl of Opti-MEM media (Gibco cat#31985-070) and 4 ul of Lipofectamine 2000 (Invitrogen cat# 11668019) at room temperature for 20 min and applied to each well of the 6 well plates with HepG2 cells. Similar mixture including 2 µl of water instead of the oligonucleotide solution was used for the mock-transfected controls. After 3-18 h of incubation at 37°C and 5% CO2 the media was changed to fresh growth media. 72 h after addition of antisense oligonucleotides the cells were redosed as described in above. 48h after the second dosing of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_ml, Hs00202030_ml and Hs00213036_ml by Applied Biosystems Inc., Foster City CA). The following PCR cycle was used: 50°C for 2 min, 95°C for 10 min, 40 cycles of (95°C for 15 seconds, 60°C for 1 min) using StepOne Plus Real Time PCR Machine (Applied Biosystems). Fold change in gene expression after treatment with antisense oligonucleotides was calculated based on the difference in 18S-normalized dCt values between treated and mock-transfected samples.

Primers and probe for the custom designed Taqman assay for exon 4: AACTGGAGCTGGGGTGTCTGTTTCA (SEQ ID NO: 95) the SIRT1 natural antisense CV396200.
Forward Primer Seq. CCATCAGACGACATCCCTTAACAAA (SEQ ID NO: 96)
Reverse Primer Seq. ACATTATATCATAGCTCCTAAAGGAGATGCA (SEQ ID NO: 97)
Reporter Seq. CAGAGTTTCAATTCCC (SEQ ID NO: 98)

### Results:

The results show that the levels of the SIRT1 mRNA in HepG2 cells are significantly increased 48 h after treatment with one of the siRNAs designed to sirtas (sirtas_5, P=0.01). In the same samples the levels of sirtas RNA were significantly decreased after treatment with sirtas_5, but unchanged after treatment with sirtas_6 and sirtas_7, which also had no effect on the SIRT1 mRNA levels (Fig. 2), sirtas_5, sirtas_6 and sirtas_7 correspond to SEQ ID NO: 38, 39 and 40 respectively.

### Treatment of primary monkey hepatocytes

Primary monkey hepatocytes were introduced into culture by RxGen Inc. and plated in 6 well plates. They were treated with oligonucleotides as follows. The media in the 6 well plates was changed to fresh growth media consisting of William's Medium E (Sigma cat#W4128) supplemented with 5% FBS, 50 U/ml penicillin and 50 ug/ml streptomycin, 4 ug/ml insulin, 1 uM dexamethasone, 10 ug/ml Fungin (InVivogen, San Diego CA). All antisense oligonucleotides were diluted to the concentration of 20 µM. 2 µl of this solution was incubated with 400 µl of Opti-MEM media (Gibco cat#31985-070) and 4 µl of Lipofectamine 2000 (Invitrogen cat# 11668019) at room temperature for 20 min and applied to each well of the 6 well plates with cells. Similar mixture including 2 µl of water instead of the oligonucleotide solution was used for the mock-transfected controls. After 3-18 h of incubation at 37°C and 5% CO2 the media was changed to fresh growth media. 48 h after addition of antisense oligonucleotides the media was removed and RNA was extracted from the cells using SV Total RNA Isolation System from Promega (cat # Z3105) or RNeasy Total RNA Isolation kit from Qiagen (cat# 74181) following the manufacturers' instructions. 600 ng of RNA was added to the reverse transcription reaction performed using Verso cDNA kit from Thermo Scientific (cat#AB1453B) as described in the manufacturer's protocol. The cDNA from this reverse transcription reaction was used to monitor gene expression by real time PCR using ABI Taqman Gene Expression Mix (cat#4369510) and primers/probes designed by ABI (Applied Biosystems Taqman Gene Expression Assay: Hs00202021_m1, Hs00202030_m1 and Hs00213036_m1 by Applied Biosystems Inc., Foster City CA). The following PCR cycle was used: 50°C for 2 min, 95°C for 10 min, 40 cycles of (95°C for 15 seconds, 60°C for 1 min) using Mx4000 thermal cycler (Stratagene). Fold change in gene expression after treatment with antisense oligonucleotides was calculated based on the difference in 18S-normalized dCt values between treated and mock-transfected samples.
*Results:* The results are shown in Figure 7. Real time PCR results show an increase in SIRT1 mRNA levels after treatment with an oligonucleotide against SIRT1 antisense.

### Example 4: Efficacy and Duration of Action Study of CUR 963 in the African Green Monkey

The objective of this study was to assess and compare the effect of antisense knockdown of the discordant noncoding antisense sequences that regulate the SIRT1 genes following intravenous administration in a nonhuman primate model. The antisense oligonucleotide test articles designed to inhibit the SIRT1 regulatory sequences were designated as CUR 963.
CUR 963: +G*+T*C*T*G*A*T*G*G*+A*+G*+A (SEQ ID NO: 34).
CUR 962 (control): +G*+C*T*A*G*T*C*T*G*+T*+T*+G (SEQ ID NO: 99).

### REGULATORY TEST GUIDELINES

This study was designed in accordance with accepted toxicological principles and to comply with International Conference of Harmonization (ICH) Harmonized Tripartite Guidelines (Non-Clinical Safety Studies for the Conduct of Human Clinical Trials for Pharmaceuticals ICH M3(m), 2000 November 9), and generally accepted procedures for the testing of therapeutic agents.

### TEST AND CONTROL ARTICLES

### Test Article Identity and Preparation

The test article, CUR-963, is a chemically stabilized antisense oligonucleotide. The vehicle for intravenous delivery is phosphate-buffered saline (PBS).

### Vehicle characterization

For the PBS vehicle, the composition, batch number, expiry date and storage conditions (temperature and light/dark) was obtained from the supplier.

### Test Article Storage and Handling

The test substance and vehicle were stored according to the received storage conditions supplied by the Sponsor and manufacturer, accordingly.

### Analysis of the Test Article formulations

Samples of the test article formulation will be cryopreserved for analysis of the concentration, stability and homogeneity of the test substance formulations.

### TEST SYSTEM RATIONALE

The primate is a suitable non rodent species, acceptable to regulatory authorities as an indicator of potential hazards, and for which extensive background data are available. The African green monkey specifically is a highly clinically relevant model of multiple human physiologic and disease states.

The intravenous route of administration corresponds to a possible human therapeutic route. The dose of the test articles was based on the results of the dose finding studies of analogous compounds previously performed in the African green monkey.

African green monkeys were chosen as the primate of choice as the test substances' target sequences are conserved across species with 100 % homology in primates. Additionally, the test substance is a synthetic oligonucleotide. Consequently, dosing in primates allows for a superior assessment of the efficacy of these compounds that would be more reflective of the uptake likely to be seen in humans than in any other species.

### ANIMALS

*Species:Chlorocebus sabaeus,* non-human primate

*Breed:* African green monkey indigenous to St. Kitts.

*Source:* RxGen, Lower Bourryeau, St. Kitts, West Indies.

*Expected Age:* The test animals were adults.

*Expected Body Weight:* The monkeys weigh approximately 3-4 kg. The actual range may vary but will be documented in the data.

*Sex:* The test animals were adult females.

*Number of Animals:* Ten animals were screened to ensure identification of 8 animals appropriate for enrollment in the study.

*Number on Study:* **Females: 8**

*Justification for Number on Study:* This study was designed to use the fewest number of animals possible, consistent with the primary objective of evaluating the therapeutic efficacy of the test article in the African green monkey and prior studies of the systemic administration of this type of oligonucleotide in this species.

*Animal Specification:* Ten adult African Green monkeys in the weight range of 3 to 4 kg, were employed in the study. The monkeys were drug-naïve adult animals humanely trapped from the feral population that inhabits the island. Trapped monkeys were treated with antihelminthics to eliminate any possible intestinal parasite burden and were observed in quarantine for a minimum of 4 weeks prior to screening for study enrollment. The age of trapped monkeys were estimated by size and dentation, with the exclusion of older animals from the study. Prior to study enrollment, a clinical exam was performed on each monkey, including evaluation of locomotion and dexterity. Blood samples were taken and sent to Antech Diagnostics (Memphis, TN) for comprehensive clinical chemistries and a complete blood count and lipid profiles (see sections 9.2 and 319567928 for specifications). Monkeys with abnormal lab values, as determined by comparison to the established normal range for monkeys in the St. Kitts colony, were excluded from the study. In order to identify 8 monkeys that satisfy this criterion, 10 monkeys were screened, with the screening of additional animals as needed. Before study initiation, the selected monkeys will be transferred to individual cages to acclimate to individual housing for a one-week period. Only animals deemed suitable for experimentation will be enrolled in the study. The actual (or estimated) age and weight ranges at the start of the study will be detailed in the raw data and final report.

*Animal Health and Welfare:* The highest standards of animal welfare were followed and adhered to guidelines stipulated by the St Kitts Department of Agriculture and the U.S. Department of Health and Human Services. All studies will be conducted in accordance with these requirements and all applicable codes of practice for the care and housing of laboratory animals. All applicable standards for veterinary care, operation, and review as contained in the NIH Guide for the Care and Use of Animals. The St. Kitts facility maintains an animal research committee that reviews the protocols and inspects the facilities as required by the Guide. The Foundation has an approved assurance filed with the Office of Laboratory *Animal* Welfare, as required by the Guide, #A4384-0I (Axion Research Foundation St. Kitts Biomedical Foundation). There are no special nonhuman primate veterinary care issues and biohazard issues raised by the research specified in this study.

*Housing* and *Environment:* To allow detection of any treatment-related clinical signs, the animals were housed individually prior to surgery and postoperatively until sacrifice. The primate building in which the individual cages were situated were illuminated entirely by ambient light, which at 17 degrees north latitude approximates a 1.2hr:12hr light-dark cycle as recommended in the U.S. D.M.H.S guidelines. The RxGen primate building was completely ventilated to the outside. Additional air movement was assured by ceiling fans to maintain a constant target temperature of 23-35°C, as is typical of St. Kitts throughout the year. Twenty-four hour extremes of temperature and relative humidity (which also will not be controlled) were measured daily, During the study, the cages were cleaned at regular intervals.

*Diet and* Water: Each animal was offered approximately 90 grams per day of a standard monkey chow diet (TekLad, Madison, WI). The specific nutritional composition of the diet was recorded. The water was periodically analyzed for microbiological purity. The criteria for acceptable levels of contaminants in stock diet and water supply were within the analytical specifications established by the diet manufacturer and the periodic facility water evaluations, respectively. The water met all criteria necessary for certification as acceptable for human consumption.

### EXPERIMENTAL DESIGN

*Animal Identification and Randomization:* Allocation was done by means of a stratified randomization procedure based on bodyweight and plasma cholesterol profiles. Prior to and after allocation to a group, each animal was identified by a tattoo on the abdomen. Tattoos are placed on all colony animals as a means of identification in the course of routine health inspections. A cage plan was drawn up to identify the individuals housed within, and individual monkeys were further identified by a labeled tag attached to their respective cage.

*Group* sizes, doses *and identification numbers*: The animals were assigned to 2 treatment groups, comprised of 4 monkeys in each group. Specific animal identification numbers were provided to each monkey according to the facility numbering system. This system uniquely identifies each monkey by a letter followed by a three digit number, e.g. Y032.

*Route* and Frequency *of Administration:* Animals were dosed once daily on Days 1, 3, and 5 delivered intravenously by manual infusion over ∼10 min. The infusion rate will be 24 mL/kg/h. The animals were sedated with ketamine and xylazine prior to and during the dosing procedure. A venous catheter (Tenumo mini vein infusion set, 20 gauge needle, or similar appropriate infusion set) was inserted into the saphenous vein. Dosing took place in each monkey between 8:00 and 10:00 a.m. shortly after the animals wake and prior to feeding. A blood sample to assess plasma cholesterol and other lipid levels as described in Blood Chemistry section below, was collected just prior to each infusion. Blood collection preceded feeding at both sampling intervals to minimize dietary effects on cholesterol measurements.

*Clinical Observations:* All visible signs of reaction to treatment were recorded on each day of dosing. In addition, the animals were examined at least once each week for physical attributes such as appearance and general condition.

*Body Weights:* Body weights were recorded at weekly intervals during the treatment and post-treatment periods.

*Food Consumption:* Individual food consumption was not quantified. Feeding patterns were however monitored and a note made of any major changes.

*Mortality and Morbidity:* Mortality and morbidity will be recorded. Any decision regarding premature sacrifice will be made after consultation with the Study Director and with the Sponsor's Monitoring Scientist, if possible. Animals that are found dead or killed prematurely will be subjected to necropsy with collection of liver, kidney, heart and spleen lung tissues for histopathology. In the event of premature sacrifice a blood sample will also be taken (if possible) and the parameters determined. Animals that are found dead after regular working hours will be refrigerated overnight and necropsies performed at the start of the next working day. If the condition of an animal requires premature sacrifice, it will be euthanized by intravenous overdose of sodium pentobarbital. All research is governed by the Principles for Use of Animals. RxGen is required by law to comply with the U.S. Department of Health and Human Services standards for primate facility, which dictates the levels of severity that the procedures within this study, specified as mild, must abide.

### CLINICAL LABORATORY STUDIES

*Fat Biopsies:* A subcutaneous fat biopsy was performed on all study monkeys except Y775 on study days 26 by tissue extraction through a 1 cm midline incision inferior to the umbilicus. Biopsies were immediately immersed in a labeled eryotube containing 2 rills of RNAlater (Qiagen) and incubated at 4°C overnight, after which the RNAlater was aspirated and the sample tube flash frozen in liquid nitrogen. Following transportation in liquid nitrogen total RNA was isolated for real-time qPCR of target genes.

*Results:* Real time PCR results show an increase in SIRT1 mRNA levels in fat biopsies from monkeys dosed with CUR-963, an oligonucleotide designed to SIRT1 antisense CV396200.1, compared to monkeys dosed with CUR-962 (SEQ ID NO.: 99), an oligonucleotide which had no effect on SIRT1 expression *in vitro* (designed to ApoA1 antisense DA327409, data not shown). mRNA levels were determined by real time PCR (Fig 6).

### Example 5: In vivo modulation of Sirtuin (SIRT) by antisense DNA oligonucleotides

*Treatment with Antisense DNA Oligonucleotides (ASO)*. Antisense oligonucleotides (ASO) specific for SIRT1 AS are administered to C57BI/6J mice which are fed a high fat diet for 12 weeks to induce obesity and diabetes. (Purushotham A. et al., (2009) Cell Metabolism 9, p. 327-338,). The treatment of the mice with ASO will start at the time of the implementation of the high fat diet. Mice are injected IP once a week with ASO prepared in normal saline, at a concentration of 5mg/kg.

*Measurements of body weight and food intake:* Body weight and food intake of mice are measured twice per week, prior to IP injection of the ASO.

Blood *glucose measurements:* Fed and fasted blood glucose concentrations are measured each week by taking a sample of blood from the tail vein.

*Glucose Tolerance Tests (GTT):* The GTT will be done totally twice per mouse, halfway through the diet (at week 4) and near the end (at week 10) of the high fat diet. The GTT will inform us about the glucose tolerance of the mice that is the capacity to rapidly clear a glucose bolus from the blood stream. This is a measure for diabetes. Mice are fasted overnight for 16 hours. Mice are injected IP glucose 2g/kg. This translates into a final volume of 0.2ml 30% (w/v) glucose solution for a mouse of 30g weight. *Glucose* measurements are taken prior to glucose injection and at 5, 15, 30, 60, 90 and 120 min post-injection. *Glucose* is measured by cutting the tail tip 1mm from the end of the tail under isoflurane anesthesia prior to IP glucose injection. The blood droplet is aspirated into a strip and glucose concentration is measured with a glucometer. The GTT will be done totally twice per mouse, halfway through the diet (at week 4) and near the end (at week 10) of the high fat diet. The GTT will inform us about the glucose tolerance of the mice that is the capacity to rapidly clear a glucose bolus from the blood stream. This is a measure for diabetes.

*Insulin* Tolerance *Test (ITT):* Mice are fasted for 6 hours from 9am till 3pm. Mice are then injected IP 0.5-1U Insulin/kg, The insulin concentration will be adjusted such that the final injected volume is 0.1-0.15ml. Blood glucose measurements are taken prior to injection and at 5. 15, 30, 45, and 60 minutes post-injection. Blood is collected exactly as described under GTT. In addition to monitoring the glucose levels, the behavior of the mice is constantly observed during the ITT. Hypoglycemia can manifest as a change in behavior with the animals becoming very quiet and showing discomfort. To prevent hypoglycemia, glucose (1g/kg) is injected IP in a final volume of 0.1-0.15ml as soon as the blood glucose concentration falls below 50mg/ml or signs of discomfort are observed.

*Blood Collection by Facial Vein Puncture:* Mice are restrained by the scruff of the neck and base of the tail, slightly compressing the blood vessels of the neck through the tautness of the grip on the neck skin. The sampling site is on the jaw slightly in front of the angle of the mandible. The skin at the sampling site is punctured with an 180 needle or a lancet at a 90° angle until the tip of the needle/lancet just passes through the skin. Blood samples are collected using microhematocrit tubes. After blood has been collected, the grip on the neck is loosened and pressure is applied at the insertion site with a gauze sponge to ensure hemostasis. 0.05-0.2ml of blood will be collected by this method. This procedure will be performed only once in week 5 of the high fat diet and eventually in week 12 if the intracardiac puncture is not working (see below). Blood hormones which regulate the metabolism of glucose and lipids (such as insulin, adiponeetin and leptin) are measured using commercially available ELISA kits. (e.g., R&D Systems, Minneapolis, MN, Assay Pro St. Charles, MO, Mabtech, Mariemont, OH)

*Intracardiac Puncture:* At the end of the 12 week high fat diet, mice will be anesthetized by continuous isoflurane inhalation. Anesthesia is induced by placing the mice in an induction box, which is supplied with isoflurane and oxygen. Mice will be restrained on their back. The heart is punctured with a 27G needle. Following exsanguineation, the head is decapitated to ensure death. Tissues (liver, pancreas, white and brown adipose tissue, and skeletal muscle) are collected for further investigations (RNA and protein measurements and histology). Around 0.5 - 1 ml of blood will be obtained and used to determine several critical parameters of of glucose and lipid metabolism (glucose, insulin, cholesterol, triglycerides, free fatty acids, leptin, adipokines, corticosteroids, thyroid hormones). If difficulties occur in this method, we will collect blood by facial vein puncture under isoflurane anesthesia instead (see above).

## Claims

1. An antisense oligonucleotide which binds a human Sirtuin 6 (SIRT6) natural antisense transcript for use as a therapeutic compound, wherein said antisense oligonucleotide increases the expression of human Sirtuin 6 (SIRT6) (SEQ ID NO: 4), and wherein the natural antisense transcript has the nucleic acid sequence as set forth in SEQ ID NOs: 13 or 14.

2. An antisense oligonucleotide which binds a human Sirtuin 6 (SIRT6) natural antisense transcript for use in the prevention or treatment of a Sirtuin 6 (SIRT6) associated disease or disorder which is cancer, wherein said antisense oligonucleotide increases the expression of human Sirtuin 6 (SIRT6) (SEQ ID NO: 4), and wherein the natural antisense transcript has the nucleic acid sequence as set forth in SEQ ID NOs: 13 or 14.

3. Use of an antisense oligonucleotide which binds a human Sirtuin 6 (SIRT6) natural antisense transcript for the manufacture of a medicament for the prevention or treatment of a Sirtuin 6 (SIRT6) associated disease or disorder which is cancer, wherein said antisense oligonucleotide increases the expression of human Sirtuin 6 (SIRT6) (SEQ ID NO: 4), and wherein the natural antisense transcript has the nucleic acid sequence as set forth in SEQ ID NOs: 13 or 14.

4. An antisense oligonucleotide for use according to Claim 2 or use of an antisense oligonucleotide according to Claim 3, wherein the cancer is selected from breast cancer, colorectal cancer or prostate cancer.

5. An *in vitro* method of increasing the expression of Sirtuin (SIRT) in patient cells or tissues comprising: contacting said cells or tissues with an antisense oligonucleotide which binds a human Sirtuin 6 (SIRT6) natural antisense transcript; thereby increasing the expression of human Sirtuin 6 (SIRT6) (SEQ ID NO: 4) and wherein the natural antisense transcript has the nucleic acid sequence as set forth in SEQ ID NOs: 13 or 14.

6. An antisense oligonucleotide for use according to any one of Claims 1, 2, or 4, or use of an antisense oligonucleotide according to any one of Claims 3 or 4, or a method according to Claim 5, wherein:
a. the antisense oligonucleotide is a siRNA compound;
b. the antisense oligonucleotide is single-stranded.

7. An antisense oligonucleotide for use according to any one of Claims 1, 2, 4, or 6, or use of an antisense oligonucleotide according to any one of Claims 3, 4, or 6, or a method according to Claims 5 or 6, wherein the antisense oligonucleotide comprises at least one of SEQ ID NOs: 89, 91 and 92.

8. An antisense oligonucleotide for use according to any one of Claims 1, 2, 4, 6 or 7, or use of an antisense oligonucleotide according to any one of Claims 3, 4, 6 or 7, or a method according to any one of Claims 5 to 7, wherein the expression of said Sirtuin (SIRT) is increased by at least 10%.

9. An antisense oligonucleotide for use according to any one of Claims 1, 2, 5, 6 to 8, or use of an antisense oligonucleotide according to any one of Claims 3, 4, or 6 to 8, or a method according to any one of Claims 5 to 8, wherein the antisense oligonucleotide further comprises one or more modifications comprising:
a. at least one modified internucleoside linkage selected from: a phosphorothioate, 2'- O-methoxyethyl (MOE), 2'-fluoro, alkylphosphonate, phosphorodithioate, alkylphosphonothioate, phosphoramidate, carbamate, carbonate, phosphate triester, acetamidate, carboxymethyl ester, and combinations thereof;
b. at least one modified nucleotide selected from: a peptide nucleic acid (PNA), a locked nucleic acid (LNA), an arabino-nucleic acid (FANA), analogues, derivatives, and combinations thereof; or
c. at least one modified sugar moiety selected from: a 2'-O-methoxyethyl modified sugar moiety, a 2'-methoxy modified sugar moiety, a 2'-O-alkyl modified sugar moiety, a bicyclic sugar moiety, and combinations thereof.

10. An antisense oligonucleotide having the features of an oligonucleotide defined in any one of Claims 1 to 9.

11. An antisense oligonucleotide according to Claim 10, wherein the antisense oligonucleotide is between 10 to 30 nucleotides in length.

12. An antisense oligonucleotide according to Claim 10 or Claim 11, wherein the antisense oligonucleotide has at least 90% sequence identity to a complement of a Sirtuin 6 (SIRT6) natural antisense transcript having the nucleic acid sequence as set forth in SEQ ID NOs: 13 or 14.

13. A pharmaceutical composition comprising at least one antisense oligonucleotide as defined in any one of Claims 10 to 12 and a pharmaceutically acceptable excipient.

## Patentansprüche

1. Ein Antisense-Oligonukleotid, das ein humanes natürliches Antisense-Transkript von Sirtuin 6 (SIRT6) bindet, zur Verwendung als eine therapeutische Verbindung, wobei das genannte Antisense-Oligonukleotid die Expression von humanem Sirtuin 6 (SIRT6) (SEQ ID NO: 4) steigert, und wobei das natürliche Antisense-Transkript die Nukleinsäuresequenz wie in einer von SEQ ID NOs: 13 oder 14 dargelegt aufweist.

2. Antisense-Oligonukleotid, das ein humanes natürliches Antisense-Transkript von Sirtuin 6 (SIRT6) bindet, zur Verwendung bei der Prävention oder Behandlung einer mit Sirtuin 6 (SIRT6) assoziierten Erkrankung oder Störung, die Krebs ist, wobei das genannte Antisense-Oligonukleotid die Expression von humanem Sirtuin 6 (SIRT6) (SEQ ID NO: 4) steigert, und wobei das natürliche Antisense-Transkript die Nukleinsäuresequenz wie in einer von SEQ ID NOs: 13 oder 14 dargelegt aufweist.

3. Verwendung eines Antisense-Oligonukleotids, das ein humanes natürliches Antisense-Transkript von Sirtuin 6 (SIRT6) bindet, zur Herstellung eines Medikaments für die Prävention oder Behandlung einer mit Sirtuin 6 (SIRT6) assoziierten Erkrankung oder Störung, die Krebs ist, wobei das genannte Antisense-Oligonukleotid die Expression von humanem Sirtuin 6 (SIRT6) (SEQ ID NO: 4) steigert, und wobei das natürliche Antisense-Transkript die Nukleinsäuresequenz wie in einer von SEQ ID NOs: 13 oder 14 dargelegt aufweist.

4. Antisense-Oligonukleotid zur Verwendung nach Anspruch 2 oder Verwendung eines Antisense-Oligonukleotids nach Anspruch 3, wobei der Krebs aus Brustkrebs, Kolorektalkrebs oder Prostatakrebs ausgewählt ist.

5. In-vitro-Verfahren zur Steigerung der Expression von Sirtuin (SIRT) in Patientenzellen oder -geweben, umfassend: In-Kontakt-Bringen der genannten Zellen oder Gewebe mit einem Antisense-Oligonukleotid, das ein humanes natürliches Antisense-Transkript von Sirtuin 6 (SIRT6) bindet; wobei auf diese Weise die Expression von humanem Sirtuin 6 (SIRT6) (SEQ ID NO: 4) gesteigert wird, und wobei das natürliche Antisense-Transkript die Nukleinsäuresequenz wie in einer von SEQ ID NOs: 13 oder 14 dargelegt aufweist.

6. Antisense-Oligonukleotid zur Verwendung nach einem der Ansprüche 1, 2 oder 4 oder Verwendung eines Antisense-Oligonukleotids nach einem der Ansprüche 3 oder 4 oder ein Verfahren nach Anspruch 5, wobei:
a. das Antisense-Oligonukleotid eine siRNA-Verbindung ist;
b. das Antisense-Oligonukleotid einzelsträngig ist.

7. Antisense-Oligonukleotid zur Verwendung nach einem der Ansprüche 1, 2, 4 oder 6 oder Verwendung eines Antisense-Oligonukleotids nach einem der Ansprüche 3, 4 oder 6 oder Verfahren nach Ansprüchen 5 oder 6, wobei das Antisense-Oligonukleotid mindestens eine von SEQ ID NOs: 89, 91 und 92 umfasst.

8. Antisense-Oligonukleotid zur Verwendung nach einem der Ansprüche 1, 2, 4, 6 oder 7 oder Verwendung eines Antisense-Oligonukleotids nach einem der Ansprüche 3, 4, 6 oder 7 oder Verfahren nach einem der Ansprüche 5 bis 7, wobei die Expression des genannten Sirtuins (SIRT) um mindestens 10% gesteigert wird.

9. Antisense-Oligonukleotid zur Verwendung nach einem der Ansprüche 1, 2, 5, 6 bis 8 oder Verwendung eines Antisense-Oligonukleotids nach einem der Ansprüche 3, 4, oder 6 bis 8 oder Verfahren nach einem der Ansprüche 5 bis 8, wobei das Antisense-Oligonukleotid ferner eine oder mehrere Modifikationen umfasst, umfassend:
a. mindestens eine modifizierte Internukleosid-Verknüpfung, ausgewählt aus: einem Phosphorothioat, 2'-0-Methoxyethyl (MOE), 2'-Fluor, Alkylphosphonat, Phosphorodithioat, Alkylphosphonothioat, Phosphoramidat, Carbamat, Carbonat, Phosphattriester, Acetamidat, Carboxymethylester und Kombinationen davon;
b. mindestens ein modifiziertes Nukleotid, ausgewählt aus: einer Peptidnukleinsäure (PNA, einer verbrückten Nukleinsäure (locked nucleic acid, LNA), einer Arabinonukleinsäure(FANA, Analoga, Derivaten und Kombinationen davon; oder
c. mindestens einen modifizierten Zuckerteil, ausgewählt aus: einem 2'-O-Methoxyethyl-modifizierten Zuckerteil, einem 2'-Methoxy-modifizierten Zuckerteil, einem 2'-O-Alkyl-modifizierten Zuckerteil, einem bicyclischen Zuckerteil und Kombinationen davon.

10. Antisense-Oligonukleotid mit den Merkmalen eines Oligonukleotids, die in einem der Ansprüche 1 bis 9 definiert sind.

11. Antisense-Oligonukleotid nach Anspruch 10, wobei das Antisense-Oligonukleotid zwischen 10 und 30 Nukleotide lang ist.

12. Antisense-Oligonukleotid nach Anspruch 10 oder Anspruch 11, wobei das Antisense-Oligonukleotid eine mindestens 90%ige Sequenzidentität mit einem Komplement von einem natürlichen Antisense-Transkript von Sirtuin 6 (SIRT6) aufweist, das die Nukleinsäuresequenz wie in einer von SEQ ID NOs: 13 oder 14 dargelegt aufweist.

13. Pharmazeutische Zusammensetzung, umfassend mindestens ein Antisense-Oligonukleotid wie in einem der Ansprüche 10 oder 12 definiert und einen pharmazeutisch verträglichen Hilfsstoff.

## Revendications

1. Oligonucléotide antisens qui se lie à un transcrit antisens naturel de la Sirtuine 6 humaine (SIRT6), destiné à une utilisation comme composé thérapeutique, ledit oligonucléotide antisens augmentant l'expression de la Sirtuine 6 humaine (SIRT6) (SÉQ. ID n° 4), et le transcrit antisens naturel ayant la séquence d'acide nucléique telle qu'énoncée dans les SÉQ. ID n^{os} 13 ou 14.

2. Oligonucléotide antisens qui se lie à un transcrit antisens naturel de la Sirtuine 6 humaine (SIRT6), destiné à une utilisation dans la prévention ou le traitement d'une maladie ou d'une affection associée à la Sirtuine 6 (SIRT6), laquelle est un cancer, ledit oligonucléotide antisens augmentant l'expression de la Sirtuine 6 humaine (SIRT6) (SÉQ. ID n° 4), et le transcrit antisens naturel ayant la séquence d'acide nucléique telle qu'énoncée dans les SÉQ. ID n^{os} 13 ou 14.

3. Utilisation d'un oligonucléotide antisens qui se lie à un transcrit antisens naturel de la Sirtuine 6 humaine (SIRT6) pour la fabrication d'un médicament destiné à la prévention ou au traitement d'une maladie ou d'une affection associée à la Sirtuine 6 (SIRT6), laquelle est un cancer, ledit oligonucléotide antisens augmentant l'expression de la Sirtuine 6 humaine (SIRT6) (SÉQ. ID n° 4), et le transcrit antisens naturel ayant la séquence d'acide nucléique telle qu'énoncée dans les SÉQ. ID n^{os} 13 ou 14.

4. Oligonucléotide antisens destiné à une utilisation selon la revendication 2, ou utilisation d'un oligonucléotide antisens selon la revendication 3, le cancer étant sélectionné parmi un cancer du sein, un cancer colorectal ou un cancer de la prostate.

5. Procédé *in vitro* d'augmentation de l'expression de la Sirtuine (SIRT) dans des cellules ou des tissus de patients, comprenant: la mise en contact desdites cellules ou desdits tissus avec un oligonucléotide antisens qui se lie à un transcrit antisens naturel de la Sirtuine 6 humaine (SIRT6); en augmentant de ce fait l'expression de la Sirtuine 6 humaine (SIRT6) (SÉQ. ID n° 4), et le transcrit antisens naturel ayant la séquence d'acide nucléique telle qu'énoncée dans les SÉQ. ID n^{os} 13 ou 14.

6. Oligonucléotide antisens destiné à une utilisation selon l'une quelconque des revendications 1, 2 ou 4, ou utilisation d'un oligonucléotide antisens selon l'une quelconque des revendications 3 ou 4, ou procédé selon la revendication 5, dans lequel ou dans laquelle:
a. l'oligonucléotide antisens est un composé d'ARNsi ;
b. l'oligonucléotide antisens est monobrin.

7. Oligonucléotide antisens destiné à une utilisation selon l'une quelconque des revendications 1, 2, 4 ou 6, ou utilisation d'un oligonucléotide antisens selon l'une quelconque des revendications 3, 4 ou 6, ou procédé selon les revendications 5 ou 6, l'oligonucléotide antisens comprenant au moins l'une des SÉQ. ID n^{os} 89, 91 et 92.

8. Oligonucléotide antisens destiné à une utilisation selon l'une quelconque des revendications 1, 2, 4, 6 ou 7, ou utilisation d'un oligonucléotide antisens selon l'une quelconque des revendications 3, 4, 6 ou 7, ou procédé selon l'une quelconque des revendications 5 à 7, l'expression de ladite Sirtuine (SIRT) étant augmentée d'au moins 10%.

9. Oligonucléotide antisens destiné à une utilisation selon l'une quelconque des revendications 1, 2, 5, 6 à 8, ou utilisation d'un oligonucléotide antisens selon l'une quelconque des revendications 3, 4, ou 6 à 8, ou procédé selon l'une quelconque des revendications 5 à 8, l'oligonucléotide antisens comportant en outre une ou plusieurs modifications comprenant:
a. au moins une liaison internucléosidique modifiée sélectionnée parmi : un phosphorothioate, 2'-0-méthoxyéthyle (MOE), 2'-fluoro, alkylphosphonate, phosphorodithioate, alkylphosphonothioate, phosphoramidate, carbamate, carbonate, triester de phosphate, acétamidate, ester carboxyméthylique, et des combinaisons de ceux-ci ;
b. au moins un nucléotide modifié sélectionné parmi : un acide nucléique peptidique (PNA), un acide nucléique bloqué (LNA), un acide arabino-nucléique (FANA), des analogues, des dérivés, et des combinaisons de ceux-ci ; ou
c. au moins une fraction sucre modifiée sélectionnée parmi: une fraction sucre modifiée par un groupe 2'-O-méthoxyéthyle, une fraction sucre modifiée par un groupe 2'-méthoxy, une fraction sucre modifiée par un groupe 2'-O-alkyle, une fraction sucre bicyclique, et des combinaisons de celles-ci.

10. Oligonucléotide antisens ayant les caractéristiques d'un oligonucléotide défini dans l'une quelconque des revendications 1 à 9.

11. Oligonucléotide antisens selon la revendication 10, l'oligonucléotide antisens ayant une longueur de 10 à 30 nucléotides.

12. Oligonucléotide antisens selon la revendication 10 ou la revendication 11, l'oligonucléotide antisens ayant une identité de séquence d'au moins 90 % avec un complément d'un transcrit antisens naturel de la Sirtuine 6 (SIRT6) ayant la séquence d'acide nucléique telle qu'énoncée dans les SÉQ. ID n^{os} 13 ou 14.

13. Composition pharmaceutique comprenant au moins un oligonucléotide antisens tel que défini dans l'une quelconque des revendications 10 à 12 et un excipient pharmaceutiquement acceptable.
